# EUROPEAN PATENT APPLICATION

(11) **EP 4 242 232 A1**
(43) Date of publication of application: **13.09.2023**
(21) Application number: 21888662.0
(22) Date of filing: 05.11.2021
(51) Int. Cl.: C07K 16/28, C12N 15/13, C12N 15/63, C12N 5/10, A61K 39/395, A61P 35/00

(54) **BISPECIFIC ANTIBODY AND USE THEREOF**

(30) Priority: 06.11.2020 CN 202011232115
(71) Applicant: Bio-Thera Solutions, Ltd., Guangzhou, Guangdong 510530 (CN)
(72) Inventor: CHEN, Yifan, Guangzhou, Guangdong 510530 (CN); LIANG, Qiulian, Guangzhou, Guangdong 510530 (CN); PEI, Shujun, Guangzhou, Guangdong 510530 (CN); YU, Jin-Chen, Guangzhou, Guangdong 510530 (CN); LI, Shengfeng, Guangzhou, Guangdong 510530 (CN)
(74) Representative: Engelhard, Markus
(86) International application number: PCT/CN2021/129045
(87) International publication number: WO 2022/095970

(57) **Abstract**

The present invention provides a bispecific antibody and use thereof, and the bispecific antibody or an antigen-binding fragment of the present invention can bind to two or more antigens or two or more epitopes of a same antigen. The antibody or the antigen-binding fragment of the present invention can be used to treat a variety of diseases, such as inflammatory diseases, autoimmune diseases, cancers or spinal cord injury, and can also be used for diagnosis and prognosis of related diseases.

## Description

### TECHNICAL FIELD

The present invention relates the field of bio-pharmaceuticals, and particularly to a bispecific antibody and use thereof.

### BACKGROUND

Cancer immunotherapy, a revolutionary breakthrough in cancer therapy in recent years, is a research fever in the field of biological science. As more new antibody drugs are approved for marketing, the prospect of cancer therapy has shifted from complementary elements of traditional therapies to central and standard cancer treatment regimens. On the road to conquering cancer by activating the function of T cells with an immune checkpoint as a target and thus enhancing enhance the ability of the acquired immune system, the development of antibody therapy around the PD-L1/PD-1 pathway has become a hotspot in the field. When an organism develops inflammation or is infected, the programmed death-ligand 1 (PD-L1) can be induced to be expressed on hematopoietic, endothelial or epithelial cells, suppressing the immune response by binding to programmed death receptor 1 (PD-1) expressed on T cells, B cells and monocytes. The PD-1 cytoplasmic region contains two tyrosine-based signaling domains, an immunoreceptor tyrosine-based inhibitory motif (ITIM) and an immunoreceptor tyrosine-based switch motif (ITSM). PD-L 1 is overexpressed in many cancers, including a wide variety of solid tumors and hematological tumors, such as bladder cancer, breast cancer, colon cancer, lung cancer, melanoma, ovarian cancer, gastric cancer, thyroid cancer, primary mediastinal large B-cell lymphoma and classical Hodgkin's lymphoma. PD-L1 overexpressed on tumor cells binds to PD-1 on T cells and activates ITIM domain of PD-1, which results in T cell dysfunction and failure, prevents cytotoxic T cells from effectively targeting tumor cells, and sends out a "don't find me" signal to the immune system. Thus, tumor cells obtain adaptive immune tolerance, thereby promoting tumor invasion and causing poor prognosis.

The innate immune system is the first line of nonspecific defense against infection and malignant cell transformation. In the innate immune system, monocytes, macrophages and dendritic cells act as antigen presenting cells (APCs) by phagocytosis. The ability of APCs to phagocytose tumor cells by phagocytosis is an indispensable bridge linking the innate immunity and the adaptive immunity. Targeting checkpoints that regulate phagocytosis (phagocytosis checkpoints, such as CD47-SIRPα) provides a new approach to the development of cancer immunotherapy. CD47 (integrin associated protein, IAP, CD47) is widely expressed on the surface of normal cells and, by binding to signal regulatory protein α (SIRPα) on the surface of macrophages, releases a "don't eat me" signal, thereby protecting healthy cells from being "eaten" by macrophages. Cancer cells take advantage of this mechanism and overexpress CD47 on their surface, such that macrophages treat them as "normal cells" and thus they escape from the monitoring of the innate immunity and the macrophage-mediated phagocytic attack. Studies have confirmed that almost all tumor cells highly express CD47, such as thyroid cancer, ovarian cancer, prostate cancer, cervical cancer, bladder cancer, head and neck cancer, gastric cancer, acute myelogenous leukemia, B- and T-cell acute leukemia and non-Hodgkin's lymphoma, and that CD47 overexpression is associated with poor clinical prognosis.

Monoclonal antibody immunotherapy brings long-lasting survival benefits to some patients, and patients' hope for cure is realized. With the intensive research on cancer and other various diseases, it is recognized that the development and progression of the diseases involve various signal pathways and mechanisms, and the single stimulation or inhibition of the monoclonal antibody cannot achieve the effect of completely killing the tumor, so that the response rate of single drug therapy is limited. A bispecific antibody (bispecific monoclonal antibody, BsAb) can bind to two antigens or two different epitopes of an antigen simultaneously, block or activate different signaling pathways, and mediate tumor cell killing by immune cells more effectively, so that better therapeutic effects than those of monoclonal antibodies and even combination therapy can be obtained. With the rapid development of antibody drug development technologies, there comes the research and development boom of bispecific antibodies. A plurality of bispecific antibodies are in clinical development stage, and three bispecific antibodies have been approved for marketing. In 2009, the European Union approved the first therapeutic bispecific antibody, catumaxomab (targeting CD3 and EpCAM), which is structurally a Triomab and is used for the treatment of cancerous ascites. Compared with the monoclonal antibody, the bispecific antibody has the advantages of high specificity, strong targeting property, small dose and the like. It is of great significance in clinical treatment of tumors and has breakthroughs in the treatment of inflammatory diseases as well, and thus has very wide application prospects.

According to the existing knowledge, PD-L1 and CD47 proteins are simultaneously overexpressed on tumor cells, and the tumor cells escape from the monitoring of the innate immune system through PD-L1/PD1 and CD47/SIRPα pathways and obtain adaptive immune tolerance. Therefore, by blocking the PD-L1/PD1 and CD47/SIRPα pathways at the same time and activating the innate immunity and the adaptive immunity system, better tumor growth inhibition effect can be possibly exerted.

### SUMMARY

The present invention provides a multivalent and multispecific antibody or an antigen-binding fragment and use thereof. The present invention provides a novel anti-PD-L1 antibody featuring better binding to PD-L1 and good druggability. In addition, the present invention provides a novel anti-CD47 antibody that does not bind to human red blood cells, does not cause agglutination of human red blood cells, and has good target specificity and relatively small side effects. In addition, by adopting a "knobs-into-holes" symmetrical IgG-like bispecific antibody structure with a common light chain, the present invention realizes the correct assembly of different heavy chains and light chains and increases the yield of the bispecific antibody. The bispecific antibody is easy to be stably expressed in culture cells *in vitro* without complex production processes. In addition, the bispecific antibody structure adopted by the present invention can maintain the affinity of each antigen-binding site in binding to corresponding different epitopes without the antigen-binding sites interfering with each other when binding to different epitopes, thereby having good druggability. Further, the bispecific antibody of the present invention has stable physical and biological properties, which allow the antibody to have higher potential in being produced and developed.

In some embodiments, the present invention provides a bispecific antibody or an antigen-binding fragment. The antibody or the antigen-binding fragment provided herein can bind to two or more antigens, or two or more epitopes of a same antigen, or two or more copies of a same epitope.

In some embodiments, the present invention provides a bispecific antibody or an antigen-binding fragment, wherein the antibody or the antigen-binding fragment comprises a variable region a specifically binding to PD-L1, wherein the variable region a comprises one or more amino acid sequences of (a)-(f):
(a) a VHa CDR1 comprising an amino acid sequence set forth in any one of SEQ ID NOs: 4-8 or an amino acid sequence having one or more conservative amino acid substitutions as compared with the amino acid sequence set forth in any one of SEQ ID NOs: 4-8;
(b) a VHa CDR2 comprising an amino acid sequence set forth in any one of SEQ ID NOs: 9-18 or an amino acid sequence having one or more conservative amino acid substitutions as compared with the amino acid sequence set forth in any one of SEQ ID NOs: 9-14;
(c) a VHa CDR3 comprising an amino acid sequence set forth in SEQ ID NO: 19 or 20 or an amino acid sequence having one or more conservative amino acid substitutions as compared with the amino acid sequence set forth in SEQ ID NO: 19 or 20;
(d) a VLa CDR1 comprising an amino acid sequence set forth in any one of SEQ ID NOs: 36-40 or an amino acid sequence having one or more conservative amino acid substitutions as compared with the amino acid sequence set forth in any one of SEQ ID NOs: 36-40;
(e) a VLa CDR2 comprising an amino acid sequence set forth in any one of SEQ ID NOs: 41-44 or an amino acid sequence having one or more conservative amino acid substitutions as compared with the amino acid sequence set forth in any one of SEQ ID NOs: 41-44; and
(f) a VLa CDR3 comprising an amino acid sequence set forth in any one of SEQ ID NOs: 45-48 or an amino acid sequence having one or more conservative amino acid substitutions as compared with the amino acid sequence set forth in any one of SEQ ID NOs: 45-48.

In some embodiments, the variable region a comprises a heavy chain variable region a (VHa) comprising a VHa CDR1, a VHa CDR2 and a VHa CDR3, wherein the VHa CDR1 comprises an amino acid sequence set forth in any one of SEQ ID NOs: 4-8; the VHa CDR2 comprises an amino acid sequence set forth in any one of SEQ ID NOs: 9-18; and the VHa CDR3 comprises an amino acid sequence set forth in SEQ ID NO: 19 or 20.

In some embodiments, the variable region a comprises a light chain variable region a (VLa) comprising a VLa CDR1, a VLa CDR2 and a VLa CDR3, wherein the VLa CDR1 comprises an amino acid sequence set forth in any one of SEQ ID NOs: 36-40; the VLa CDR2 comprises an amino acid sequence set forth in any one of SEQ ID NOs: 41-44; and the VLa CDR3 comprises an amino acid sequence set forth in any one of SEQ ID NOs: 45-48.

In some embodiments, the variable region a comprises a VHa and a VLa, wherein the VHa comprises a VHa CDR1, a VHa CDR2 and a VHa CDR3; and the VLa comprises a VLa CDR1, a VLa CDR2 and a VLa CDR3, wherein the VHa CDR1 comprises an amino acid sequence set forth in any one of SEQ ID NOs: 4-8; the VHa CDR2 comprises an amino acid sequence set forth in any one of SEQ ID NOs: 9-18; the VHa CDR3 comprises an amino acid sequence set forth in SEQ ID NO: 19 or 20; the VLa CDR1 comprises an amino acid sequence set forth in any one of SEQ ID NOs: 36-40; the VLa CDR2 comprises an amino acid sequence set forth in any one of SEQ ID NOs: 41-44; and the VLa CDR3 comprises an amino acid sequence set forth in any one of SEQ ID NOs: 45-48.

In some embodiments, the VHa CDR1 comprises an amino acid sequence set forth in SEQ ID NO: 4 or an amino acid sequence having one or more conservative amino acid substitutions as compared with the amino acid sequence set forth in SEQ ID NO: 4; the VHa CDR2 comprises an amino acid sequence set forth in any one of SEQ ID NOs: 9-14 or an amino acid sequence having one or more conservative amino acid substitutions as compared with the amino acid sequence set forth in any one of SEQ ID NOs: 9-14; and the VHa CDR3 comprises an amino acid sequence set forth in SEQ ID NO: 19 or an amino acid sequence having one or more conservative amino acid substitutions as compared with the amino acid sequence set forth in SEQ ID NO: 19.

In some embodiments, the VHa CDR1 comprises an amino acid sequence set forth in SEQ ID NO: 4 or an amino acid sequence having one or more conservative amino acid substitutions as compared with the amino acid sequence set forth in SEQ ID NO: 4; the VHa CDR2 comprises an amino acid sequence set forth in SEQ ID NO: 14 or an amino acid sequence having one or more conservative amino acid substitutions as compared with the amino acid sequence set forth in SEQ ID NO: 14; and the VHa CDR3 comprises an amino acid sequence set forth in SEQ ID NO: 19 or an amino acid sequence having one or more conservative amino acid substitutions as compared with the amino acid sequence set forth in SEQ ID NO: 19.

In some embodiments, the VLa CDR1 comprises an amino acid sequence set forth in SEQ ID NO: 36 or an amino acid sequence having one or more conservative amino acid substitutions as compared with the amino acid sequence set forth in SEQ ID NO: 36; the VLa CDR2 comprises an amino acid sequence set forth in SEQ ID NO: 41 or an amino acid sequence having one or more conservative amino acid substitutions as compared with the amino acid sequence set forth in SEQ ID NO: 41; and the VLa CDR3 comprises an amino acid sequence set forth in SEQ ID NO: 45 or an amino acid sequence having one or more conservative amino acid substitutions as compared with the amino acid sequence set forth in SEQ ID NO: 45.

In some embodiments, the VHa CDR1 comprises an amino acid sequence set forth in SEQ ID NO: 4 or an amino acid sequence having one or more conservative amino acid substitutions as compared with the amino acid sequence set forth in SEQ ID NO: 4; the VHa CDR2 comprises an amino acid sequence set forth in SEQ ID NO: 14 or an amino acid sequence having one or more conservative amino acid substitutions as compared with the amino acid sequence set forth in SEQ ID NO: 14; the VHa CDR3 comprises an amino acid sequence set forth in SEQ ID NO: 19 or an amino acid sequence having one or more conservative amino acid substitutions as compared with the amino acid sequence set forth in SEQ ID NO: 19; the VLa CDR1 comprises an amino acid sequence set forth in SEQ ID NO: 36 or an amino acid sequence having one or more conservative amino acid substitutions as compared with the amino acid sequence set forth in SEQ ID NO: 36; the VLa CDR2 comprises an amino acid sequence set forth in SEQ ID NO: 41 or an amino acid sequence having one or more conservative amino acid substitutions as compared with the amino acid sequence set forth in SEQ ID NO: 41; and the VLa CDR3 comprises an amino acid sequence set forth in SEQ ID NO: 45 or an amino acid sequence having one or more conservative amino acid substitutions as compared with the amino acid sequence set forth in SEQ ID NO: 45.

In some embodiments, the VHa comprises an amino acid sequence set forth in any one of SEQ ID NOs: 49-63, or an amino acid sequence having at least 90% homology to the amino acid sequence set forth in any one of SEQ ID NOs: 49-63, or an amino acid sequence having one or more conservative amino acid substitutions as compared with the amino acid sequence set forth in any one of SEQ ID NOs: 49-63.

In some embodiments, the VHa comprises an amino acid sequence set forth in SEQ ID NO: 54, or an amino acid sequence having at least 90% homology to the amino acid sequence set forth in SEQ ID NO: 54, or an amino acid sequence having one or more conservative amino acid substitutions as compared with the amino acid sequence set forth in SEQ ID NO: 54.

In some embodiments, the VLa comprises an amino acid sequence set forth in any one of SEQ ID NOs: 75-80, or an amino acid sequence having at least 90% homology to the amino acid sequence set forth in any one of SEQ ID NOs: 75-80, or an amino acid sequence having one or more conservative amino acid substitutions as compared with the amino acid sequence set forth in any one of SEQ ID NOs: 75-80.

In some embodiments, the VLa comprises an amino acid sequence set forth in SEQ ID NO: 75, or an amino acid sequence having at least 90% homology to the amino acid sequence set forth in SEQ ID NO: 75, or an amino acid sequence having one or more conservative amino acid substitutions as compared with the amino acid sequence set forth in SEQ ID NO: 75.

In some embodiments, the variable region a comprises a VHa and a VLa. The VHa comprises an amino acid sequence set forth in SEQ ID NO: 54, or an amino acid sequence having at least 90% homology to the amino acid sequence set forth in SEQ ID NO: 54, or an amino acid sequence having one or more conservative amino acid substitutions as compared with the amino acid sequence set forth in SEQ ID NO: 54; and the VLa comprises an amino acid sequence set forth in SEQ ID NO: 75, or an amino acid sequence having at least 90% homology to the amino acid sequence set forth in SEQ ID NO: 75, or an amino acid sequence having one or more conservative amino acid substitutions as compared with the amino acid sequence set forth in SEQ ID NO: 75.

In some embodiments, the present invention provides a bispecific antibody or an antigen-binding fragment, wherein the antibody or the antigen-binding fragment further comprises a light chain constant region a (CLa) and a heavy chain constant region a (CHa).

In some embodiments, the CHa comprises an amino acid sequence set forth in any one of SEQ ID NOs: 81 and 83-86, or an amino acid sequence having at least 90% homology to the amino acid sequence set forth in any one of SEQ ID NOs: 81 and 83-86, or an amino acid sequence having one or more conservative amino acid substitutions as compared with the amino acid sequence set forth in any one of SEQ ID NOs: 81 and 83-86.

In some embodiments, the CLa comprises an amino acid sequence set forth in SEQ ID NO: 82, or an amino acid sequence having at least 90% homology to the amino acid sequence set forth in SEQ ID NO: 82, or an amino acid sequence having one or more conservative amino acid substitutions as compared with the amino acid sequence set forth in SEQ ID NO: 82.

In some embodiments, the present invention provides a bispecific antibody or an antigen-binding fragment, wherein the antibody or the antigen-binding fragment comprises a heavy chain a, a heavy chain x, and a light chain a and a light chain x, wherein the heavy chain a is paired with the light chain a to form a PD-L1 antigen-binding site, and the heavy chain x is paired with the light chain x to form the other antigen-binding site. In some embodiments, the heavy chain a comprises the VHa CDR1, the VHa CDR2 and the VHa CDR3 described herein. In some embodiments, the heavy chain a comprises the VHa described herein. In some embodiments, the heavy chain a comprises the VHa and the CHa described herein. In some embodiments, the light chain a comprises the VLa CDR1, the VLa CDR2 and the VLa CDR3 described herein. In some embodiments, the light chain a comprises the VLa described herein. In some embodiments, the light chain a comprises the VLa and the CLa described herein. In some embodiments, the heavy chain x is the same as a heavy chain b described herein. In some embodiments, the light chain x is the same as the light chain a.

In some embodiments, the other antigen is CD47, LAG3, TGFβ, CTLA-4, 4-1BB, PD1, TIGIT, KIR, c-Met, VISTA or BCMA. In some embodiments, the other antigen is CD47.

In some embodiments, the CDRs of the heavy chain x are selected from heavy chain CDRs in magrolimab, AO-176 (Arch Oncology), TJC4 (I-Mab biopharma), AK117 (Akesobio), IBI188 (Innovent Biologics), and the like. In some embodiments, the variable region of the heavy chain x is selected from heavy chain variable regions in magrolimab, AO-176 (Arch Oncology), TJC4 (I-Mab biopharma), AK117 (Akesobio), IBI188 (Innovent Biologics), and the like. In some embodiments, the CDRs of the light chain x are selected from light chain CDRs in magrolimab, AO-176 (Arch Oncology), TJC4 (I-Mab biopharma), AK117 (Akesobio), IBI188 (Innovent Biologics), and the like. In some embodiments, the variable region of the light chain x is selected from light chain variable regions in magrolimab, AO-176 (Arch Oncology), TJC4 (I-Mab biopharma), AK117 (Akesobio), IBI188 (Innovent Biologics), and the like. In some embodiments, the light chain x is the same as the light chain a.

In some embodiments, the heavy chain a comprises an amino acid sequence set forth in SEQ ID NO: 92 or 94, or an amino acid sequence having at least 90% homology to the amino acid sequence set forth in SEQ ID NO: 92 or 94, or an amino acid sequence having one or more conservative amino acid substitutions as compared with the amino acid sequence set forth in SEQ ID NO: 92 or 94.

In some embodiments, the bispecific antibody or the antigen-binding fragment provided herein has an affinity index KD of ≤ 10 nM for PD-L1. In some embodiments, the bispecific antibody or the antigen-binding fragment provided herein has an affinity index KD of ≤ 1 nM for PD-L1. In some embodiments, the bispecific antibody or the antigen-binding fragment provided herein has an affinity index KD of ≤ 0.5 nM for PD-L1.

In another aspect, the present invention provides a bispecific antibody or an antigen-binding fragment, wherein the antibody or the antigen-binding fragment comprises a variable region b specifically binding to CD47, wherein the variable region b comprises one or more amino acid sequences of (g)-(1):
(g) a VHb CDR1 comprising an amino acid sequence set forth in any one of SEQ ID NOs: 21-23 or an amino acid sequence having one or more conservative amino acid substitutions as compared with the amino acid sequence set forth in any one of SEQ ID NOs: 21-23;
(h) a VHb CDR2 comprising an amino acid sequence set forth in any one of SEQ ID NOs: 24-28 or an amino acid sequence having one or more conservative amino acid substitutions as compared with the amino acid sequence set forth in any one of SEQ ID NOs: 24-28;
(i) a VHb CDR3 comprising an amino acid sequence set forth in any one of SEQ ID NOs: 29-35 or an amino acid sequence having one or more conservative amino acid substitutions as compared with the amino acid sequence set forth in any one of SEQ ID NOs: 29-35;
(j) a VLb CDR1 comprising an amino acid sequence set forth in any one of SEQ ID NOs: 36-40 or an amino acid sequence having one or more conservative amino acid substitutions as compared with the amino acid sequence set forth in any one of SEQ ID NOs: 36-40;
(k) a VLb CDR2 comprising an amino acid sequence set forth in any one of SEQ ID NOs: 41-44 or an amino acid sequence having one or more conservative amino acid substitutions as compared with the amino acid sequence set forth in any one of SEQ ID NOs: 41-44; and
(1) a VLb CDR3 comprising an amino acid sequence set forth in any one of SEQ ID NOs: 45-48 or an amino acid sequence having one or more conservative amino acid substitutions as compared with the amino acid sequence set forth in any one of SEQ ID NOs: 45-48.

In some embodiments, the variable region b comprises a heavy chain variable region b (VHb) comprising a VHb CDR1, a VHb CDR2 and a VHb CDR3, wherein the VHb CDR1 comprises an amino acid sequence set forth in any one of SEQ ID NOs: 21-23; the VHb CDR2 comprises an amino acid sequence set forth in any one of SEQ ID NOs: 24-28; and the VHb CDR3 comprises an amino acid sequence set forth in any one of SEQ ID NOs: 29-35.

In some embodiments, the variable region b comprises a light chain variable region b (VLb) comprising a VLb CDR1, a VLb CDR2 and a VLb CDR3, wherein the VLb CDR1 comprises an amino acid sequence set forth in any one of SEQ ID NOs: 36-40; the VLb CDR2 comprises an amino acid sequence set forth in any one of SEQ ID NOs: 41-44; and the VLb CDR3 comprises an amino acid sequence set forth in any one of SEQ ID NOs: 45-48.

In some embodiments, the variable region b comprises a VHb and a VLb, wherein the VHb comprises a VHb CDR1, a VHb CDR2 and a VHb CDR3; and the VLb comprises a VLb CDR1, a VLb CDR2 and a VLb CDR3, wherein the VHb CDR1 comprises an amino acid sequence set forth in any one of SEQ ID NOs: 21-23; the VHb CDR2 comprises an amino acid sequence set forth in any one of SEQ ID NOs: 24-28; the VHb CDR3 comprises an amino acid sequence set forth in any one of SEQ ID NOs: 29-35; the VLb CDR1 comprises an amino acid sequence set forth in any one of SEQ ID NOs: 36-40; the VLb CDR2 comprises an amino acid sequence set forth in any one of SEQ ID NOs: 41-44; and the VLb CDR3 comprises an amino acid sequence set forth in any one of SEQ ID NOs: 45-48.

In some embodiments, the VHb CDR1 comprises an amino acid sequence set forth in SEQ ID NO: 21 or an amino acid sequence having one or more conservative amino acid substitutions as compared with the amino acid sequence set forth in SEQ ID NO: 21; the VHb CDR2 comprises an amino acid sequence set forth in SEQ ID NO: 24 or an amino acid sequence having one or more conservative amino acid substitutions as compared with the amino acid sequence set forth in SEQ ID NO: 24; and the VHb CDR3 comprises an amino acid sequence set forth in SEQ ID NO: 29 or an amino acid sequence having one or more conservative amino acid substitutions as compared with the amino acid sequence set forth in SEQ ID NO: 29.

In some embodiments, the VLb CDR1 comprises an amino acid sequence set forth in SEQ ID NO: 36 or an amino acid sequence having one or more conservative amino acid substitutions as compared with the amino acid sequence set forth in SEQ ID NO: 36; the VLb CDR2 comprises an amino acid sequence set forth in SEQ ID NO: 41 or an amino acid sequence having one or more conservative amino acid substitutions as compared with the amino acid sequence set forth in SEQ ID NO: 41; and the VLb CDR3 comprises an amino acid sequence set forth in SEQ ID NO: 45 or an amino acid sequence having one or more conservative amino acid substitutions as compared with the amino acid sequence set forth in SEQ ID NO: 45.

In some embodiments, the VHb CDR1 comprises an amino acid sequence set forth in SEQ ID NO: 21 or an amino acid sequence having one or more conservative amino acid substitutions as compared with the amino acid sequence set forth in SEQ ID NO: 21; the VHb CDR2 comprises an amino acid sequence set forth in SEQ ID NO: 24 or an amino acid sequence having one or more conservative amino acid substitutions as compared with the amino acid sequence set forth in SEQ ID NO: 24; the VHb CDR3 comprises an amino acid sequence set forth in SEQ ID NO: 29 or an amino acid sequence having one or more conservative amino acid substitutions as compared with the amino acid sequence set forth in SEQ ID NO: 29; the VLb CDR1 comprises an amino acid sequence set forth in SEQ ID NO: 36 or an amino acid sequence having one or more conservative amino acid substitutions as compared with the amino acid sequence set forth in SEQ ID NO: 36; the VLb CDR2 comprises an amino acid sequence set forth in SEQ ID NO: 41 or an amino acid sequence having one or more conservative amino acid substitutions as compared with the amino acid sequence set forth in SEQ ID NO: 41; and the VLb CDR3 comprises an amino acid sequence set forth in SEQ ID NO: 45 or an amino acid sequence having one or more conservative amino acid substitutions as compared with the amino acid sequence set forth in SEQ ID NO: 45.

In some embodiments, the VHb comprises an amino acid sequence set forth in any one of SEQ ID NOs: 64-74, or an amino acid sequence having at least 90% homology to the amino acid sequence set forth in any one of SEQ ID NOs: 64-74, or an amino acid sequence having one or more conservative amino acid substitutions as compared with the amino acid sequence set forth in any one of SEQ ID NOs: 64-74.

In some embodiments, the VHb comprises an amino acid sequence set forth in SEQ ID NO: 64, or an amino acid sequence having at least 90% homology to the amino acid sequence set forth in SEQ ID NO: 64, or an amino acid sequence having one or more conservative amino acid substitutions as compared with the amino acid sequence set forth in SEQ ID NO: 64.

In some embodiments, the VLb comprises an amino acid sequence set forth in any one of SEQ ID NOs: 75-80, or an amino acid sequence having at least 90% homology to the amino acid sequence set forth in any one of SEQ ID NOs: 75-80, or an amino acid sequence having one or more conservative amino acid substitutions as compared with the amino acid sequence set forth in any one of SEQ ID NOs: 75-80.

In some embodiments, the VLb comprises an amino acid sequence set forth in SEQ ID NO: 75, or an amino acid sequence having at least 90% homology to the amino acid sequence set forth in SEQ ID NO: 75, or an amino acid sequence having one or more conservative amino acid substitutions as compared with the amino acid sequence set forth in SEQ ID NO: 75.

In some embodiments, the variable region b comprises a VHb and a VLb. The VHb comprises an amino acid sequence set forth in SEQ ID NO: 64, or an amino acid sequence having at least 90% homology to the amino acid sequence set forth in SEQ ID NO: 64, or an amino acid sequence having one or more conservative amino acid substitutions as compared with the amino acid sequence set forth in SEQ ID NO: 64; and the VLb comprises an amino acid sequence set forth in SEQ ID NO: 75, or an amino acid sequence having at least 90% homology to the amino acid sequence set forth in SEQ ID NO: 75, or an amino acid sequence having one or more conservative amino acid substitutions as compared with the amino acid sequence set forth in SEQ ID NO: 75.

In some embodiments, the present invention provides a bispecific antibody or an antigen-binding fragment, wherein the antibody or the antigen-binding fragment further comprises a light chain constant region b (CLb) and a heavy chain constant region b (CHb).

In some embodiments, the CHb comprises an amino acid sequence set forth in any one of SEQ ID NOs: 81 and 83-86, or an amino acid sequence having at least 90% homology to the amino acid sequence set forth in any one of SEQ ID NOs: 81 and 83-86, or an amino acid sequence having one or more conservative amino acid substitutions as compared with the amino acid sequence set forth in any one of SEQ ID NOs: 81 and 83-86.

In some embodiments, the CLb comprises an amino acid sequence set forth in SEQ ID NO: 82, or an amino acid sequence having at least 90% homology to the amino acid sequence set forth in SEQ ID NO: 82, or an amino acid sequence having one or more conservative amino acid substitutions as compared with the amino acid sequence set forth in SEQ ID NO: 82.

In some embodiments, the present invention provides a bispecific antibody or an antigen-binding fragment, wherein the antibody or the antigen-binding fragment comprises a heavy chain b described herein, a heavy chain y, and a light chain b and a light chain y, wherein the heavy chain b is paired with the light chain b to form a CD47 antigen-binding site, and the heavy chain y is paired with the light chain y to form the other antigen-binding site. In some embodiments, the heavy chain b comprises the VHb CDR1, the VHb CDR2 and the VHb CDR3 described herein. In some embodiments, the heavy chain b comprises the VHb described herein. In some embodiments, the heavy chain b comprises the VHb and the CHb described herein. In some embodiments, the light chain b comprises the VLb CDR1, the VLb CDR2 and the VLb CDR3 described herein. In some embodiments, the light chain b comprises the VLb described herein. In some embodiments, the light chain b comprises the VLb and the CLb described herein. In some embodiments, the light chain y is the same as the light chain b.

In some embodiments, the other antigen is PD-L1, PD-1, BCMA, MSLN, CD19, CD20 or VEGF. In some embodiments, the other antigen is PD-L1.

In some embodiments, the CDRs of the heavy chain y are selected from heavy chain CDRs in atezolizumab, avelumab, durvalumab, envafolimab or cosibelimab. In some embodiments, the variable region of the heavy chain y is selected from heavy chain variable regions in atezolizumab, avelumab, durvalumab, envafolimab or cosibelimab. In some embodiments, the CDRs of the light chain y are selected from light chain CDRs in atezolizumab, avelumab, durvalumab, envafolimab or cosibelimab. In some embodiments, the variable region of the light chain is selected from light chain variable regions in atezolizumab, avelumab, durvalumab, envafolimab or cosibelimab. In some embodiments, the heavy chain y is the same as the heavy chain a described herein. In some embodiments, the light chain y is the same as the light chain b.

In some embodiments, the heavy chain b comprises an amino acid sequence set forth in SEQ ID NO: 93 or 95, or an amino acid sequence having at least 90% homology to the amino acid sequence set forth in SEQ ID NO: 93 or 95, or an amino acid sequence having one or more conservative amino acid substitutions as compared with the amino acid sequence set forth in SEQ ID NO: 93 or 95.

In some embodiments, the bispecific antibody or the antigen-binding fragment provided herein has an affinity index KD of ≤ 30 nM for CD47. In some embodiments, the bispecific antibody or the antigen-binding fragment provided herein has an affinity index KD of ≤ 10 nM for CD47. In some embodiments, the bispecific antibody or the antigen-binding fragment provided herein has an affinity index KD of ≤ 5 nM for CD47.

In some embodiments, the bispecific antibody or the antigen-binding fragment provided herein does not cause agglutination of red blood cells.

In another aspect, the present invention provides a bispecific antibody or an antigen-binding fragment, wherein the antibody or the antigen-biding fragment comprises a variable region a specifically binding to PD-L1 and a variable region b specifically binding to CD47.

The variable region a comprises one or more amino acid sequences of (a)-(f):
(a) a VHa CDR1 comprising an amino acid sequence set forth in any one of SEQ ID NOs: 4-8 or an amino acid sequence having one or more conservative amino acid substitutions as compared with the amino acid sequence set forth in any one of SEQ ID NOs: 4-8;
(b) a VHa CDR2 comprising an amino acid sequence set forth in any one of SEQ ID NOs: 9-18 or an amino acid sequence having one or more conservative amino acid substitutions as compared with the amino acid sequence set forth in any one of SEQ ID NOs: 9-18;
(c) a VHa CDR3 comprising an amino acid sequence set forth in SEQ ID NO: 19 or 20 or an amino acid sequence having one or more conservative amino acid substitutions as compared with the amino acid sequence set forth in SEQ ID NO: 19 or 20;
(d) a VLa CDR1 comprising an amino acid sequence set forth in any one of SEQ ID NOs: 36-40 or an amino acid sequence having one or more conservative amino acid substitutions as compared with the amino acid sequence set forth in any one of SEQ ID NOs: 36-40;
(e) a VLa CDR2 comprising an amino acid sequence set forth in any one of SEQ ID NOs: 41-44 or an amino acid sequence having one or more conservative amino acid substitutions as compared with the amino acid sequence set forth in any one of SEQ ID NOs: 41-44; and
(f) a VLa CDR3 comprising an amino acid sequence set forth in any one of SEQ ID NOs: 45-48 or an amino acid sequence having one or more conservative amino acid substitutions as compared with the amino acid sequence set forth in any one of SEQ ID NOs: 45-48.

The variable region b comprises one or more amino acid sequences of (g)-(1):
(g) a VHb CDR1 comprising an amino acid sequence set forth in any one of SEQ ID NOs: 21-23 or an amino acid sequence having one or more conservative amino acid substitutions as compared with the amino acid sequence set forth in any one of SEQ ID NOs: 21-23;
(h) a VHb CDR2 comprising an amino acid sequence set forth in any one of SEQ ID NOs: 24-28 or an amino acid sequence having one or more conservative amino acid substitutions as compared with the amino acid sequence set forth in any one of SEQ ID NOs: 24-28;
(i) a VHb CDR3 comprising an amino acid sequence set forth in any one of SEQ ID NOs: 29-35 or an amino acid sequence having one or more conservative amino acid substitutions as compared with the amino acid sequence set forth in any one of SEQ ID NOs: 29-35;
(j) a VLb CDR1 comprising an amino acid sequence set forth in any one of SEQ ID NOs: 36-40 or an amino acid sequence having one or more conservative amino acid substitutions as compared with the amino acid sequence set forth in any one of SEQ ID NOs: 36-40;
(k) a VLb CDR2 comprising an amino acid sequence set forth in any one of SEQ ID NOs: 41-44 or an amino acid sequence having one or more conservative amino acid substitutions as compared with the amino acid sequence set forth in any one of SEQ ID NOs: 41-44; and
(1) a VLb CDR3 comprising an amino acid sequence set forth in any one of SEQ ID NOs: 45-48 or an amino acid sequence having one or more conservative amino acid substitutions as compared with the amino acid sequence set forth in any one of SEQ ID NOs: 45-48.

In some embodiments, the variable region a comprises a heavy chain variable region a (VHa) comprising a VHa CDR1, a VHa CDR2 and a VHa CDR3, wherein the VHa CDR1 comprises an amino acid sequence set forth in any one of SEQ ID NOs: 4-8, the VHa CDR2 comprises an amino acid sequence set forth in any one of SEQ ID NOs: 9-18, and the VHa CDR3 comprises an amino acid sequence set forth in SEQ ID NO: 19 or 20; and the variable region b comprises a heavy chain variable region b (VHb) comprising a VHb CDR1, a VHb CDR2 and a VHb CDR3, wherein the VHb CDR1 comprises an amino acid sequence set forth in any one of SEQ ID NOs: 21-23, the VHb CDR2 comprises an amino acid sequence set forth in any one of SEQ ID NOs: 24-28, and the VHb CDR3 comprises an amino acid sequence set forth in any one of SEQ ID NOs: 29-35.

In some embodiments, the variable region a comprises a light chain variable region a (VLa) comprising a VLa CDR1, a VLa CDR2 and a VLa CDR3, wherein the VLa CDR1 comprises an amino acid sequence set forth in any one of SEQ ID NOs: 36-40, the VLa CDR2 comprises an amino acid sequence set forth in any one of SEQ ID NOs: 41-44, and the VLa CDR3 comprises an amino acid sequence set forth in any one of SEQ ID NOs: 45-48; and the variable region b comprises a light chain variable region b (VLb) comprising a VLb CDR1, a VLb CDR2 and a VLb CDR3, wherein the VLb CDR1 comprises an amino acid sequence set forth in any one of SEQ ID NOs: 36-40; the VLb CDR2 comprises an amino acid sequence set forth in any one of SEQ ID NOs: 41-44, and the VLb CDR3 comprises an amino acid sequence set forth in any one of SEQ ID NOs: 45-48.

In some embodiments, the variable region a comprises a VHa and a VLa; and the variable region b comprises a VHb and a VLb. The VHa comprises a VHa CDR1, a VHa CDR2 and a VHa CDR3; and the VLa comprises a VLa CDR1, a VLa CDR2 and a VLa CDR3, wherein the VHa CDR1 comprises an amino acid sequence set forth in any one of SEQ ID NOs: 4-8; the VHa CDR2 comprises an amino acid sequence set forth in any one of SEQ ID NOs: 9-18; and the VHa CDR3 comprises an amino acid sequence set forth in SEQ ID NO: 19 or 20. The VHb comprises a VHb CDR1, a VHb CDR2 and a VHb CDR3; and the VLb comprises a VLb CDR1, a VLb CDR2 and a VLb CDR3, wherein the VHb CDR1 comprises an amino acid sequence set forth in any one of SEQ ID NOs: 21-23; the VHb CDR2 comprises an amino acid sequence set forth in any one of SEQ ID NOs: 24-28; the VHb CDR3 comprises an amino acid sequence set forth in any one of SEQ ID NOs: 29-35; the VLb CDR1 comprises an amino acid sequence set forth in any one of SEQ ID NOs: 36-40; the VLb CDR2 comprises an amino acid sequence set forth in any one of SEQ ID NOs: 41-44; and the VLb CDR3 comprises an amino acid sequence set forth in any one of SEQ ID NOs: 45-48. The VLa CDR1 and the VLb CDR1 comprise an amino acid sequence independently selected from an amino acid sequence set forth in any one of SEQ ID NOs: 36-40; the VLa CDR2 and the VLb CDR2 comprise an amino acid sequence independently selected from an amino acid sequence set forth in any one of SEQ ID NOs: 41-44; and the VLa CDR3 and the VLb CDR3 comprise an amino acid sequence independently selected from an amino acid sequence set forth in any one of SEQ ID NOs: 45-48.

In some embodiments, the VHa CDR1 comprises an amino acid sequence set forth in SEQ ID NO: 4 or an amino acid sequence having one or more conservative amino acid substitutions as compared with the amino acid sequence set forth in SEQ ID NO: 4; the VHa CDR2 comprises an amino acid sequence set forth in any one of SEQ ID NOs: 9-14 or an amino acid sequence having one or more conservative amino acid substitutions as compared with the amino acid sequence set forth in any one of SEQ ID NOs: 9-14; the VHa CDR3 comprises an amino acid sequence set forth in SEQ ID NO: 19 or an amino acid sequence having one or more conservative amino acid substitutions as compared with the amino acid sequence set forth in SEQ ID NO: 19; the VHb CDR1 comprises an amino acid sequence set forth in SEQ ID NO: 21 or an amino acid sequence having one or more conservative amino acid substitutions as compared with the amino acid sequence set forth in SEQ ID NO: 21; the VHb CDR2 comprises an amino acid sequence set forth in SEQ ID NO: 24 or an amino acid sequence having one or more conservative amino acid substitutions as compared with the amino acid sequence set forth in SEQ ID NO: 24; and the VHb CDR3 comprises an amino acid sequence set forth in SEQ ID NO: 29 or an amino acid sequence having one or more conservative amino acid substitutions as compared with the amino acid sequence set forth in SEQ ID NO: 29.

In some embodiments, the VHa CDR1 comprises an amino acid sequence set forth in SEQ ID NO: 4 or an amino acid sequence having one or more conservative amino acid substitutions as compared with the amino acid sequence set forth in SEQ ID NO: 4; the VHa CDR2 comprises an amino acid sequence set forth in SEQ ID NO: 14 or an amino acid sequence having one or more conservative amino acid substitutions as compared with the amino acid sequence set forth in SEQ ID NO: 14; the VHa CDR3 comprises an amino acid sequence set forth in SEQ ID NO: 19 or an amino acid sequence having one or more conservative amino acid substitutions as compared with the amino acid sequence set forth in SEQ ID NO: 19; the VHb CDR1 comprises an amino acid sequence set forth in SEQ ID NO: 21 or an amino acid sequence having one or more conservative amino acid substitutions as compared with the amino acid sequence set forth in SEQ ID NO: 21; the VHb CDR2 comprises an amino acid sequence set forth in SEQ ID NO: 24 or an amino acid sequence having one or more conservative amino acid substitutions as compared with the amino acid sequence set forth in SEQ ID NO: 24; and the VHb CDR3 comprises an amino acid sequence set forth in SEQ ID NO: 29 or an amino acid sequence having one or more conservative amino acid substitutions as compared with the amino acid sequence set forth in SEQ ID NO: 29.

In some embodiments, the VLa CDR1 or the VLb CDR1 comprises an amino acid sequence set forth in SEQ ID NO: 36 or an amino acid sequence having one or more conservative amino acid substitutions as compared with the amino acid sequence set forth in SEQ ID NO: 36; the VLa CDR2 or the VLb CDR2 comprises an amino acid sequence set forth in SEQ ID NO: 41 or an amino acid sequence having one or more conservative amino acid substitutions as compared with the amino acid sequence set forth in SEQ ID NO: 41; and the VLa CDR3 or the VLb CDR3 comprises an amino acid sequence set forth in SEQ ID NO: 45 or an amino acid sequence having one or more conservative amino acid substitutions as compared with the amino acid sequence set forth in SEQ ID NO: 45.

In some embodiments, the VHa CDR1 comprises an amino acid sequence set forth in SEQ ID NO: 4 or an amino acid sequence having one or more conservative amino acid substitutions as compared with the amino acid sequence set forth in SEQ ID NO: 4; the VHa CDR2 comprises an amino acid sequence set forth in SEQ ID NO: 14 or an amino acid sequence having one or more conservative amino acid substitutions as compared with the amino acid sequence set forth in SEQ ID NO: 14; the VHa CDR3 comprises an amino acid sequence set forth in SEQ ID NO: 19 or an amino acid sequence having one or more conservative amino acid substitutions as compared with the amino acid sequence set forth in SEQ ID NO: 19; the VHb CDR1 comprises an amino acid sequence set forth in SEQ ID NO: 21 or an amino acid sequence having one or more conservative amino acid substitutions as compared with the amino acid sequence set forth in SEQ ID NO: 21; the VHb CDR2 comprises an amino acid sequence set forth in SEQ ID NO: 24 or an amino acid sequence having one or more conservative amino acid substitutions as compared with the amino acid sequence set forth in SEQ ID NO: 24; and the VHb CDR3 comprises an amino acid sequence set forth in SEQ ID NO: 29 or an amino acid sequence having one or more conservative amino acid substitutions as compared with the amino acid sequence set forth in SEQ ID NO: 29. The VLa CDR1 or the VLb CDR1 comprises an amino acid sequence set forth in SEQ ID NO: 36 or an amino acid sequence having one or more conservative amino acid substitutions as compared with the amino acid sequence set forth in SEQ ID NO: 36; the VLa CDR2 or the VLb CDR2 comprises an amino acid sequence set forth in SEQ ID NO: 41 or an amino acid sequence having one or more conservative amino acid substitutions as compared with the amino acid sequence set forth in SEQ ID NO: 41; and the VLa CDR3 or the VLb CDR3 comprises an amino acid sequence set forth in SEQ ID NO: 45 or an amino acid sequence having one or more conservative amino acid substitutions as compared with the amino acid sequence set forth in SEQ ID NO: 45.

In some embodiments, the VLa CDR1 and the VLb CDR1 comprise a same amino acid sequence.

In some embodiments, the VLa CDR2 and the VLb CDR2 comprise a same amino acid sequence.

In some embodiments, the VLa CDR3 and the VLb CDR3 comprise a same amino acid sequence.

In some embodiments, the VLa CDR1 and the VLb CDR1 comprise a same amino acid sequence, the VLa CDR2 and the VLb CDR2 comprise a same amino acid sequence, and the VLa CDR3 and the VLb CDR3 comprise a same amino acid sequence.

In some embodiments, the variable region a comprises a heavy chain variable region VHa and a light chain variable region VLa, wherein the VHa comprises an amino acid sequence set forth in any one of SEQ ID NOs: 49-63 or an amino acid sequence having at least 90% homology to the amino acid sequence set forth in any one of SEQ ID NOs: 49-63; and/or the VLa comprises an amino acid sequence set forth in any one of SEQ ID NOs: 75-80 or an amino acid sequence having at least 90% homology to the amino acid sequence set forth in any one of SEQ ID NOs: 75-80.

In some embodiments, the variable region a comprises a heavy chain variable region VHa and a light chain variable region VLa, wherein the VHa comprises an amino acid sequence set forth in SEQ ID NO: 54 or an amino acid sequence having at least 90% homology to the amino acid sequence set forth in SEQ ID NO: 54; and/or the VLa comprises an amino acid sequence set forth in SEQ ID NO: 75 or an amino acid sequence having at least 90% homology to the amino acid sequence set forth in SEQ ID NO: 75.

In some embodiments, the variable region b comprises a heavy chain variable region VHb and a light chain variable region VLb; wherein the VHb comprises an amino acid sequence set forth in any one of SEQ ID NOs: 64-74 or an amino acid sequence having at least 90% homology to the amino acid sequence set forth in any one of SEQ ID NOs: 64-74; and/or the VLb comprises an amino acid sequence set forth in any one of SEQ ID NOs: 75-80 or an amino acid sequence having at least 90% homology to the amino acid sequence set forth in any one of SEQ ID NOs: 75-80.

In some embodiments, the variable region b comprises a heavy chain variable region VHb and a light chain variable region VLb, wherein the VHb comprises an amino acid sequence set forth in SEQ ID NO: 64 or an amino acid sequence having at least 90% homology to the amino acid sequence set forth in SEQ ID NO: 64; and/or the VLb comprises an amino acid sequence set forth in SEQ ID NO: 75 or an amino acid sequence having at least 90% homology to the amino acid sequence set forth in SEQ ID NO: 75.

In some embodiments, the VHa comprises an amino acid sequence set forth in any one of SEQ ID NOs: 49-63, or an amino acid sequence having at least 90% homology to the amino acid sequence set forth in any one of SEQ ID NOs: 49-63, or an amino acid sequence having one or more conservative amino acid substitutions as compared with the amino acid sequence set forth in any one of SEQ ID NOs: 49-63; and the VHb comprises an amino acid sequence set forth in any one of SEQ ID NOs: 64-74, or an amino acid sequence having at least 90% homology to the amino acid sequence set forth in any one of SEQ ID NOs: 64-74, or an amino acid sequence having one or more conservative amino acid substitutions as compared with the amino acid sequence set forth in any one of SEQ ID NOs: 64-74.

In some embodiments, the VHa comprises an amino acid sequence set forth in SEQ ID NO: 54, or an amino acid sequence having at least 90% homology to the amino acid sequence set forth in SEQ ID NO: 54, or an amino acid sequence having one or more conservative amino acid substitutions as compared with the amino acid sequence set forth in SEQ ID NO: 54; and the VHb comprises an amino acid sequence set forth in SEQ ID NO: 64, or an amino acid sequence having at least 90% homology to the amino acid sequence set forth in SEQ ID NO: 64, or an amino acid sequence having one or more conservative amino acid substitutions as compared with the amino acid sequence set forth in SEQ ID NO: 64.

In some embodiments, the VLa and the VLb comprise an amino acid sequence independently selected from an amino acid sequence set forth in any one of SEQ ID NOs: 75-80, or an amino acid sequence having at least 90% homology to the amino acid sequence set forth in any one of SEQ ID NOs: 75-80, or an amino acid sequence having one or more conservative amino acid substitutions as compared with the amino acid sequence set forth in any one of SEQ ID NOs: 75-80.

In some embodiments, the VLa and the VLb comprise an amino acid sequence independently selected from an amino acid sequence set forth in SEQ ID NO: 75, or an amino acid sequence having at least 90% homology to the amino acid sequence set forth in SEQ ID NO: 75, or an amino acid sequence having one or more conservative amino acid substitutions as compared with the amino acid sequence set forth in SEQ ID NO: 75.

In some embodiments, the variable region a comprises a VHa and a VLa; and the variable region b comprises a VHb and a VLb. The VHa comprises an amino acid sequence set forth in SEQ ID NO: 54, or an amino acid sequence having at least 90% homology to the amino acid sequence set forth in SEQ ID NO: 54, or an amino acid sequence having one or more conservative amino acid substitutions as compared with the amino acid sequence set forth in SEQ ID NO: 54. The VHb comprises an amino acid sequence set forth in SEQ ID NO: 64, or an amino acid sequence having at least 90% homology to the amino acid sequence set forth in SEQ ID NO: 64, or an amino acid sequence having one or more conservative amino acid substitutions as compared with the amino acid sequence set forth in SEQ ID NO: 64. The VLa or the VLb comprises an amino acid sequence set forth in SEQ ID NO: 75, or an amino acid sequence having at least 90% homology to the amino acid sequence set forth in SEQ ID NO: 75, or an amino acid sequence having one or more conservative amino acid substitutions as compared with the amino acid sequence set forth in SEQ ID NO: 75.

In some embodiments, the VLa and the VLb comprise a same amino acid sequence.

In some embodiments, the antibody or the antigen-binding fragment described herein further comprises a light chain constant region a (CLa), a heavy chain constant region a (CHa), a light chain constant region b (CLb) and a heavy chain constant region b (CHb).

In some embodiments, the CHa is of IgG1 subtype. In some embodiments, the CHb is of IgG1 subtype.

In some embodiments, the CHa and/or the CHb are of IgG1 subtype comprising one or more of the following amino acid mutations: Y349C, S354C, T366W, T366S, L368A and Y407V, wherein amino acid positions are Eu numbered.

In some embodiments, the CHa and/or the CHb is of IgG1 subtype comprising the following amino acid mutation: N297A, wherein amino acid positions are Eu numbered.

In some embodiments, one heavy chain constant region of the CHa and the CHb comprises one or more of the following amino acid mutations: N297A, Y349C, T366S, L368A and Y407V In some embodiments, the other heavy chain constant region of the CHa and the CHb comprises one or more of the following amino acid mutations: N297A, S354C, and T366W.

In some embodiments, the CHa comprises an amino acid sequence set forth in any one of SEQ ID NOs: 81 and 83-86, or an amino acid sequence having at least 90% homology to the amino acid sequence set forth in any one of SEQ ID NOs: 81 and 83-86, or an amino acid sequence having one or more conservative amino acid substitutions as compared with the amino acid sequence set forth in any one of SEQ ID NOs: 81 and 83-86. In some embodiments, the CHb comprises an amino acid sequence set forth in any one of SEQ ID NOs: 81 and 83-86, or an amino acid sequence having at least 90% homology to the amino acid sequence set forth in any one of SEQ ID NOs: 81 and 83-86, or an amino acid sequence having one or more conservative amino acid substitutions as compared with the amino acid sequence set forth in any one of SEQ ID NOs: 81 and 83-86.

In some embodiments, the CHa comprises an amino acid sequence set forth in SEQ ID NO: 81, 83 or 85, or an amino acid sequence having at least 90% homology to the amino acid sequence set forth in SEQ ID NO: 81, 83 or 85, or an amino acid sequence having one or more conservative amino acid substitutions as compared with the amino acid sequence set forth in SEQ ID NO: 81, 83 or 85.

In some embodiments, the CHb comprises an amino acid sequence set forth in SEQ ID NO: 81, 84 or 86, or an amino acid sequence having at least 90% homology to the amino acid sequence set forth in SEQ ID NO: 81, 84 or 86, or an amino acid sequence having one or more conservative amino acid substitutions as compared with the amino acid sequence set forth in SEQ ID NO: 81, 84 or 86.

In some embodiments, the CHa comprises an amino acid sequence set forth in SEQ ID NO: 83, and the CHb comprises an amino acid sequence set forth in SEQ ID NO: 84. In some embodiments, the CHa comprises an amino acid sequence set forth in SEQ ID NO: 85, and the CHb comprises an amino acid sequence set forth in SEQ ID NO: 86. In some embodiments, the CHa comprises an amino acid sequence set forth in SEQ ID NO: 84, and the CHb comprises an amino acid sequence set forth in SEQ ID NO: 83. In some embodiments, the CHa comprises an amino acid sequence set forth in SEQ ID NO: 86, and the CHb comprises an amino acid sequence set forth in SEQ ID NO: 85.

In some embodiments, the CLa comprises an amino acid sequence set forth in SEQ ID NO: 82, or an amino acid sequence having at least 90% homology to the amino acid sequence set forth in SEQ ID NO: 82, or an amino acid sequence having one or more conservative amino acid substitutions as compared with the amino acid sequence set forth in SEQ ID NO: 82. In some embodiments, the CLb comprises an amino acid sequence set forth in SEQ ID NO: 82, or an amino acid sequence having at least 90% homology to the amino acid sequence set forth in SEQ ID NO: 82, or an amino acid sequence having one or more conservative amino acid substitutions as compared with the amino acid sequence set forth in SEQ ID NO: 82.

In some embodiments, the heavy chain a comprises an amino acid sequence set forth in SEQ ID NO: 92 or 94, or an amino acid sequence having at least 90% homology to the amino acid sequence set forth in SEQ ID NO: 92 or 94, or an amino acid sequence having one or more conservative amino acid substitutions as compared with the amino acid sequence set forth in SEQ ID NO: 92 or 94; and the heavy chain b comprises an amino acid sequence set forth in SEQ ID NO: 93 or 95, or an amino acid sequence having at least 90% homology to the amino acid sequence set forth in SEQ ID NO: 93 or 95, or an amino acid sequence having one or more conservative amino acid substitutions as compared with the amino acid sequence set forth in SEQ ID NO: 93 or 95. In some embodiments, the light chain a and the light chain b both comprise an amino acid sequence set forth in SEQ ID NO: 96 or an amino acid sequence having at least 90% homology to the amino acid sequence set forth in SEQ ID NO: 96.

In some embodiments, the bispecific antibody or the antigen-binding fragment provided herein has an affinity index KD of ≤ 10 nM for PD-L1. In some embodiments, the bispecific antibody or the antigen-binding fragment provided herein has an affinity index KD of ≤ 1 nM for PD-L1. In some embodiments, the bispecific antibody or the antigen-binding fragment provided herein has an affinity index KD of ≤ 30 nM for CD47. In some embodiments, the bispecific antibody or the antigen-binding fragment provided herein has an affinity index KD of ≤ 10 nM for CD47. In some embodiments, the bispecific antibody or the antigen-binding fragment provided herein has an affinity index KD of ≤ 5 nM for CD47.

In another aspect, the present invention provides a bispecific antibody or an antigen-binding fragment, wherein the antibody or the antigen-binding fragment comprises a variable region a specifically binding to PD-L1, a variable region b specifically binding to CD47, a light chain constant region and a heavy chain constant region, wherein the heavy chain constant region is of IgG1 subtype, or IgG1 subtype comprising one or more of the following amino acid mutations: Y349C, S354C, T366W, T366S, L368A and Y407V, wherein amino acid positions are Eu numbered.

In some embodiments, the heavy chain constant region is further of IgG1 subtype comprising the following amino acid mutation in which amino acid positions are Eu numbered: N297A. In some embodiments, the heavy chain constant region comprises a first heavy chain constant region and a second heavy chain constant region, wherein the first heavy chain constant region comprises one or more of the following amino acid mutations:
N297A, S354C and T366W; and/or
the second heavy chain constant region comprises one or more of the following amino acid mutations:
   N297A, Y349C, T366S, L368A and Y407V

In some embodiments, the first heavy chain constant region comprises an amino acid sequence set forth in SEQ ID NO: 81, 83 or 85 or an amino acid sequence having at least 90% homology to the amino acid sequence set forth in SEQ ID NO: 81, 83 or 85; and/or the second heavy chain constant region comprises an amino acid sequence set forth in SEQ ID NO: 81, 84 or 86 or an amino acid sequence having at least 90% homology to the amino acid sequence set forth in SEQ ID NO: 81, 84 or 86.

In some embodiments, the VHa in the variable region a is linked to the first heavy chain constant region, and the VHb in the variable region b is linked to the second heavy chain constant region; or, the VHa in the variable region a is linked to the second heavy chain constant region, and the VHb in the variable region b is linked to the first heavy chain constant region.

In some embodiments, the light chain constant region comprises an amino acid sequence set forth in SEQ ID NO: 82 or an amino acid sequence having at least 90% homology to the amino acid sequence set forth in SEQ ID NO: 82.

In some embodiments, the antibody or the antigen-binding fragment comprises the variable region a described herein and the variable region b described herein.

In another aspect, the present invention provides a bispecific antibody or an antigen-binding fragment, wherein the antibody or the antigen-binding fragment comprises a heavy chain a, a heavy chain b and two identical light chains.

In some embodiments, the variable region (VL) of the light chain comprises a VL CDR1, a VL CDR2 and a VL CDR3; wherein the VL CDR1 comprises an amino acid sequence set forth in any one of SEQ ID NOs: 36-40, the VL CDR2 comprises an amino acid sequence set forth in any one of SEQ ID NOs: 41-44, and the VL CDR3 comprises an amino acid sequence set forth in any one of SEQ ID NOs: 45-48.

In some embodiments, the VL CDR1 comprises an amino acid sequence set forth in SEQ ID NO: 36 or an amino acid sequence having one or more conservative amino acid substitutions as compared with the amino acid sequence set forth in SEQ ID NO: 36; the VL CDR2 comprises an amino acid sequence set forth in SEQ ID NO: 41 or an amino acid sequence having one or more conservative amino acid substitutions as compared with the amino acid sequence set forth in SEQ ID NO: 41; and the VL CDR3 comprises an amino acid sequence set forth in SEQ ID NO: 45 or an amino acid sequence having one or more conservative amino acid substitutions as compared with the amino acid sequence set forth in SEQ ID NO: 45.

In some embodiments, the VL comprises an amino acid sequence set forth in any one of SEQ ID NOs: 75-80, or an amino acid sequence having at least 90% homology to the amino acid sequence set forth in any one of SEQ ID NOs: 75-80, or an amino acid sequence having one or more conservative amino acid substitutions as compared with the amino acid sequence set forth in any one of SEQ ID NOs: 75-80.

In some embodiments, the VL comprises an amino acid sequence set forth in SEQ ID NO: 75, or an amino acid sequence having at least 90% homology to the amino acid sequence set forth in SEQ ID NO: 75, or an amino acid sequence having one or more conservative amino acid substitutions as compared with the amino acid sequence set forth in SEQ ID NO: 75.

In some embodiments, the light chain comprises an amino acid sequence set forth in SEQ ID NO: 96, or an amino acid sequence having at least 90% homology to the amino acid sequence set forth in SEQ ID NO: 96, or an amino acid sequence having one or more conservative amino acid substitutions as compared with the amino acid sequence set forth in SEQ ID NO: 96.

In some embodiments, the heavy chain a is paired with one of the light chains to form a PD-L1 antigen-binding site, and the heavy chain b is paired with the other of the light chains to form a CD47, LAG3, TGFβ, CTLA-4, 4-1BB, PD-1, TIGIT, KIR, c-Met, VISTA or BCMA antigen-binding site. In some embodiments, the heavy chain a is paired with one of the light chains to form a PD-L1, PD-1, BCMA, MSLN, CD19, CD20 or VEGF antigen-binding site, and the heavy chain b is paired with the other of the light chains to form a CD47 antigen-binding site. In some embodiments, the heavy chain a is paired with one of the light chains to form a PD-L1 antigen-binding site, and the heavy chain b is paired with the other of the light chains to form a CD47 antigen-binding site.

In some embodiments, the heavy chain a comprises an amino acid sequence set forth in SEQ ID NO: 92 or 94 or an amino acid sequence having at least 90% homology to the amino acid sequence set forth in SEQ ID NO: 92 or 94; the heavy chain b comprises an amino acid sequence set forth in SEQ ID NO: 93 or 95 or an amino acid sequence having at least 90% homology to the amino acid sequence set forth in SEQ ID NO: 93 or 95; and the light chains comprise an amino acid sequence set forth in SEQ ID NO: 96 or an amino acid sequence having at least 90% homology to the amino acid sequence set forth in SEQ ID NO: 96.

In some embodiments, the antibody or the antigen-binding fragment has an affinity index KD of ≤ 1 nM for PD-L1.

In some embodiments, the antibody or the antigen-binding fragment has an affinity index KD of ≤ 10 nM for CD47.

In some embodiments, the antibody or the antigen-binding fragment has an affinity index KD of ≤ 30 nM for CD47.

In another aspect, the present invention provides a polynucleotide encoding the antibody or the antigen-binding fragment described above. In some embodiments, the polynucleotide is an isolated polynucleotide.

In another aspect, the present invention provides an expression vector comprising the polynucleotide described above. In some embodiments, the expression vector is an isolated expression vector.

In another aspect, the present invention provides a host cell comprising the polynucleotide described above or the expression vector described above. In some embodiments, the host cell is an isolated host cell. In some embodiments, the cell is a CHO cell, a HEK cell (e.g., a HEK293F cell), a BHK cell, a Cos1 cell, a Cos7 cell, a CV1 cell or a murine L cell.

In another aspect, the present invention provides a method for preparing the antibody or the antigen-binding fragment described herein, which comprises culturing the host cell described above in a medium to produce the antibody or the antigen-binding fragment. In some embodiments, the method further comprises isolating the antibody or the antigen-binding fragment from the host cell or the medium.

In another aspect, the present invention provides a composition comprising the antibody or the antigen-binding fragment described above, the polynucleotide described above, the expression vector described above or the cell described above, and a pharmaceutically acceptable carrier. In another aspect, the present invention provides use of the antibody or the antigen-binding fragment described above, the polynucleotide described above, the expression vector described above, the cell described above or the composition described above in the manufacture of a medicament for treating a disease.

In some embodiments, the disease is an autoimmune disease, an acute and chronic inflammatory disease, an infectious disease (e.g., chronic infectious disease or sepsis) or cancer. In some embodiments, the disease is a disorder associated with PD-L1 and CD47. In some embodiments, the disease is a disorder associated with PD-L1 and CD47, including but not limited to various blood diseases and solid tumors, such as acute myeloid leukemia (AML), chronic myeloid leukemia, acute lymphocytic leukemia (ALL), non-Hodgkin's lymphoma (NHL), multiple myeloma (MM), lymphoma, breast cancer, gastric cancer, lung cancer, esophageal cancer, intestinal cancer, ovarian cancer, cervical cancer, renal cancer, pancreatic cancer, bladder cancer, neuroglioma, melanoma and other solid tumors. In one embodiment, the cancer is gastrointestinal cancer, such as colon cancer.

In another aspect, the present invention provides a method for treating a disease, which comprises administering to a patient in need thereof an effective dose of the bispecific antibody or the antigen-binding fragment described above, the polynucleotide described above, the expression vector described above, the cell described above or the composition described above.

In some embodiments, the disease is an autoimmune disease, an acute and chronic inflammatory disease, an infectious disease (e.g., chronic infectious disease or sepsis) or cancer. In some embodiments, the disease is a disorder associated with PD-L1 and CD47. In some embodiments, the disease is a disorder associated with PD-L1 and CD47, including but not limited to various blood diseases and solid tumors, such as acute myeloid leukemia (AML), chronic myeloid leukemia, acute lymphocytic leukemia (ALL), non-Hodgkin's lymphoma (NHL), multiple myeloma (MM), lymphoma, breast cancer, gastric cancer, lung cancer, esophageal cancer, intestinal cancer, ovarian cancer, cervical cancer, renal cancer, pancreatic cancer, bladder cancer, neuroglioma, melanoma and other solid tumors. In one embodiment, the cancer is gastrointestinal cancer, such as colon cancer.

In some embodiments, the present invention relates to a kit or an article of manufacture, which comprises the bispecific antibody or the antigen-binding fragment described above, the polynucleotide described above or the expression vector described above.

The present invention provides a bispecific antibody or an antigen-binding fragment and use thereof, and the bispecific antibody or the antigen-binding fragment of the present invention can bind to two or more antigens or two or more epitopes of a same antigen. The bispecific antibody or the antigen-binding fragment of the present invention can be used to treat or prevent various diseases, such as autoimmune diseases, acute and chronic inflammatory diseases, infectious diseases and cancers, and can also be used for diagnosis and prognosis of related diseases.

### BRIEF DESCRIPTION OF THE DRAWINGS

FIG. 1 illustrates the structure of a bispecific antibody of the present invention.
FIG. 2 is a set of graphs. FIG. 2A and FIG. 2B show the purities of two anti-PD-L1/CD47 bispecific antibodies BsAb-46 and BsAb-71, respectively, as detected by SEC.
FIG. 3 shows the binding avidity of anti-PD-L1 scFvs to PD-L1-His-Biotin antigen protein, where the clone number indicates a positive yeast clone expressing the corresponding anti-PD-L1 scFv.
FIG. 4 shows the effect of the anti-PD-L1 antibodies consisting of the light chain R2-4 and the different anti-PD-L1 VH and the negative control IgG1 antibody (IgG-Isotype) on PD-1/PD-L1 signal transduction pathway by the MOA-based assay.
FIG. 5 shows the effect of the anti-PD-L1 antibodies with different light chains and the negative control IgG1 antibody (IgG-Isotype) on PD-1/PD-L1 signal transduction pathway by the MOA-based assay.
FIG. 6 shows the effect of the anti-CD47 antibodies with different light chains, the positive control antibody Hu5F9-G4 and the control IgG1 antibody (IgG-Isotype) on red blood cells agglutination, where Hu5F9 represents Hu5F9-G4. If the appearance shows that the red blood cell cluster flows in a line, it means no hemagglutination is caused; if the cluster forms a point, it means slight agglutination; if the whole well is completely blurred, it means significant agglutination.
FIG. 7 shows the ability of the anti-CD47 antibodies and the positive control antibody Hu5F9-G4 to promote phagocytosis of tumor cells by macrophages, where Hu5F9 represents Hu5F9-G4.
FIG. 8 is a set of graphs showing the binding of anti-PD-L1/CD47 bispecific antibodies to PDL1 and CD47. In FIG. 8A, the antibodies bind to PD-L1-His then to CD47-His; in FIG. 8B, the antibodies bind to CD47-His then to PD-L1-His; FIG. 8C shows the binding of anti-PD-L1/CD47 bispecific antibody BsAb-71-N297A, with the upper part of the figure showing first binding to PD-L1-His and then to CD47-His, and the lower part showing first binding to CD47-His and then to PD-L1-His; in the figure, hPDL1-His represents PD-L1-His, and hCD47-His represents CD47-His.

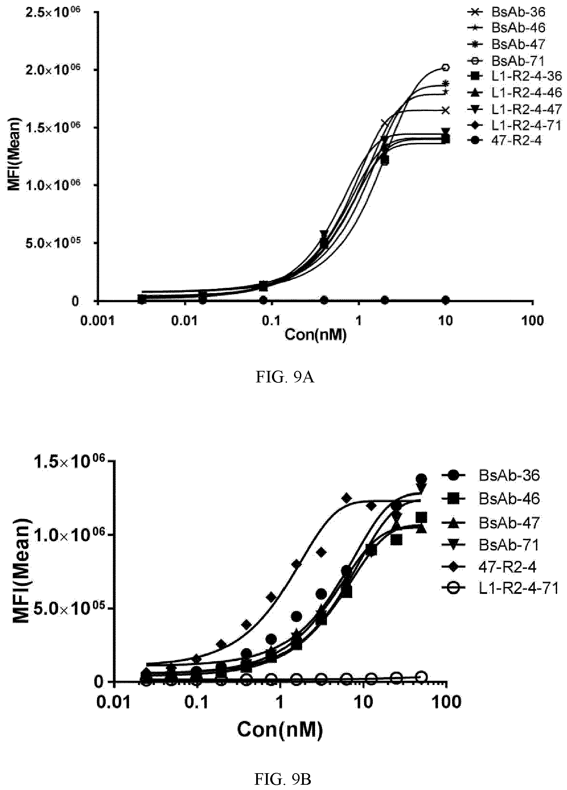

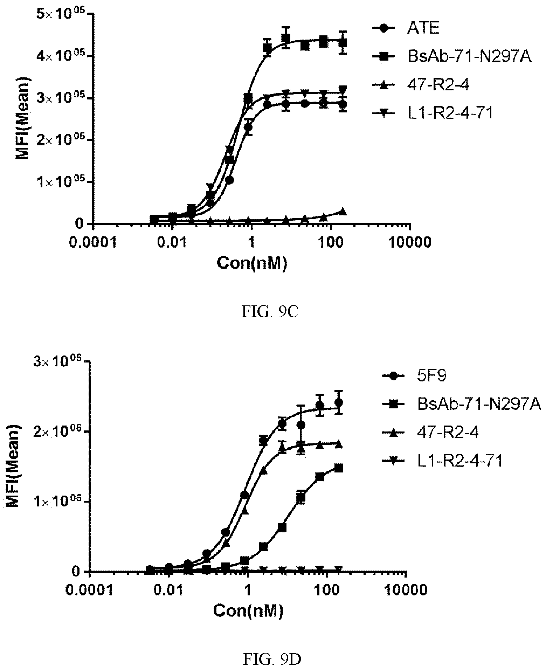

FIG. 9 is a set of graphs. FIGs. 9A and 9C show the binding of the exemplary anti-PD-L1/CD47 bispecific antibodies, the parental anti-PD-L1 antibodies and the anti-CD47 antibody 47-R2-4 as the negative control to CHO cells overexpressing PD-L1, measured by FACS. FIGs. 9B and 9D show the binding of the exemplary anti-PD-L1/CD47 bispecific antibodies, the parental anti-CD47 antibody 47-R2-4 and the anti-PD-L1 antibody L1-R2-4-71 as the negative control to CHO cells overexpressing CD47, measured by FACS. In the figures, the horizontal axis represents the antibody concentration, and the vertical axis represents the mean fluorescence intensity (MFI).
FIG. 10 is a set of graphs showing the effect of the exemplary anti-PD-L1/CD47 bispecific antibodies of the present invention, the parental anti-PD-L1 antibody L1-R2-4-71 and the IgG1 antibody (IgG-Isotype) as the negative control on PD-1/PD-L1 signal transduction pathway measured by a MOA assay. FIG. 10A shows the results for the anti-PD-L1/CD47 bispecific antibodies BsAb-36, BsAb-46, BsAb-47 and BsAb-71, and FIG. 10B shows the results for the anti-PD-L1/CD47 bispecific antibody BsAb-71-N297A.
FIG. 11 shows the effect of the exemplary anti-PD-L1/CD47 bispecific antibodies, the anti-CD47 positive antibody Hu5F9-G4, the parental anti-CD47 antibody 47-R2-4 and the IgG1 control antibody (IgG-Isotype) on erythrocyte agglutination, where Hu5F9 represents Hu5F9-G4.
FIG. 12 shows the ability of the exemplary anti-PD-L1/CD47 bispecific antibodies, the parental anti-CD47 antibody 47-R2-4 and the IgG1 control antibody (IgG-Isotype) to promote phagocytosis of tumor cells by macrophages.

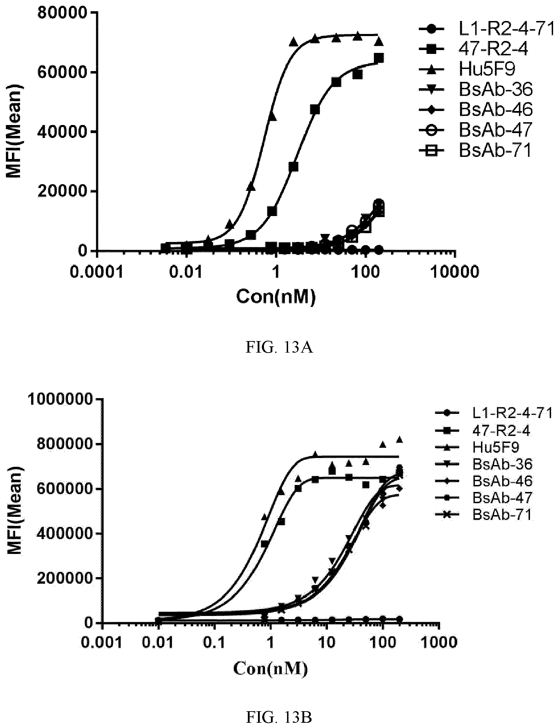

FIG. 13 is a set of graphs. FIG. 13A shows the binding of the exemplary anti-PD-L1/CD47 bispecific antibodies, the anti-CD47 positive antibody Hu5F9-G4, the parental anti-CD47 antibody 47-R2-4 and the anti-PD-L1 antibody L1-R2-4-71 as the negative control to human red blood cells as detected by FACS. FIG. 13B shows the binding of the exemplary anti-PD-L1/CD47 bispecific antibodies, the anti-CD47 positive antibody Hu5F9-G4, the parental anti-CD47 antibody 47-R2-4 and the anti-PD-L1 antibody L1-R2-4-71 as the negative control to human T-lymphocyte leukemia Jurkat cells as detected by FACS. In the figures, the horizontal axis represents the antibody concentration, and the vertical axis represents the mean fluorescence intensity (MFI).
FIG. 14 shows the effect of anti-PD-L1/CD47 bispecific antibodies on IL2 cytokine release from PBMCs under exogenous stimulation.
FIG. 15 shows tumor suppressive activities of the anti-PD-L1 antibody L1-R2-4-71, the anti-CD47 antibody 47-R2-4, the anti-PD-L1 antibody L1-R2-4-71 in combination with the anti-CD47 antibody 47-R2-4 and the anti-PD-L1/CD47 bispecific antibody BsAb-71-N297Ain the MC38-hCD47 (Tg)/C57BL/6-hSIRPα mouse models compared with the IgG1 control antibody.

### DETAILED DESCRIPTION

Unless otherwise stated, each of the following terms shall have the meaning described below. Unless otherwise stated herein, amino acid residues in the constant regions are numbered according to the EU numbering scheme described in, for example, Kabat et al., Sequences of Proteins of Immunological Interes, 5th Edition, Public Health Service, National Institutes of Health, Bethesda, MD, 1991.

### Definitions

It should be noted that the term "an" entity refers to one or more of the entities. For example, "an antibody" should be interpreted as one or more antibodies. As such, the terms "a" (or "an"), "one or more" and "at least one" can be used interchangeably herein.

"About" or "approximately" refers to a general error range for corresponding values as readily understood by those skilled in the relevant art. In some embodiments, "about" or "approximately" mentioned herein refers to the numerical value described as well as its ranges of ±10%, ±5%, ±1% or ±0.1%.

The term "polypeptide" is intended to encompass both the singular form "polypeptide" and the plural form "polypeptides", and refers to a molecule consisting of amino acid monomers linearly linked by amide bonds (also known as peptide bonds). The term "polypeptide" refers to any single chain or multiple chains of two or more amino acids and does not refer to a specific length of the product. Thus, included within the definition of "polypeptide" are peptides, dipeptides, tripeptides, oligopeptides, "proteins", "amino acid chains", or any other term used to refer to chains of two or more amino acids, and the term "polypeptide" may be used in place of, or interchangeably with, any of the above terms. The term "polypeptide" is also intended to refer to a product of post-expression modification of a polypeptide, including but not limited to glycosylation, acetylation, phosphorylation, amidation, derivatization by known protecting/blocking groups, proteolytic cleavage or non-naturally occurring amino acid modification. The polypeptide may be derived from a natural biological source or produced by recombinant techniques, but it is not necessarily translated from a specified nucleic acid sequence. It may be produced in any manner, including chemical synthesis.

"Amino acid" refers to an organic compound containing both an amino group and a carboxyl group, such as an α-amino acid that can be encoded by a nucleic acid, either directly or in the form of a precursor. A single amino acid is encoded by a nucleic acid consisting of three nucleotides (so-called codons or base triplets). Each amino acid is encoded by at least one codon. The encoding of the same amino acid by different codons is known as "degeneration of the genetic code". Amino acids include natural amino acids and non-natural amino acids. Natural amino acids include alanine (three-letter code: Ala, one-letter code: A), arginine (Arg, R), asparagine (Asn, N), aspartic acid (Asp, D), cysteine (Cys, C), glutamine (Gln, Q), glutamic acid (Glu, E), glycine (Gly, G), histidine (His, H), isoleucine (Ile, I ), leucine (Leu, L), lysine (Lys, K), methionine (Met, M), phenylalanine (Phe, F), proline (Pro, P), serine (Ser, S), threonine (Thr, T), tryptophan (Trp, W), tyrosine (Tyr, Y) and valine (Val, V).

"Conservative amino acid substitution" refers to the substitution of one amino acid residue with another amino acid residue having a side chain (R group) with similar chemical properties (e.g., charge or hydrophobicity). Generally, conservative amino acid substitutions do not substantially alter the functional properties of the protein. Examples of groups of amino acids that have side chains with similar chemical properties include: 1) aliphatic side chains: glycine, alanine, valine, leucine and isoleucine; 2) aliphatic hydroxyl side chains: serine and threonine; 3) amide-containing side chains: asparagine and glutamine; 4) aromatic side chains: phenylalanine, tyrosine and tryptophan; 5) basic side chains: lysine, arginine and histidine; and 6) acidic side chains: aspartic acid and glutamic acid.

The term "isolated" as used herein with respect to cells, nucleic acids, polypeptides, antibodies and the like, e.g., "isolated" DNA, RNA, polypeptides, or antibodies, refers to molecules that are separated from one or more other components, e.g., DNA or RNA, in the natural environment of the cell. The term "isolated" as used herein also refers to nucleic acids or peptides that are substantially free of cellular materials, viral materials or cell media when produced by recombinant DNA techniques, or free of chemical precursors or other chemicals when chemically synthesized. In addition, "isolated nucleic acid" is intended to include nucleic acid fragments that do not and will not occur in nature. The term "isolated" is also used herein to refer to cells or polypeptides that are separated from other cellular proteins or tissues. Isolated polypeptides are intended to include both purified and recombinant polypeptides. Isolated polypeptides, antibodies and the like are usually prepared by at least one purification step. In some embodiments, the purity of the isolated nucleic acids, polypeptides, antibodies, etc. is at least about 50%, about 60%, about 70%, about 80%, about 90%, about 95%, about 99%, or a range between any two of these values (inclusive) or any value therein.

The term "recombinant", with regard to a polypeptide or polynucleotide, is intended to refer to a polypeptide or polynucleotide that does not occur in nature, and non-limiting examples can be combined to produce a polynucleotide or polypeptide that does not normally occur.

"Homology", "identity" or "similarity" refers to sequence similarity between two peptides or between two nucleic acid molecules. Homology can be determined by comparing the positions that can be aligned in the sequences. When a position of the compared sequences is occupied by the same base or amino acid, then the molecules are homologous at that position. The degree of homology between the sequences is a function of the number of matching or homologous positions shared by the sequences.

"At least 80% identity" refers to about 80% identity, about 81% identity, about 82% identity, about 83% identity, about 85% identity, about 86% identity, about 87% identity, about 88% identity, about 90% identity, about 91% identity, about 92% identity, about 94% identity, about 95% identity, about 98% identity, about 99% identity, or a range between any two of these values (inclusive) or any value therein.

"At least 90% identity" refers to about 90% identity, about 91% identity, about 92% identity, about 93% identity, about 95% identity, about 96% identity, about 97% identity, about 98% identity, about 99% identity, or a range between any two of these values (inclusive) or any value therein.

A polynucleotide or a polynucleotide sequence (or a polypeptide or an antibody sequence) having a certain percentage (e.g., 90%, 95%, 98% or 99%) of "identity or sequence identity" to another sequence means that when the sequences are aligned, the percentage of bases (or amino acids) in the sequences are the same. This alignment and identity percentage or sequence identity can be determined using visual inspection or software programs known in the art, such as the software programs described in Ausubel et al. eds., (2007), Current Protocols in Molecular Biology*.* Preferably, the alignment is performed using default parameters. One alignment program is BLAST using default parameters, such as BLASTN and BLASTP, both using the following default parameters: Genetic code=standard; filter=none; strand=both; cutoff=60; expect=10; Matrix=BLOSUM62; Descriptions=50 sequences; sort by=HIGHSCORE; Databases=non-redundant; GenBank+EMBL+DDBJ+PDB+GenBank CDS translations+Swi ss Protein+SPupdate+PIR. Biologically equivalent polynucleotides are polynucleotides having the above-specified percentage identity and encoding polypeptides having identical or similar biological activity.

A polynucleotide consists of a specific sequence of four nucleotide bases: adenine (A), cytosine (C), guanine (G) and thymine (T)/uracil (U, instead of thymine when the polynucleotide is RNA). A "polynucleotide sequence" can be a letter representation of a polynucleotide molecule. The letter representation can be input into a database in a computer with a central processing unit and used for bioinformatics applications, such as functional genomics and homology searches.

The terms "polynucleotide" and "oligonucleotide" are used interchangeably to refer to a polymeric form of nucleotides of any length, whether deoxyribonucleotides or ribonucleotides or analogs thereof. The polynucleotide may have any three-dimensional structure and may perform any function, known or unknown. The following are non-limiting examples of polynucleotides: genes or gene fragments (such as probes, primers, EST or SAGE tags), exons, introns, messenger RNA (mRNA), transfer RNA, ribosomal RNA, ribozymes, cDNA, dsRNA, siRNA, miRNA, recombinant polynucleotides, branched polynucleotides, plasmids, vectors, isolated DNA of any sequence, isolated RNA, nucleic acid probes and primers of any sequence, Polynucleotides can include modified nucleotides, such as methylated nucleotides and nucleotide analogs. If present, structural modifications to the nucleotide can be made before or after the assembly of the polynucleotide. The sequence of nucleotides can be interrupted by non-nucleotide components. The polynucleotide may be further modified after polymerization, such as by conjugation with a labeling component. This term also refers to double-stranded and single-stranded molecules. Unless otherwise stated or required, examples of any polynucleotide disclosed herein include a double-stranded form and each of the two complementary single-stranded forms known or predicted to constitute the double-stranded form.

The term "encoding" as applied to a polynucleotide refers to a polynucleotide known to "encode" a polypeptide. When in its native state or manipulated by methods well known to those skilled in the art, the polynucleotide can be transcribed and/or translated to produce the polypeptide and/or a fragment thereof.

"Antibody" or "antigen-binding fragment" refers to a polypeptide or polypeptide complex that specifically recognizes and binds to an antigen. The antibody may be an intact antibody and any antigen-binding fragment or single chain thereof. The term "antibody" thus includes any protein or peptide comprising, in its molecule, at least a portion of an immunoglobulin molecule that has biological activity for binding to an antigen. The antibody and the antigen-binding fragment include, but are not limited to, complementarity determining regions (CDRs) of a heavy or light chain or a ligand binding portion thereof, heavy chain variable regions (VHs), light chain variable regions (VLs), heavy chain constant regions (CHs), light chain constant regions (CLs), framework regions (FRs) or any portion thereof, or at least a portion of a binding protein. The CDRs include light chain CDRs (VL CDR1-3) and heavy chain CDRs (VH CDR1-3). The antibody or the antigen-binding fragment described herein is bispecific antibody comprising an antibody fragment specifically binding to antigen a and antigen b. In some embodiments, a first polypeptide chain comprises the structure VHa-CHa, a second polypeptide chain comprises the structure VLa-CLa, a third polypeptide chain comprises the structure VHb-CHb, and a fourth polypeptide chain comprises the structure VLb-CLb. In some embodiments, the second polypeptide chain is identical to the fourth polypeptide chain in amino acid sequence.

The term "antibody fragment" or "antigen-binding fragment" refers to a part of an antibody, and the composition of the antibody fragment of the present invention may be similar to F(ab')₂, F(ab)₂, Fab', Fab, Fv, scFv and the like in monospecific antibody fragments. Regardless of its structure, the antibody fragment binds to the same antigen recognized by an intact antibody. The term "antibody fragment" includes aptamers, mirror image isomers and bivalent antibodies. The term "antigen-binding fragment" also includes any synthetic or genetically engineered protein that functions as an antibody by binding to a specific antigen to form a complex.

"Single chain variable fragment" or "scFv" refers to a fusion protein of a heavy chain variable region (VH) and a light chain variable region (VL) of an immunoglobulin. In some aspects, these regions are linked to a short linker peptide having 10 to about 25 amino acids. The linker may be enriched with glycine to improve flexibility, and enriched with serine or threonine to improve solubility, and may link the N terminus of VH and the C terminus of VL, or vice versa. Although the protein has the constant region removed and the linker introduced, it retains the specificity of the original immunoglobulin. ScFv molecules are generally known in the art and are described, for example, in U.S. patent No. 5,892,019.

The term "antibody" includes a wide variety of polypeptides that can be biochemically distinguished. Those skilled in the art will appreciate that the classes of heavy chains include gamma, mu, alpha, delta, or epsilon (γ, µ, α, δ, or ε) and some subclasses (e.g., γ1-γ4). The nature of this chain determines the "type" of the antibody as IgG, IgM, IgA, IgG or IgE. Immunoglobulin subclasses (isotypes), such as IgG1, IgG2, IgG3, IgG4, etc., have been well characterized and the functional specificity imparted is also known. All types of immunoglobulins are within the scope of the present invention. In some embodiments, the immunoglobulin molecule is an IgG species. These four chains are connected in a "Y" configuration through disulfide bonds, wherein the light chain starts at the opening of "Y" configuration and extends through the variable region to surround the heavy chain.

The antibodies, antigen-binding fragments or derivatives disclosed herein include, but are not limited to, polyclonal antibodies, monoclonal antibodies, multispecific antibodies, fully human antibodies, humanized antibodies, primatized antibodies, chimeric antibodies, single-chain antibodies, epitope-binding fragments (e.g., Fab, Fab' and F(ab')₂), and single chain Fv (scFv).

Light chains can be classified into kappa (κ) or lambda (λ). Each heavy chain may bind to a x or λ light chain. In general, when an immunoglobulin is produced by a hybridoma, a B cell or a genetically engineered host cell, the light and heavy chains are bound by covalent bonds, and the "tail" portions of the two heavy chains are bound by covalent disulfide bonds or non-covalent bonds. In the heavy chains, the amino acid sequence extends from the N terminus at the forked end of the Y configuration to the C terminus at the bottom of each chain. The immunoglobulin x light chain variable region is Vκ; and the immunoglobulin λ light chain variable region is V_{λ}.

Both the light and heavy chains are divided into structurally and functionally homologous regions. The terms "constant" and "variable" are used in accordance with function. The light chain variable region (VL) and heavy chain variable region (VH) determine the antigen recognition and specificity. The light chain constant region and the heavy chain constant region impart important biological properties such as secretion, transplacental movement, Fc receptor binding, and complement fixation. By convention, the numbering of amino acids in the constant regions increases as they become further away from the antigen-binding site of the antibody or amino terminus. The N-terminal portion is the variable region, and the C-terminal portion is the constant region; the CH3 and CL domains comprise the carboxyl termini of the heavy chain and light chain, respectively.

In naturally occurring antibodies, the six "complementarity determining regions" or "CDRs" present in each antigen-binding domain are short, non-contiguous, antigen-specific binding amino acid sequences that form the antigen-binding domain, assuming that the antibody is present in its three-dimensional configuration in an aqueous environment. The remaining amino acids in the antigen-binding domain, referred to as the "framework" region, exhibit little intermolecular variability. Most of the framework regions adopt a β-sheet conformation, with the CDRs forming a loop structure connected to, or in some cases forming part of, the β-sheet structure. Thus, the framework regions position the CDRs in a correct orientation by interchain non-covalent interactions through forming a scaffold. The antigen-binding domain with the specifically positioned CDRs forms a surface complementary to an epitope on the antigen that facilitates non-covalent binding of the antibody to its antigenic epitope. For a given heavy or light chain variable region, amino acids comprising the CDRs and the framework regions may be identified by one of ordinary skills in the art according to known methods (see, Kabat, E., et al., U.S. Department of Health and Human Services, Sequences of Proteins of Immunological Interest, (1983) and Chothia and Lesk, J. Mol. Biol., 196:901-917 (1987)).

Based on different assignment systems, the boundaries of the CDRs of the variable regions of the same antibody may differ. Thus, when it comes to defining an antibody with a particular CDR sequence defined in the present invention, the scope of the antibody also encompasses antibodies whose variable region sequences comprise the CDR sequence of the present invention but whose claimed CDR boundaries differ from the particular CDR boundaries defined in the present invention due to the application of different schemes. CDRs defined according to Kabat and Chothia schemes include overlaps or subsets of amino acid residues when compared with each other. Nevertheless, it is within the scope of the present invention to apply any definition to refer to the CDRs of an antibody or a variant thereof.

Kabat et al. also define a numbering scheme applicable to the variable region sequence of any antibody. One of ordinary skills in the art can apply the "Kabat numbering" scheme to any variable region sequence without depending on other experimental data beyond the sequence itself. "Kabat numbering" refers to the numbering scheme proposed by Kabat et al., U.S. Dept. of Health and Human Services in Sequence of Proteins of Immunological Interest (1983). EU or Chothia numbering scheme can also be applicable to the antibody.

The antibodies disclosed herein may be derived from any animal, including birds and mammals. Preferably, the antibody is derived from a human, a mouse, a donkey, a rabbit, a goat, a camel, a llama, a horse, or a chicken source. In another embodiment, the variable region may be derived from a condricthoid source (e.g., from a shark).

The heavy chain constant region comprises at least one of a CH1 domain, a hinge (e.g., upper, middle and/or lower hinge region) domain, a CH2 domain, a CH3 domain, or a variant or a fragment. The heavy chain constant region of the antibody may be derived from different immunoglobulin molecules. For example, the heavy chain constant region of the polypeptide may comprise a CH1 domain derived from an IgG1 molecule and a hinge region derived from an IgG3 molecule. In another embodiment, the heavy chain constant region may comprise a hinge region derived partially from an IgG1 molecule and partially from an IgG3 molecule. In another embodiment, a portion of the heavy chain may comprise a chimeric hinge region derived partially from an IgG1 molecule and partially from an IgG4 molecule.

"Light chain constant region" includes a part of amino acid sequence from the light chain of an antibody. Preferably, the light chain constant region comprises at least one of a constant x domain or a constant λ domain. "Light chain-heavy chain pair" refers to a collection of light and heavy chains that can form dimers through disulfide bonds between the CL domain of the light chain and the CH1 domain of the heavy chain.

"VH domain" includes the variable domain at the amino terminus of an immunoglobulin heavy chain, and "CH1 domain" includes the first constant region of an immunoglobulin heavy chain. The CH2 domain is not closely paired with other domains, but rather two N-linked branched carbohydrate chains are inserted between the two CH2 domains of an intact native IgG molecule. The CH3 domain extends from the CH2 domain to the C terminus of the IgG molecule and comprises about 108 residues. "Hinge region" includes a portion of the heavy chain region connecting the CH1 domain and CH2 domain. The hinge region comprises about 25 residues and is flexible, thereby enabling independent movement of the two N-terminal antigen-binding regions. The hinge region can be subdivided into three distinct domains: upper, middle and lower hinge domains (Roux et al., J. Immunol., 161:4083 (1998)).

"Disulfide bond" refers to a covalent bond formed between two sulfur atoms. The thiol group of cysteine can form a disulfide bond or a bridge with a second thiol group. In most naturally occurring IgG molecules, the CH1 and CL regions are linked by a disulfide bond.

"Chimeric antibody" refers to any antibody in which the variable region of the antibody is obtained or derived from a first species, and the constant region thereof (which may be intact, partial or modified) is derived from a second species. In certain embodiments, the variable region is derived from a non-human source (e.g., mouse or primate) and the constant region is derived from a human source.

"Specifically bind to" generally means that an antibody or an antigen-binding fragment forms a relatively stable complex with a specific antigen, through complementary binding of its antigen-binding domain and epitope. "Specificity" can be expressed by relative affinity of an antibody or an antigen-binding fragment for binding to a specific antigen or epitope. For example, an antibody "A" can be considered to have a higher specificity for an antigen than an antibody "B" if the antibody "A" has a greater relative affinity for the antigen than the antibody "B". Specific binding can be described by the equilibrium dissociation constant (KD). A smaller KD means a tighter binding. Methods for determining whether two molecules specifically bind to each other are well known in the art, and include, for example, equilibrium dialysis, surface plasmon resonance, biofilm layer interferometry, and the like. Antibodies that "specifically bind" to antigen a include antibodies that have an equilibrium dissociation constant KD of less than or equal to about 100 nM, less than or equal to about 10 nM, less than or equal to about 5 nM, less than or equal to about 1 nM or less than or equal to about 0.5 nM with the antigen a.

"EC₅₀", i.e., concentration for 50% of maximal effect, refers to the concentration that can cause 50% of maximal effect.

"Bispecific" antibody refers to an antibody having two antigen-binding sites, which may be different epitopes of the same antigen or different epitopes of different antigens.

The term "common light chain" refers to a light chain that is capable of being assembled simultaneously with different heavy chains into a complete antibody with corresponding function; the light chain can be used in expressing bispecific antibodies, or in expressing a mixture of two antibodies.

The term "effector function" refers to biological activities attributed to an immunoglobulin Fc region. Examples of immunoglobulin effector functions include: C1q binding and complement-dependent cytotoxicity (CDC), antibody-dependent cell-mediated cytotoxicity (ADCC), antibody-dependent cellular phagocytosis (ADCP), cytokine secretion, Fc receptor binding, immune complex-mediated antigen uptake by antigen-presenting cells, down regulation of cell surface receptors (such as B-cell receptors), and B-cell activation.

"Treatment" refers to both therapeutic treatment and prophylactic or preventative measures, the purpose of which is to prevent, slow down, ameliorate, or halt undesirable physiological changes or disorders, such as the progression of a disease, including, but not limited to, alleviation of symptoms, diminishment of extent of disease, stabilized (i.e., not worsening) state of disease, delay or slowing of disease progression, amelioration, palliation, alleviation, or elimination of the disease state (whether partial or total) of state of disease, prolongation of life expectancy as compared with that in the absence of treatment, and the like, as either detectable or undetectable. Patients in need of treatment include patients already with a condition or disorder, patients susceptible to a condition or disorder, or patients in need of prevention of the condition or disorder, or patients who may or are expected to benefit from administration of the antibody or the pharmaceutical composition disclosed herein for detection, diagnostic procedures, and/or treatment.

"Patient" refers to any mammal in need of diagnosis, prevention, prognosis or treatment, including humans, dogs, cats, rabbits, mice, horses, cattle and the like.

### Bispecific antibody

Compared with monoclonal antibodies, bispecific antibodies have such remarkable advantages as stronger specificity, tumor cell-targeting property and reduction in off-target toxicity. Bispecific antibodies have one more specific antigen-binding site than monoclonal antibodies and show the following therapeutic advantages: (1) the two antigen-binding sites can bind to tumor cells and immune cells, respectively, so that T immune cells are gathered around the tumor cells, and the killing of tumors is enhanced; (2) the bispecific antibodies can block two different medium pathways simultaneously to play a unique or overlapped function and mediate various immune signaling pathways, thus enhancing the cell killing toxicity; (3) after binding to two different cell surface antigens, the binding specificity can be potentially increased relatively, and the side effects such as off target can be reduced. Therefore, the bispecific antibody exhibits wide application prospects in tumor immunotherapy and inflammation therapy.

In tumor immunity, the treatment schemes that target multiple targets can cooperate with each other, which is beneficial to prevent tumor immune escape. Therefore, at present, co-administration of antibodies targeting different targets in tumor immunity has also entered clinical trials. However, co-administration requires an injection of two separate antibody products or a single injection of a combined formulation of two different antibodies. Although two injections allow flexibility in dosage and timing, it causes inconvenience and pain for patients. In addition, although the combined formulation may provide some flexibility in dosage, it is often difficult to find preparation conditions that allow the chemical and physical stability of two antibodies in solution because of the different molecular characteristics of the two antibodies. Moreover, therapies of co-administration and combined formulation of two different antibodies may increase the extra cost of the patient and/or payer; therefore, there is a need for alternative immunotherapy for the treatment of tumors, and preferably such alternative immunotherapy involves bispecific antibodies.

### 1) Portion of a bispecific antibody that binds to PD-L1

In some embodiments, the bispecific antibody or the antigen-binding fragment of the present invention can specifically bind to PD-L1. In some embodiments, the bispecific antibody or the antigen-binding fragment of the present invention can specifically bind to mammalian PD-L1. In some embodiments, the PD-L1 is human PD-L1. In some embodiments, the antibody molecule binds to one or more extracellular domains of PD-L1.

In some embodiments, the antibody binding affinity is determined using surface plasmon resonance (e.g., Biacore affinity measurement).

In some embodiments, the bispecific antibody or the antigen-binding fragment of the present invention has one or more of the following properties:
(a) The bispecific antibody or the antigen-binding fragment of the present invention binds to PD-L1 (e.g., human PD-L1) with high affinity, e.g., binds to PD-L1 with an equilibrium dissociation constant (KD) of less than about 12 nM, such as less than or equal to about 10 nM, less than or equal to about 5 nM, or less than or equal to about 4 nM, 3 nM, 2 nM or 1 nM. In some embodiments, the KD is less than or equal to about 0.9 nM or 0.5 nM.
(b) The bispecific antibody or the antigen-binding fragment of the present invention binds to cells expressing human PD-L1, and in some embodiments, e.g., the EC₅₀ is less than or equal to about 2 nM, 1 nM, 0.9 nM, 0.8 nM, 0.7 nM, 0.6 nM or 0.5 nM. In some embodiments, the binding is determined by flow cytometry (e.g., FACS). In some embodiments, the cells expressing human PD-L1 are CHO cells expressing human PD-L1.
(c) In some embodiments, the bispecific antibody or the antigen-binding fragment of the present invention blocks a relevant activity of PD-L1/PD-1; for example, the EC₅₀ is less than or equal to about 6 µg/mL, 5 µg/mL, 4 µg/mL, 3 µg/mL, 2 µg/mL or 1 µg/mL. In some embodiments, the relevant activity of PD-L1 is the binding of PD-L1 to PD-1. In some embodiments, the antibody or the fragment thereof of the present invention inhibits binding of PD-L1 to PD-1 in an MOA assay with an EC₅₀ of less than or equal to about 6 µg/mL, 5 µg/mL, 4 µg/mL, 3 µg/mL, 2 µg/mL or 1 µg/mL, or with an EC₅₀ of about 0.1-3 µg/mL, 0.1-0.4 µg/mL or 0.1-0.3 µg/mL. In some embodiments, the cells are CHO cells overexpressing human PD-L1.
(d) The bispecific antibody or the antigen-binding fragment of the present invention can induce antibody-dependent cell-mediated cytotoxicity (ADCC).
(e) The bispecific antibody or the antigen-binding fragment of the present invention inhibits one or more activities of PD-L 1, for example, causing one or more of the following: increase in tumor-infiltrating lymphocytes, increase in T cell receptor-mediated proliferation, or decrease in immune evasion of cancer cells. In some embodiments, the bispecific antibody or the antigen-binding fragment can inhibit the proliferation of a tumor, and the tumor is tumor immune escape. In some embodiments, the tumor is a gastrointestinal tumor (e.g., cancer), such as colon cancer.

In some embodiments, the bispecific antibody or the antigen-binding fragment of the present invention comprises a heavy chain variable region a (VHa) and a light chain variable region a (VLa) that specifically bind to PD-L1, wherein the VHa comprises 3 CDRs (VHa CDR1, VHa CDR2 and VHa CDR3), and the VLa comprises 3 CDRs (VLa CDR1, VLa CDR2 and VLa CDR3). In some embodiments, the CDRs of the VHa are selected from heavy chain CDRs in atezolizumab, avelumab, durvalumab, envafolimab or cosibelimab. In some embodiments, the VHa is selected from heavy chain variable regions in atezolizumab, avelumab, durvalumab, envafolimab or cosibelimab. In some embodiments, the CDRs of the VLa are selected from light chain CDRs in atezolizumab, avelumab, durvalumab, envafolimab or cosibelimab. In some embodiments, the VLa is selected from light chain variable regions in atezolizumab, avelumab, durvalumab, envafolimab or cosibelimab.

In some embodiments, the VHa CDR1 comprises an amino acid sequence set forth in any one of SEQ ID NOs: 4-8; the VHa CDR2 comprises an amino acid sequence set forth in any one of SEQ ID NOs: 9-18; and the VHa CDR3 comprises an amino acid sequence set forth in SEQ ID NO: 19 or 20. In some embodiments, the VHa CDR1 comprises an amino acid sequence set forth in SEQ ID NO: 4; the VHa CDR2 comprises an amino acid sequence set forth in any one of SEQ ID NOs: 9-14; and the VHa CDR3 comprises an amino acid sequence set forth in SEQ ID NO: 19. In some embodiments, VHa CDR1 comprises an amino acid sequence set forth in SEQ ID NO: 4; the VHa CDR2 comprises an amino acid sequence set forth in SEQ ID NO: 14; and the VHa CDR3 comprises an amino acid sequence set forth in SEQ ID NO: 19.

In some embodiments, the VLa CDR1 comprises an amino acid sequence set forth in any one of SEQ ID NOs: 36-40; the VLa CDR2 comprises an amino acid sequence set forth in any one of SEQ ID NOs: 41-44; and the VLa CDR3 comprises an amino acid sequence set forth in any one of SEQ ID NOs: 45-48. In some embodiments, the VLa CDR1 comprises an amino acid sequence set forth in SEQ ID NO: 36; the VLa CDR2 comprises an amino acid sequence set forth in SEQ ID NO: 41; and the VLa CDR3 comprises an amino acid sequence set forth in SEQ ID NO: 45.

In some embodiments, the VHa CDR1 comprises an amino acid sequence set forth in SEQ ID NO: 4; the VHa CDR2 comprises an amino acid sequence set forth in SEQ ID NO: 14; the VHa CDR3 comprises an amino acid sequence set forth in SEQ ID NO: 19; the VLa CDR1 comprises an amino acid sequence set forth in SEQ ID NO: 36; the VLa CDR2 comprises an amino acid sequence set forth in SEQ ID NO: 41; and the VLa CDR3 comprises an amino acid sequence set forth in SEQ ID NO: 45.

In some embodiments, the VHa comprises an amino acid sequence set forth in any one of SEQ ID NOs: 49-63, and in some embodiments, the VLa comprises an amino acid sequence set forth in any one of SEQ ID NOs: 75-80.

In some embodiments, the bispecific antibody or the antigen-binding fragment of the present invention comprises a variable region a consisting of a VHa having an amino acid sequence set forth in SEQ ID NO: 50 and a VLa having an amino acid sequence set forth in SEQ ID NO: 75. In some embodiments, the bispecific antibody or the antigen-binding fragment of the present invention comprises a variable region a consisting of a VHa having an amino acid sequence set forth in SEQ ID NO: 51 and a VLa having an amino acid sequence set forth in SEQ ID NO: 75. In some embodiments, the bispecific antibody or the antigen-binding fragment of the present invention comprises a variable region a consisting of a VHa having an amino acid sequence set forth in SEQ ID NO: 52 and a VLa having an amino acid sequence set forth in SEQ ID NO: 75. In some embodiments, the bispecific antibody or the antigen-binding fragment of the present invention comprises a variable region a consisting of a VHa having an amino acid sequence set forth in SEQ ID NO: 54 and a VLa having an amino acid sequence set forth in SEQ ID NO: 75.

### 2) Portion of a bispecific antibody that binds to CD47

In some embodiments, the bispecific antibody or the antigen-binding fragment of the present invention can specifically bind to CD47. In some embodiments, the bispecific antibody or the antigen-binding fragment of the present invention can specifically bind to mammalian CD47. In some embodiments, the CD47 is human CD47. In some embodiments, the antibody molecule binds to one or more extracellular domains of CD47.

In some embodiments, the bispecific antibody or the antigen-binding fragment of the present invention has one or more of the following properties:
(a) The bispecific antibody or the antigen-binding fragment of the present invention binds to CD47 (e.g., human CD47) with high affinity, e.g., binds to CD47 with an equilibrium dissociation constant (KD) of less than about 700 nM, such as less than or equal to about 30 nM, less than or equal to about 10 nM, or less than or equal to about 5 nM.
(b) The bispecific antibody or the antigen-binding fragment of the present invention does not cause significant cell agglutination. For example, the bispecific antibody or the antigen-binding fragment of the present invention does not cause significant hemagglutination of red blood cells. In some embodiments, if the level of agglutination in the presence of the bispecific antibody or the antigen-binding fragment of the present invention decreases by at least 5%, at least 10%, at least 20%, at least 30%, at least 40%, at least 50%, at least 60%, at least 70%, at least 80%, at least 90% or at least 99% compared with the level of agglutination in the presence of the anti-CD47 positive control antibody Hu5F9-G4, it indicates that the bispecific antibody or the antigen-binding fragment of the present invention does not cause significant agglutination. The bispecific antibody or the antigen-binding fragment of the present invention does not cause significant cell agglutination when at an antibody concentration between 400 pM and 800 nM.
(c) The bispecific antibody or the antigen-binding fragment of the present invention does not bind to human red blood cells. In one embodiment, if the level of binding to human red blood cells in the presence of the bispecific antibody or the antigen-binding fragment of the present invention decreases by at least 5%, at least 10%, at least 20%, at least 30%, at least 40%, at least 50%, at least 60%, at least 70%, at least 80%, at least 90% or at least 99% compared with the level of binding to human red blood cells in the presence of the anti-CD47 positive control antibody Hu5F9-G4, it indicates that the bispecific antibody or the antigen-binding fragment of the present invention does not bind to human red blood cells significantly. Preferably, the bispecific antibody or the antigen-binding fragment of the present invention does not bind to human red blood cells significantly when at an antibody concentration between 200 pM and 100 nM.
(d) In some embodiments, the bispecific antibody or the antigen-binding fragment of the present invention is a blocking antibody that blocks the binding of CD47 to SIRPα. In some embodiments, the ability of macrophages to phagocytose tumor cells is increased by at least 5%, at least 10%, at least 20%, at least 30%, at least 40%, at least 50%, at least 60%, at least 70%, at least 80%, at least 90% or at least 99% in the presence of the CD47 antibody of the present invention.
(e) The bispecific antibody or the antigen-binding fragment of the present invention can induce antibody-dependent cell-mediated cytotoxicity (ADCC).
(f) The bispecific antibody or the antigen-binding fragment of the present invention inhibits one or more activities of CD47, for example, causing the increase in the phagocytic capacity of tumor-infiltrating macrophages and/or the decrease in immune evasion of cancer cells. In some embodiments, the bispecific antibody or the antigen-binding fragment can inhibit the proliferation of a tumor, and the tumor is tumor immune escape. In some embodiments, the tumor is a gastrointestinal tumor (e.g., cancer), such as colon cancer.

In some embodiments, the bispecific antibody or the antigen-binding fragment of the present invention comprises a heavy chain variable region b (VHb) and a light chain variable region b (VLb) that specifically bind to CD47, wherein the VHb comprises 3 CDRs (VHb CDR1, VHb CDR2 and VHb CDR3), and the VLb comprises 3 CDRs (VLb CDR1, VLb CDR2 and VLb CDR3). In some embodiments, the CDRs of the VHb are selected from heavy chain CDRs in magrolimab, AO-176 (Arch Oncology), TJC4 (I-Mab biopharma), AK117 (Akesobio), IBI188 (Innovent Biologics), and the like. In some embodiments, the VHb is selected from heavy chain variable regions in magrolimab, AO-176 (Arch Oncology), TJC4 (I-Mab biopharma), AK117 (Akesobio), IBI188 (Innovent Biologics), and the like. In some embodiments, the CDRs of the VLb are selected from light chain CDRs in magrolimab, AO-176 (Arch Oncology), TJC4 (I-Mab biopharma), AK117 (Akesobio), IBI188 (Innovent Biologics), and the like. In some embodiments, the VLb is selected from light chain variable regions in magrolimab, AO-176 (Arch Oncology), TJC4 (I-Mab biopharma), AK117 (Akesobio), IBI188 (Innovent Biologics), and the like.

In some embodiments, the VHb CDR1 comprises an amino acid sequence set forth in any one of SEQ ID NOs: 21-23; the VHb CDR2 comprises an amino acid sequence set forth in any one of SEQ ID NOs: 24-28; and the VHb CDR3 comprises an amino acid sequence set forth in any one of SEQ ID NOs: 29-35. In some embodiments, VHb CDR1 comprises an amino acid sequence set forth in SEQ ID NO: 21; the VHb CDR2 comprises an amino acid sequence set forth in SEQ ID NO: 24; and the VHb CDR3 comprises an amino acid sequence set forth in SEQ ID NO: 29.

In some embodiments, the VLb CDR1 comprises an amino acid sequence set forth in any one of SEQ ID NOs: 36-40; the VLb CDR2 comprises an amino acid sequence set forth in any one of SEQ ID NOs: 41-44; and the VLb CDR3 comprises an amino acid sequence set forth in any one of SEQ ID NOs: 45-48. In some embodiments, the VLb CDR1 comprises an amino acid sequence set forth in SEQ ID NO: 36; the VLb CDR2 comprises an amino acid sequence set forth in SEQ ID NO: 41; and the VLb CDR3 comprises an amino acid sequence set forth in SEQ ID NO: 45.

In some embodiments, the VHb CDR1 comprises an amino acid sequence set forth in SEQ ID NO: 21; the VHb CDR2 comprises an amino acid sequence set forth in SEQ ID NO: 24; the VHb CDR3 comprises an amino acid sequence set forth in SEQ ID NO: 29; the VLb CDR1 comprises an amino acid sequence set forth in SEQ ID NO: 36; the VLb CDR2 comprises an amino acid sequence set forth in SEQ ID NO: 41; and the VLb CDR3 comprises an amino acid sequence set forth in SEQ ID NO: 45.

In some embodiments, the VHb comprises an amino acid sequence set forth in any one of SEQ ID NOs: 64-74, and in some embodiments, the VLb comprises an amino acid sequence set forth in any one of SEQ ID NOs: 75-80.

In some embodiments, the bispecific antibody or the antigen-binding fragment of the present invention comprises a variable region b consisting of a VHb having an amino acid sequence set forth in SEQ ID NO: 64 and a VLb having an amino acid sequence set forth in SEQ ID NO: 75.

### 3) Anti-PD-L1/CD47 bispecific antibody

The innate immune system is the first line of nonspecific defense against infection and malignant cell transformation. In the innate immune system, monocytes, macrophages and dendritic cells act as antigen presenting cells (APCs) by phagocytosis. The ability of APCs to phagocytose tumor cells by phagocytosis is an indispensable bridge linking the innate immunity and the adaptive immunity. Decreased T cell activation is indirectly caused by decreased uptake of tumor cells by APCs, see, e.g., Avice MN et al., Role of CD47 in the induction of human naive T cell anergy, Journal of Immunology, 167(5): 2459-2468 (2001). Studies have confirmed that expression of both PD-L1 and CD47 proteins is regulated by the transcription factor MYC and they are overexpressed on tumor cells, see, e.g., Stephanie C et al., MYC regulates the antitumor immune response through CD47 and PD-L1, Science, 352(6282), 227-31 (2016). The tumor cells escape from the monitoring of the innate immune system through PD-L1/PD1 and CD47/SIRPα pathways and obtain adaptive immune tolerance.

In some embodiments, the present invention provides an anti-PD-L1/CD47 bispecific antibody or an antigen-binding fragment that can specifically bind to PD-L1 and CD47. In some embodiments, the antibody or the fragment thereof of the present invention binds to mammalian PD-L1 and CD47, such as human PD-L1 and CD47. For example, the antibody molecule specifically binds to an epitope (e.g., a linear or conformational epitope) on PD-L1 and CD47. In some embodiments, the antibody molecule binds to one or more extracellular domains of PD-L1 and CD47.

In some embodiments, the anti-PD-L1/CD47 bispecific antibody or the antigen-binding fragment of the present invention has one or more of the following properties:
(a) In some embodiments, the anti-PD-L1/CD47 bispecific antibody of the present invention can bind to PD-L1 and CD47 simultaneously, and maintains the affinity constant of the parent antibody, thereby being capable of blocking the PD1/PD-L1 signaling pathway and blocking the SIRPα/CD47 signaling pathway.
(b) In some embodiments, the anti-PD-L1/CD47 bispecific antibody of the present invention can bind to PD-L1 with ultra-high affinity (e.g., KD = 0.174 nM), and bind to CD47 with high affinity (e.g., KD = 3.78 nM) or moderate affinity (e.g., KD = 27.8 nM); the selective binding of the anti-PD-L1/CD47 bispecific antibody of the present invention to the tumor cells is promoted by the specific binding to PD-L1 on the tumor cells, the binding to CD47 expressed in many normal tissues is avoided, and the side effects are reduced; the effective dose range of the anti-PD-L1/CD47 bispecific antibody of the present invention can be remarkably expanded based on the fact that the affinity for CD47 is far lower than that for PD-L1.
(c) In some embodiments, in the anti-PD-L1/CD47 bispecific antibody of the present invention, a common light chain capable of avoiding pairing of unrelated heavy and light chains is designed.
(d) In some embodiments, in the anti-PD-L1/CD47 bispecific antibody of the present invention, amino acid residues capable of stabilizing the structure of the tetra-chain antibody and facilitating correct coupling or pairing between the respective chains are designed. In some embodiments, the bispecific antibody of the present invention comprises Y349C and S354C, or S354C and Y349C, respectively, in the respective Fc domains; the bispecific antibody of the present invention comprises a protuberance ("knob") or a cavity ("hole") in the respective Fc domains, and the protuberance or cavity in the Fc domain of the first polypeptide chain can be placed in the cavity or protuberance, respectively, in the Fc domain of the third polypeptide chain, and thus the first polypeptide chain and the third polypeptide chain form a stable association of "knob-into-hole" with each other, wherein the first polypeptide chain comprises the structure VHa-CHa and can specifically recognize and bind to PD-L1, and the third polypeptide chain comprises the structure VHb-CHb and can specifically recognize and bind to CD47.
(e) In some embodiments, the anti-PD-L1/CD47 bispecific antibody of the present invention has such advantages as easy stable expression in culture cells *in vitro,* good thermal stability, high antibody yield and purity, and no need of complex production process.
(f) The anti-PD-L1/CD47 bispecific antibody or the fragment thereof of the present invention binds to cells expressing human PD-L1, e.g., in some embodiments, binds to cells expressing human PD-L1 with an EC₅₀ of less than or equal to about 2 nM, 1 nM, 0.9 nM, 0.8 nM, 0.7 nM, 0.6 nM or 0.5 nM. In some embodiments, the binding is determined by flow cytometry (e.g., FACS). In some embodiments, the cells expressing human PD-L1 are CHO-S cells expressing human PD-L1.
(g) In some embodiments, the anti-PD-L1/CD47 bispecific antibody or the fragment thereof of the present invention blocks relevant activity of PD-L1/PD-1, e.g., blocks relevant activity of PD-L1/PD-1 with an EC₅₀ of less than or equal to about 0.3 µg/mL, or with an EC₅₀ of about 0.1-3 µg/mL. In some embodiments, the relevant activity of PD-L1/PD-1 refers to the activation of the inhibitory signals in the intracellular domain of PD1 after PD-L1 binds to PD-1. In some embodiments, the anti-PD-L1/CD47 bispecific antibody or the fragment thereof of the present invention inhibits binding of PD-L1 to PD-1 in an MOA assay with an EC₅₀ of less than or equal to about 0.3 µg/mL or about 0.1-3 µg/mL. In some embodiments, the cells are CHO cells overexpressing human PD-L1.
(h) The anti-PD-L1/CD47 bispecific antibody of the present invention does not cause significant cell agglutination. For example, the anti-PD-L1/CD47 bispecific antibody of the present invention does not cause significant hemagglutination of red blood cells. In some embodiments, if the level of agglutination in the presence of the anti-PD-L1/CD47 bispecific antibody of the present invention decreases by at least 5%, at least 10%, at least 20%, at least 30%, at least 40%, at least 50%, at least 60%, at least 70%, at least 80%, at least 90% or at least 99% compared with the level of agglutination in the presence of the anti-CD47 positive control antibody Hu5F9-G4, it indicates that the anti-PD-L1/CD47 bispecific antibody of the present invention does not cause significant agglutination. Preferably, the anti-PD-L1/CD47 bispecific antibody of the present invention does not cause significant cell agglutination when at an antibody concentration between 400 pM and 800 nM.
(i) The anti-PD-L1/CD47 bispecific antibody of the present invention does not bind to human red blood cells. In one embodiment, if the level of binding to human red blood cells in the presence of the anti-PD-L1/CD47 bispecific antibody of the present invention decreases by at least 5%, at least 10%, at least 20%, at least 30%, at least 40%, at least 50%, at least 60%, at least 70%, at least 80%, at least 90% or at least 99% compared with the level of binding to human red blood cells in the presence of the anti-CD47 positive control antibody Hu5F9-G4, it indicates that the anti-PD-L1/CD47 bispecific antibody of the present invention does not bind to human red blood cells significantly. Preferably, the anti-PD-L1/CD47 bispecific antibody of the present invention does not bind to human red blood cells significantly when at an antibody concentration between 200 pM and 100 nM.
(j) In some embodiments, the anti-PD-L1/CD47 bispecific antibody of the present invention is a blocking antibody that blocks the binding of CD47 to SIRPα. In some embodiments, the ability of macrophages to phagocytose tumor cells is increased by at least 5%, at least 10%, at least 20%, at least 30%, at least 40%, at least 50%, at least 60%, at least 70%, at least 80%, at least 90% or at least 99% in the presence of the anti-PD-L1/CD47 bispecific antibody of the present invention.
(k) In some embodiments, the anti-PD-L1/CD47 bispecific antibody or the fragment thereof of the present invention can induce antibody-dependent cell-mediated cytotoxicity (ADCC). In one embodiment, the anti-PD-L1/CD47 bispecific antibody or the fragment thereof of the present invention has a reduced ability to induce antibody-dependent cell-mediated cytotoxicity (ADCC). In one embodiment, the anti-PD-L1/CD47 bispecific antibody or the fragment thereof of the present invention can not induce antibody-dependent cell-mediated cytotoxicity (ADCC).
(1) The anti-PD-L1/CD47 bispecific antibody or the fragment thereof of the present invention synergistically inhibit one or more activities of PD-L1 and CD47. In some embodiments, the anti-PD-L1/CD47 bispecific antibody can inhibit the proliferation of a tumor, and the tumor is tumor immune escape. In some embodiments, the tumor is a gastrointestinal tumor (e.g., cancer), such as colon cancer.

In some embodiments, the anti-PD-L1/CD47 bispecific antibody of the present invention has four peptide chains, namely two heavy chains and two light chains. One heavy chain comprises VHa, the other heavy chain comprises VHb, one light chain comprises VLa, and the other light chain comprises VLb, wherein the variable region a composed of VHa and VLa specifically binds to PD-L1, and the variable region b composed of VHb and VLb specifically binds to CD47. In some embodiments, the two light chains are identical light chains.

In some embodiments, the bispecific antibody of the present invention uses the "Knobs-into-Holes" technique (see, e.g., John B. B. Ridgway et al., 'Knobs-into-holes' engineering of antibody CH3 domains for heavy chain heterodimerization, Protein Engineering, 9(7): p.617-21 (1996); Shane Atwell et al., Stable heterodimers form remodeling the domain interface of a homodimer using a phage display library, J. mol. Biol., 270: p.26-35 (1997); Paul Carter, Bispecific human IgG by design, Journal of Immunological Methods, 248, 7-15 (2001); patent US8216805B2). This technique enables the modification of the interfaces between different chains of the bispecific antibody of the present invention, thus facilitating the correct association of the chains of the bispecific antibody of the present invention. Generally, this technique involves introducing a "protuberance" (knob) at the interface of one chain" and introducing a corresponding "cavity"("hole") at the interface of the other chain to be paired with, such that the protuberance can be placed at the cavity. The protuberance can be constructed by replacing amino acid side chains at an interface of the CH3 domain from the heavy chain constant domains of one chain with relatively large side chains (e.g., amino acid replacement T366W (EU numbered)). The compensating cavity of the same size as, or a similar size to, the protuberance is constructed at an interface of the CH3 domain from the heavy chain constant domains of the other chain to be paired with by replacing large amino acid side chains with relatively small side chains (e.g., amino acid replacements T366S, L368A and Y407V (Eu numbered)). In some embodiments, the Fc domain of one heavy chain comprises Y349C, T366S, L368A and Y407V, and the Fc domain of the other heavy chain comprises S354C and T366W, thus forming a stable association of "knob-into-hole".

In some embodiments, the Fc region of the bispecific antibody of the present invention comprises a modification of binding affinity for the Fc receptor. In some embodiments, the Fc receptor is a Fcγ receptor, particularly a human Fcγ receptor. In one embodiment, the Fc receptor is an activating Fc receptor. In some embodiments, the modification reduces the effector function of the bispecific antibody of the present invention. In some embodiments, the effector function is antibody-dependent cell-mediated cytotoxicity (ADCC). In some embodiments, the modification is in the Fc region of the immunoglobulin molecule, particularly in its CH2 region. In some embodiments, the immunoglobulin molecule comprises an amino acid replacement at position 297 (Eu numbered) of an immunoglobulin heavy chain. In a specific embodiment, the amino acid replacement is N297A (see, e.g., J. Lund et al., Oligosaccharide-protein interactions in IgG can modulate recognition by Fc gamma receptors, FASEB. J. 9, 115-119 (1995)).

In some embodiments, the heavy chain of the anti-PD-L1/CD47 bispecific antibody of the present invention further comprises a signal peptide sequence, e.g., MEFGLSWVFLVAILKGVQC (SEQ ID NO: 90). In some embodiments, the light chain of the anti-PD-L1/CD47 bispecific antibody of the present invention further comprises a signal peptide sequence, e.g., MDMRVLAQLLGLLLLCFPGARC (SEQ ID NO: 91).

In some embodiments, the anti-PD-L1/CD47 bispecific antibody comprises a VHa set forth in any one of SEQ ID NOs: 49-63, a VHb set forth in any one of SEQ ID NOs: 64-74, and a VLa and a VLb set forth in any one of SEQ ID NOs: 75-80.

In some embodiments, the anti-PD-L1/CD47 bispecific antibody comprises a VHa set forth in SEQ ID NO: 49, a VHb set forth in SEQ ID NO: 64, and a VLa and a VLb set forth in SEQ ID NO: 75.

In some embodiments, the anti-PD-L1/CD47 bispecific antibody comprises a VHa set forth in SEQ ID NO: 49, a VHb set forth in SEQ ID NO: 64, and a VLa and a VLb set forth in SEQ ID NO: 76.

In some embodiments, the anti-PD-L1/CD47 bispecific antibody comprises a VHa set forth in SEQ ID NO: 49, a VHb set forth in SEQ ID NO: 64, and a VLa and a VLb set forth in SEQ ID NO: 77.

In some embodiments, the anti-PD-L1/CD47 bispecific antibody comprises a VHa set forth in SEQ ID NO: 49, a VHb set forth in SEQ ID NO: 64, and a VLa and a VLb set forth in SEQ ID NO: 78.

In some embodiments, the anti-PD-L1/CD47 bispecific antibody comprises a VHa set forth in SEQ ID NO: 49, a VHb set forth in SEQ ID NO: 64, and a VLa and a VLb set forth in SEQ ID NO: 79.

In some embodiments, the anti-PD-L1/CD47 bispecific antibody comprises a VHa set forth in SEQ ID NO: 49, a VHb set forth in SEQ ID NO: 64, and a VLa and a VLb set forth in SEQ ID NO: 80.

In some embodiments, the anti-PD-L1/CD47 bispecific antibody comprises a VHa set forth in SEQ ID NO: 50, a VHb set forth in SEQ ID NO: 64, and a VLa and a VLb set forth in SEQ ID NO: 75.

In some embodiments, the anti-PD-L1/CD47 bispecific antibody comprises a VHa set forth in SEQ ID NO: 50, a VHb set forth in SEQ ID NO: 64, and a VLa and a VLb set forth in SEQ ID NO: 76.

In some embodiments, the anti-PD-L1/CD47 bispecific antibody comprises a VHa set forth in SEQ ID NO: 50, a VHb set forth in SEQ ID NO: 64, and a VLa and a VLb set forth in SEQ ID NO: 77.

In some embodiments, the anti-PD-L1/CD47 bispecific antibody comprises a VHa set forth in SEQ ID NO: 50, a VHb set forth in SEQ ID NO: 64, and a VLa and a VLb set forth in SEQ ID NO: 78.

In some embodiments, the anti-PD-L1/CD47 bispecific antibody comprises a VHa set forth in SEQ ID NO: 50, a VHb set forth in SEQ ID NO: 64, and a VLa and a VLb set forth in SEQ ID NO: 79.

In some embodiments, the anti-PD-L1/CD47 bispecific antibody comprises a VHa set forth in SEQ ID NO: 50, a VHb set forth in SEQ ID NO: 64, and a VLa and a VLb set forth in SEQ ID NO: 80.

In some embodiments, the anti-PD-L1/CD47 bispecific antibody comprises a VHa set forth in SEQ ID NO: 51, a VHb set forth in SEQ ID NO: 64, and a VLa and a VLb set forth in SEQ ID NO: 75.

In some embodiments, the anti-PD-L1/CD47 bispecific antibody comprises a VHa set forth in SEQ ID NO: 51, a VHb set forth in SEQ ID NO: 64, and a VLa and a VLb set forth in SEQ ID NO: 76.

In some embodiments, the anti-PD-L1/CD47 bispecific antibody comprises a VHa set forth in SEQ ID NO: 51, a VHb set forth in SEQ ID NO: 64, and a VLa and a VLb set forth in SEQ ID NO: 77.

In some embodiments, the anti-PD-L1/CD47 bispecific antibody comprises a VHa set forth in SEQ ID NO: 51, a VHb set forth in SEQ ID NO: 64, and a VLa and a VLb set forth in SEQ ID NO: 78.

In some embodiments, the anti-PD-L1/CD47 bispecific antibody comprises a VHa set forth in SEQ ID NO: 51, a VHb set forth in SEQ ID NO: 64, and a VLa and a VLb set forth in SEQ ID NO: 79.

In some embodiments, the anti-PD-L1/CD47 bispecific antibody comprises a VHa set forth in SEQ ID NO: 51, a VHb set forth in SEQ ID NO: 64, and a VLa and a VLb set forth in SEQ ID NO: 80.

In some embodiments, the anti-PD-L1/CD47 bispecific antibody comprises a VHa set forth in SEQ ID NO: 52, a VHb set forth in SEQ ID NO: 64, and a VLa and a VLb set forth in SEQ ID NO: 75.

In some embodiments, the anti-PD-L1/CD47 bispecific antibody comprises a VHa set forth in SEQ ID NO: 52, a VHb set forth in SEQ ID NO: 64, and a VLa and a VLb set forth in SEQ ID NO: 76.

In some embodiments, the anti-PD-L1/CD47 bispecific antibody comprises a VHa set forth in SEQ ID NO: 52, a VHb set forth in SEQ ID NO: 64, and a VLa and a VLb set forth in SEQ ID NO: 77.

In some embodiments, the anti-PD-L1/CD47 bispecific antibody comprises a VHa set forth in SEQ ID NO: 52, a VHb set forth in SEQ ID NO: 64, and a VLa and a VLb set forth in SEQ ID NO: 78.

In some embodiments, the anti-PD-L1/CD47 bispecific antibody comprises a VHa set forth in SEQ ID NO: 52, a VHb set forth in SEQ ID NO: 64, and a VLa and a VLb set forth in SEQ ID NO: 79.

In some embodiments, the anti-PD-L1/CD47 bispecific antibody comprises a VHa set forth in SEQ ID NO: 52, a VHb set forth in SEQ ID NO: 64, and a VLa and a VLb set forth in SEQ ID NO: 80.

In some embodiments, the anti-PD-L1/CD47 bispecific antibody comprises a VHa set forth in SEQ ID NO: 53, a VHb set forth in SEQ ID NO: 64, and a VLa and a VLb set forth in SEQ ID NO: 75.

In some embodiments, the anti-PD-L1/CD47 bispecific antibody comprises a VHa set forth in SEQ ID NO: 53, a VHb set forth in SEQ ID NO: 64, and a VLa and a VLb set forth in SEQ ID NO: 76.

In some embodiments, the anti-PD-L1/CD47 bispecific antibody comprises a VHa set forth in SEQ ID NO: 53, a VHb set forth in SEQ ID NO: 64, and a VLa and a VLb set forth in SEQ ID NO: 77.

In some embodiments, the anti-PD-L1/CD47 bispecific antibody comprises a VHa set forth in SEQ ID NO: 53, a VHb set forth in SEQ ID NO: 64, and a VLa and a VLb set forth in SEQ ID NO: 78.

In some embodiments, the anti-PD-L1/CD47 bispecific antibody comprises a VHa set forth in SEQ ID NO: 53, a VHb set forth in SEQ ID NO: 64, and a VLa and a VLb set forth in SEQ ID NO: 79.

In some embodiments, the anti-PD-L1/CD47 bispecific antibody comprises a VHa set forth in SEQ ID NO: 53, a VHb set forth in SEQ ID NO: 64, and a VLa and a VLb set forth in SEQ ID NO: 80.

In some embodiments, the anti-PD-L1/CD47 bispecific antibody comprises a VHa set forth in SEQ ID NO: 54, a VHb set forth in SEQ ID NO: 64, and a VLa and a VLb set forth in SEQ ID NO: 75.

In some embodiments, the anti-PD-L1/CD47 bispecific antibody comprises a VHa set forth in SEQ ID NO: 54, a VHb set forth in SEQ ID NO: 64, and a VLa and a VLb set forth in SEQ ID NO: 76.

In some embodiments, the anti-PD-L1/CD47 bispecific antibody comprises a VHa set forth in SEQ ID NO: 54, a VHb set forth in SEQ ID NO: 64, and a VLa and a VLb set forth in SEQ ID NO: 77.

In some embodiments, the anti-PD-L1/CD47 bispecific antibody comprises a VHa set forth in SEQ ID NO: 54, a VHb set forth in SEQ ID NO: 64, and a VLa and a VLb set forth in SEQ ID NO: 78.

In some embodiments, the anti-PD-L1/CD47 bispecific antibody comprises a VHa set forth in SEQ ID NO: 54, a VHb set forth in SEQ ID NO: 64, and a VLa and a VLb set forth in SEQ ID NO: 79.

In some embodiments, the anti-PD-L1/CD47 bispecific antibody comprises a VHa set forth in SEQ ID NO: 54, a VHb set forth in SEQ ID NO: 64, and a VLa and a VLb set forth in SEQ ID NO: 80.

In some embodiments, the heavy chain constant region is of IgG1 subtype (SEQ ID NO: 81). In some embodiments, the heavy chain constant region comprises one or more of the following amino acid mutations: N297A, Y349C, S354C, T366W, T366S, L368A and Y407V

In some embodiments, the anti-PD-L1/CD47 bispecific antibody comprises a CHa set forth in SEQ ID NO: 83, a CHb set forth in SEQ ID NO: 84, and a CLa and a CLb set forth in SEQ ID NO: 82.

In some embodiments, the anti-PD-L1/CD47 bispecific antibody comprises a CHa set forth in SEQ ID NO: 84, a CHb set forth in SEQ ID NO: 83, and a CLa and a CLb set forth in SEQ ID NO: 82.

In some embodiments, the anti-PD-L1/CD47 bispecific antibody comprises a CHa set forth in SEQ ID NO: 85, a CHb set forth in SEQ ID NO: 86, and a CLa and a CLb set forth in SEQ ID NO: 82.

In some embodiments, the anti-PD-L1/CD47 bispecific antibody comprises a CHa set forth in SEQ ID NO: 86, a CHb set forth in SEQ ID NO: 85, and a CLa and a CLb set forth in SEQ ID NO: 82.

In some embodiments, the anti-PD-L1/CD47 bispecific antibody comprises two different heavy chains and two identical light chains. The heavy chain a is set forth in SEQ ID NO: 92, the heavy chain b is set forth in SEQ ID NO: 93, and the light chains are set forth in SEQ ID NO: 96.

In some embodiments, the anti-PD-L1/CD47 bispecific antibody comprises two different heavy chains and two identical light chains. The heavy chain a is set forth in SEQ ID NO: 94, the heavy chain b is set forth in SEQ ID NO: 95, and the light chains are set forth in SEQ ID NO: 96.

The constant regions and the full-length sequences are as follows:

| SEQ ID NO: | Sequence |
|---|---|
| 81 | |
| 82 | |
| 83 | ASTKGPSVFPLAPSSKSTSGGTAALGCLVKDYFPEPVTVSWNSGALTSGVHTFPAVLQSSG LYSLSSVVTVPSSSLGTQTYICNVNHKPSNTKVDKKVEPKSCDKTHTCPPCPAPELLGGPS |
| | VFLFPPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVYTLPPCRDEL TKNQVSLWCLVKGFYPSDIAVEWESNGQPENNYKTTPPVLDSDGSFFLYSKLTVDKSRWQ QGNVFSCSVMHEALHNHYTQKSLSLSPGK |
| 84 | ASTKGPSVFPLAPSSKSTSGGTAALGCLVKDYFPEPVTVSWNSGALTSGVHTFPAVLQSSG LYSLSSVVTVPSSSLGTQTYICNVNHKPSNTKVDKKVEPKSCDKTHTCPPCPAPELLGGPS VFLFPPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVCTLPPSRDELT KNQVSLSCAVKGFYPSDIAVEWESNGQPENNYKTTPPVLDSDGSFFLVSKLTVDKSRWQQ GNVFSCSVMHEALHNHYTQKSLSLSPGK |
| 85 | ASTKGPSVFPLAPSSKSTSGGTAALGCLVKDYFPEPVTVSWNSGALTSGVHTFPAVLQSSG LYSLSSVVTVPSSSLGTQTYICNVNHKPSNTKVDKKVEPKSCDKTHTCPPCPAPELLGGPS VFLFPPKPKDTLMISRTPEVTCVVVDVSHEDPEVKFNWYVDGVEVHNAKTKPREEQYAST YRVVSVLTVLHQDWLNGKEYKCKVSNKALPAPIEKTISKAKGQPREPQVYTLPPCRDELT KNQVSLWCLVKGFYPSDIAVEWESNGQPENNYKTTPPVLDSDGSFFLYSKLTVDKSRWQQ GNVFSCSVMHEALHNHYTQKSLSLSPGK |
| 86 | ASTKGPSVFPLAPSSKSTSGGTAALGCLVKDYFPEPVTVSWNSGALTSGVHTFPAVLQSSG LYSLSSVVTVPSSSLGTQTYICNVNHKPSNTKVDKKVEPKSCDKTHTCPPCPAPELLGGPS VFLFPPKPKDTLMISRTPEVTCVVVDVSHEDPEVKFNWYVDGVEVHNAKTKPREEQYAST YRVVSVLTVLHQDWLNGKEYKCKVSNKALPAPIEKTISKAKGQPREPQVCTLPPSRDELT KNQVSLSCAVKGFYPSDIAVEWESNGQPENNYKTTPPVLDSDGSFFLVSKLTVDKSRWQQ GNVFSCSVMHEALHNHYTQKSLSLSPGK |
| 92 | EVQLVESGGGLVQPGGSLRLSCAASGFTFSDSWIHWVRQAPGKGLEWVGWISPYGGSTY YADSYRSRFTISADTSKNTAYLQMNSLRAEDTAVYYCARRHWPGGFDYWGQGTLVTVSS ASTKGPSVFPLAPSSKSTSGGTAALGCLVKDYFPEPVTVSWNSGALTSGVHTFPAVLQSSG LYSLSSVVTVPSSSLGTQTYICNVNHKPSNTKVDKKVEPKSCDKTHTCPPCPAPELLGGPS VFLFPPKPKDTLMISRTPEVTCVVVDVSHEDPEVKFNWYVDGVEVHNAKTKPREEQYAST YRVVSVLTVLHQDWLNGKEYKCKVSNKALPAPIEKTISKAKGQPREPQVYTLPPCRDELT KNQVSLWCLVKGFYPSDIAVEWESNGQPENNYKTTPPVLDSDGSFFLYSKLTVDKSRWQQ GNVFSCSVMHEALHNHYTQKSLSLSPGK |
| 93 | QVQLQESGPGLVKPSETLSLTCTVSGGSLDNYYWSWIRQPPGKGLEWIGYIYYSGNTNYN PSLKSRVTISVDTSKNQFSLKLSSVTAADTAVYYCARGGRFLERYWGQGTLVTVSSASTKG PSVFPLAPSSKSTSGGTAALGCLVKDYFPEPVTVSWNSGALTSGVHTFPAVLQSSGLYSLSS VVTVPSSSLGTQTYICNVNHKPSNTKVDKKVEPKSCDKTHTCPPCPAPELLGGPSVFLFPP KPKDTLMISRTPEVTCVVVDVSHEDPEVKFNWYVDGVEVHNAKTKPREEQYASTYRVVS VLTVLHQDWLNGKEYKCKVSNKALPAPIEKTISKAKGQPREPQVCTLPPSRDELTKNQVS LSCAVKGFYPSDIAVEWESNGQPENNYKTTPPVLDSDGSFFLVSKLTVDKSRWQQGNVFS CSVMHEALHNHYTQKSLSLSPGK |
| 94 | EVQLVESGGGLVQPGGSLRLSCAASGFTFSDSWIHWVRQAPGKGLEWVGWISPYGGSTY YADSYRSRFTISADTSKNTAYLQMNSLRAEDTAVYYCARRHWPGGFDYWGQGTLVTVSS ASTKGPSVFPLAPSSKSTSGGTAALGCLVKDYFPEPVTVSWNSGALTSGVHTFPAVLQSSG LYSLSSVVTVPSSSLGTQTYICNVNHKPSNTKVDKKVEPKSCDKTHTCPPCPAPELLGGPS VFLFPPKPKDTLMISRTPEVTCVVVDVSHEDPEVKFNWYVDGVEVHNAKTKPREEQYAST YRVVSVLTVLHQDWLNGKEYKCKVSNKALPAPIEKTISKAKGQPREPQVCTLPPSRDELT KNQVSLSCAVKGFYPSDIAVEWESNGQPENNYKTTPPVLDSDGSFFLVSKLTVDKSRWQQ GNVFSCSVMHEALHNHYTQKSLSLSPGK |
| 95 | QVQLQESGPGLVKPSETLSLTCTVSGGSLDNYYWSWIRQPPGKGLEWIGYIYYSGNTNYN PSLKSRVTISVDTSKNQFSLKLSSVTAADTAVYYCARGGRFLERYWGQGTLVTVSSASTKG PSVFPLAPSSKSTSGGTAALGCLVKDYFPEPVTVSWNSGALTSGVHTFPAVLQSSGLYSLSS VVTVPSSSLGTQTYICNVNHKPSNTKVDKKVEPKSCDKTHTCPPCPAPELLGGPSVFLFPP KPKDTLMISRTPEVTCVVVDVSHEDPEVKFNWYVDGVEVHNAKTKPREEQYASTYRVVS VLTVLHQDWLNGKEYKCKVSNKALPAPIEKTISKAKGQPREPQVYTLPPCRDELTKNQVS LWCLVKGFYPSDIAVEWESNGQPENNYKTTPPVLDSDGSFFLYSKLTVDKSRWQQGNVFS CSVMHEALHNHYTQKSLSLSPGK |
| 96 | EIVLTQSPSSLSASVGDRVTITCLASQTIGTWLAWYQQKPGKSPQLLIYAASTLQSGVPSRF SGSGSGTDFTLTISSLQPEDVATYYCQQYYSTPRTFGQGTKVEIKRTVAAPSVFIFPPSDEQL KSGTASVVCLLNNFYPREAKVQWKVDNALQSGNSQESVTEQDSKDSTYSLSSTLTLSKAD YEKHKVYACEVTHQGLSSPVTKSFNRGEC |

It will also be appreciated by those of ordinary skills in the art that the sequence of the antibody or the antigen-binding fragment disclosed herein may be replaced, and the replaced amino acid sequence differs from the naturally occurring amino acid sequence of the antibody. For example, the replaced amino acid sequence can be similar to the starting sequence, for example, having a certain proportion of identity to the starting sequence. For example, the identity to the starting sequence may be about 80%, about 85%, about 90%, about 95%, about 98%, about 99%, or a range between any two of these values (inclusive) or any value therein.

In certain embodiments, the amino acid sequence comprised in an antibody has one or more modification groups. For example, the bispecific antibody disclosed herein can be modified to add functional groups (e.g., PEG, drugs, toxins or tags).

The antibody or the antigen-binding fragment disclosed herein includes modified derivatives, i.e., modified by covalent linking of any type of molecule to the antibody, wherein the covalent linking does not prevent the antibody from binding to the epitope. Examples of modification include, but are not limited to, the following: an antibody may be subjected to glycosylation, acetylation, pegylation, phosphorylation, amidation, derivatization by known protecting/blocking groups, proteolytic cleavage, attachment to cellular ligands or other proteins, and the like. Any of numbers of chemical modifications may be made by techniques in the prior art, including but not limited to specific chemical cleavage, acetylation, formylation, metabolic synthesis of tunicamycin, and the like.

In some embodiments, the antibody may be conjugated with a therapeutic agent, a prodrug, a peptide, a protein, an enzyme, a virus, a lipid, a biological response modifier, an agent or PEG.

The antibody may be conjugated with or fused to a therapeutic agent, which may include a detectable label (such as a radiolabel), an immunomodulator, a hormone, an enzyme, an oligonucleotide, a photoactive therapeutic agent, a diagnostic agent, a cytotoxic agent as a drug or a toxin, an ultrasound enhancing agent, a nonradioactive label, a composition thereof, and other such agents known in the art.

The antibody may be detectably labeled by coupling to a chemiluminescent compound. The presence of the chemiluminescent-labeled antibody is then determined by detecting the luminescence that occurs during chemical reactions. Examples of chemiluminescent-labeled compounds include luminol, isoluminol, aromatic acridinium esters, imidazole, acridinium salts and oxalate esters.

### Methods for preparing antibodies and polynucleotides encoding antibodies

The present invention further discloses a polynucleotide or a nucleic acid molecule encoding the antibody, the antigen-binding fragment, and a derivative thereof of the present invention. The polynucleotides disclosed herein may encode the VHa, the VHb, the VLa, the VLb, the CHa, the CHb, the CLa, the CLb, the Fc region, the heavy chain a, the heavy chain b, the light chain, and the like. Methods for preparing antibodies are well known in the art and are described herein. In certain embodiments, the variable and constant regions of the antibody and the antigen-binding fragment disclosed herein are fully human. The fully human antibody and antigen-binding fragment can be prepared using techniques disclosed in the art and described herein. For example, the fully human antibody against a specific antigen can be prepared by administering the antigen to transgenic animals that have been modified to prepare the fully human antibody in response to antigen challenge. Exemplary techniques that can be used to prepare such an antibody are found in U.S. Patent Nos. 6,458,592 and 6,420,140, which are incorporated herein by reference in their entireties. The bispecific antibody of the present invention is prepared by fusing the fragments specifically binding to antigen a and antigen b, and some fragments of the bispecific antibody can be obtained by the above-mentioned method for preparing an antibody binding to a single antigen.

In some embodiments, the prepared antibody does not elicit a deleterious immune response in the animal, e.g., human, to be treated. In one embodiment, the antibody, the antigen-binding fragment or the derivative thereof disclosed herein is modified using techniques well known in the art to reduce the immunogenicity. For example, the antibody can be humanized, primatized or deimmunized, or a chimeric antibody can be prepared. Such antibodies are derived from non-human antibodies, typically murine or primate antibodies, which retain or substantially retain the antigen-binding properties of the parent antibody but are less immunogenic in humans. This can be accomplished by a variety of methods, including: (a) grafting an entire variable region of a non-human origin to a constant region of a human origin to produce a chimeric antibody; (b) grafting at least a portion of one or more non-human complementarity determining regions (CDRs) to framework regions and a constant region of a human origin, with or without retention of critical framework residues; or (c) grafting an entire variable region of a non-human origin, but "hiding" the surface residues by replacing them with portions of a human-like origin. Generally, framework residues in the human framework regions will be substituted with corresponding residues from the CDR donor antibody, preferably residues that may improve the antigen binding. Such framework substitutions can be identified by methods well known in the art, for example, by modeling the interaction of the CDR and framework residues to identify framework residues that play an important role in antigen binding and by sequence alignment to identify abnormal framework residues at particular positions (see U.S. Patent No. 5,585,089, which is incorporated herein by reference in its entirety). Antibodies can be humanized using a variety of techniques well known in the art, such as CDR grafting (EP 239,400; WO 91/09967; U.S. Pat. Nos. 5,225,539, 5,530,101 and 5,585,089), repair or surface rearrangement (EP 592,106; EP 519,596), and chain rearrangement (U.S. Pat. No. 5,565,332), which are incorporated herein by reference in their entireties.

Deimmunization may also be used to reduce the immunogenicity of the antibody. In the present invention, the term "deimmunization" includes the alteration of the antibody to modify T cell epitopes (see, e.g., Pat. Nos. WO/9852976 A1 and WO/0034317 A2). For example, a heavy chain variable region sequence and a light chain variable region sequence from the original antibody are analyzed, and a "map" of human T cell epitopes from each variable region is generated, showing the positions of the epitopes relative to the complementarity determining regions (CDRs) and other critical residues within the sequence. Individual T cell epitopes from the T cell epitope map are analyzed to identify alternative amino acid substitutions with a lower risk of altering antibody activity. A series of alternative heavy chain variable region sequences and light chain variable region sequences comprising combinations of amino acid substitutions are designed and subsequently incorporated into a series of binding polypeptides. The genes of intact heavy and light chains comprising the modified variable regions and human constant regions are cloned into an expression vector, and the plasmid is then transferred into a cell line to produce an intact antibody. The antibodies are compared in appropriate biochemical and biological experiments to identify the optimal antibody.

The binding specificity of the bispecific antibody or the antigen-binding fragment disclosed herein can be detected by an *in vitro* assay, such as co-immunoprecipitation, radioimmunoassay (RIA) or enzyme-linked immunosorbent assay (ELISA).

Alternatively, for scFvs in the bispecific antibody of the present invention, reference can be made to the technology for the production of single-chain units (U.S. Pat. No. 4,694,778). Single-chain units are formed by linking the heavy and light chain fragments of the Fv region via an amino acid bridge, resulting in a single-chain fusion peptide. Techniques for the assembly of functional Fv fragments in *E. coli* may also be used (Skerra et al., Science 242: 1038-1041 (1988)). scFvs may be obtained by yeast display. The yeast display vector may be the pYD1 vector (Addgene), and the host saccharomyces cerevisiae may be saccharomyces cerevisiae EBY100 (Invitrogen).

Examples of techniques that may be used to produce single chain Fvs (scFvs) and antibodies include those described in U.S. Pat. Nos. 4,946,778 and 5,258,498. For certain use, including *in vivo* use of antibodies in humans and *in vitro* detection assays, a chimeric antibody, a humanized antibody or a fully human antibody may be used. Chimeric antibodies are molecules in which different portions of the antibody are derived from different animal species, such as antibodies having variable regions of a murine monoclonal antibody and constant regions of a human immunoglobulin. Methods for producing chimeric antibodies are known in the art. See U.S. Pat. Nos. 5,807,715, 4,816,567 and 4,816,397, which are incorporated herein by reference in their entireties.

In addition, another efficient method for producing recombinant antibodies, which in particular is capable of producing primate antibodies comprising monkey variable region and human constant region sequences, is disclosed in Newman, Biotechnology 10: 1455-1460 (1992), which is incorporated herein by reference in its entirety. Furthermore, this technique is also described in commonly assigned U.S. Pat. Nos. 5,658,570, 5,693,780 and 5,756,096, each of which is incorporated herein by reference in its entirety.

The antibodies can be prepared by a variety of methods known in the art, including phage display methods using antibody libraries from immunoglobulin sequences. Reference may also be made to U.S. Pat. Nos. 4,444,887 and 4,716,111, and PCT Publication Nos. WO 98/46645, WO 98/50433, WO 98/24893, WO 98/16654, WO 96/34096, WO 96/33735 and WO 91/10741, each of which is incorporated herein by reference in its entirety.

Fully human antibodies that recognize selective epitopes can be produced using a technique known as "guided selection". In this method, a selected non-human monoclonal antibody such as a mouse antibody is used to guide the screening of fully human antibodies that recognize the same epitope (see U.S. Pat. No. 5,565,332, which is incorporated herein by reference in its entirety).

In another embodiment, DNA encoding the desired monoclonal antibody can be isolated and sequenced using conventional methods (e.g., using oligonucleotide probes that can specifically bind to genes encoding the heavy and light chains of a murine antibody). Isolated and subcloned hybridoma cells can be sources of such DNA. Once isolated, the DNA can be inserted into an expression vector and then transfected into prokaryotic or eukaryotic host cells such as *E. coli* cells, simian COS cells, Chinese hamster ovary (CHO) cells, or myeloma cells that do not produce other immunoglobulins. The isolated DNA (which may be synthetic as described herein) can be used to prepare sequences of the constant and variable regions of an antibody, as described in U.S. Pat. No. 5,658,570, which is incorporated herein by reference in its entirety. This method extracts RNA from the selected cells and converts it into cDNA, followed by amplification by PCR using Ig-specific primers. Suitable probes for this purpose are also described in U.S. Pat. No. 5,658,570.

In addition, by using conventional recombinant DNA techniques, one or more CDRs of the antibody of the present invention can be inserted into framework regions, e.g., into human framework regions to construct a humanized non-fully human antibody. The framework regions may be naturally occurring or consensus framework regions, preferably human framework regions (see Chothia et al., J. Mol. Biol. 278:457-479 (1998), in which a range of human framework regions are listed). Some polynucleotides may encode antibodies produced by the combination of framework regions and CDRs that specifically bind to at least one epitope of a target antigen. One or more amino acid substitutions may be made within the framework regions, and amino acid substitutions capable of improving the binding of the antibody to the antigen thereof may be selected. Additionally, substitution or deletion of one or more cysteine residues in the variable regions involved in interchain disulfide bond formation can be made in this manner, thereby producing an antibody molecule lacking one or more interchain disulfide bonds. Other alterations to the polynucleotides made within the technology scope of the art are also encompassed by the present invention.

Antibodies can be prepared by using conventional recombinant DNA techniques. Vectors and cell lines for antibody production can be selected, constructed and cultured using techniques well known to those skilled in the art. These techniques are described in various laboratory manuals and main publications, such as Recombinant DNA Technology for Production of Protein Therapeutics in Cultured Mammalian Cells, D. L. Hacker, F. M. Wurm, Reference Module in Life Sciences, 2017, which is incorporated herein by reference in its entirety (including the supplements).

In some embodiments, the DNA encoding the antibody can be designed and synthesized according to the antibody amino acid sequence described herein by conventional methods, inserted into an expression vector, and transfected into a host cell. The transfected host cell is then cultured in a medium to produce the antibody. The DNAs expressing the heavy chain and the light chain of the antibody may be placed in the same vector or placed in different vectors; if placed in different vectors, the vectors expressing the heavy and light chains of the antibody may transfect host cells in an appropriate ratio (e.g., Tihomir S. Dodev et al., A tool kit for rapid cloning and expression of recombinant antibodies, Scientific Reports, volume 4, Article number: 5885 (2014); Stefan Schlatter et al., On the Optimal Ratio of Heavy to Light Chain Genes for Efficient Recombinant Antibody Production by CHO Cells, Biotechnol Progress, 21: 122-133 (2005); Hadi Bayat et al., Evaluation of different vector design strategies for the expression of recombinant monoclonal antibody in CHO cells, Preparative Biochemistry & Biotechnology, 48(8): 822-829 (2018)). In some embodiments, the vector expressing the antibody comprises at least one promoter element, an antibody encoding sequence, a transcription termination signal and a polyA tail. Other elements include enhancers, Kozak sequences, and donor and recipient sites flanking the inserted sequence for RNA splicing. Efficient transcription can be obtained by early and late promoters of SV40, long terminal repeats from retroviruses such as RSV, HTLV1 and HIVI, and early promoters of cytomegalovirus, and promoters from other cells such as actin promoter can also be used. Suitable expression vectors may include pIRES1neo, pRetro-Off, pRetro-On, PLXSN, Plncx, pcDNA3.1 (+/-), pcDNA/Zeo (+/-), pcDNA3.1/Hygro(+/-), PSVL, PMSG, pRSVcat, pSV2dhfr, pBC12MI, pCS2, etc. Commonly used mammalian cells include 293 cells, Cos1 cells, Cos7 cells, CV1 cells, murine L cells, CHO cells and the like.

In some embodiments, the inserted gene fragment should comprise a screening label, common ones of which include screening genes such as dihydrofolate reductase, glutamine synthetase, neomycin resistance and hygromycin resistance, to facilitate the screening and separation of cells that have been successfully transfected. The constructed plasmid is transfected to a host cell without the above genes, and the successfully transfected cells are cultured in a large quantity in a selective culture medium to produce the desired target protein.

Methods for preparing bispecific antibodies are documented in large quantity in the literature, e.g., Qiong Wang, et al., Design and Production of Bispecific Antibodies, Antibodies, 8, 43 (2019); Zhuang Zuo, et al., An efficient route to the production of an IgG-like bispecific antibody, Protein Engineering, Design and Selection, 13(5): 361-367 (2000); Matthias Mack, et al., A small bispecific antibody construct expressed as a functional single-chain molecule with high tumor cell cytotoxicity, Proc. Natl. Acad. Sci., 92: 7021-7025, (1995); Rodrigo Vazquez-Lombardi, et al., Transient expression of human antibodies in mammalian cells, Nature Protocols 13(1): 99-117 (2018); Elisa Corsiero, Monoclonal Antibodies: Expression and Purification in a Basic Research Laboratory, Mater Methods, 6:1481 (2016).

In addition, mutations can be introduced in the nucleotide sequence encoding the antibody disclosed herein using standard techniques known to those skilled in the art, including but not limited to site-directed mutations resulting in amino acid substitutions and PCR-mediated mutations. The variant (including derivative) encodes a substitution of less than 50 amino acids, a substitution of less than 40 amino acids, a substitution of less than 30 amino acids, a substitution of less than 25 amino acids, a substitution of less than 20 amino acids, a substitution of less than 15 amino acids, a substitution of less than 10 amino acids, a substitution of less than 5 amino acids, a substitution of less than 4 amino acids, a substitution of less than 3 amino acids or a substitution of less than 2 amino acids relative to the VH CDR1, VH CDR2 and VH CDR3 of the original heavy chain variable region and the VL CDR1, VL CDR2 or VL CDR3 of the light chain variable region. Alternatively, mutations can be introduced randomly along all or part of the encoding sequence, for example by saturation mutagenesis, and the resulting mutants can be screened for the biological activity to identify mutants that retain the activity.

In some embodiments, the substitutions described herein are conservative amino acid substitutions.

### Treatment method

The present invention further provides a treatment method and use. In some embodiments, provided is a method for treating or ameliorating various types of cancers, tumors, infections and other related diseases, wherein the method comprises administering to a patient an effective dose of the bispecific antibody. In some embodiments, provided is use of the bispecific antibody in the treatment or amelioration of cancers, tumors, infections and other related diseases. In some embodiments, provided is use of the bispecific antibody in the manufacture of a medicament for treating or ameliorating cancers, tumors, infections and other related diseases.

The present invention relates to: a method for treating related diseases whose therapeutic targets are PD-L1 and/or CD47; a method used for any disease or disorder that can be ameliorated, slowed, inhibited or prevented by eliminating, inhibiting or reducing binding of PD-L1 to PD1 and/or binding of CD47 to SIRPα; a method for treating cancer or a tumor in a subject, a method for alleviating symptoms of cancer or a tumor in a subject, or a method for avoiding recurrence of a tumor or cancer in a subject, wherein the method comprises administering to the subject an effective amount of any of the anti-PD-L1/CD47 bispecific antibodies or the fragments thereof described herein.

The antibody and the antigen-binding fragment thereof and the pharmaceutical composition comprising the same provided herein can be used as a therapeutic agent for diagnosis, prognosis, monitoring, treatment, alleviation and/or prevention of diseases and disorders related to abnormal expression, activity and/or signaling of PD-L1 and/or CD47 in a subject. When a disease and a disorder related to abnormal expression, activity and/or signaling of PD-L1 and/or CD47 in a subject are identified by using a standard method, the monoclonal antibody, the bispecific antibody and the antigen-binding fragment thereof and the pharmaceutical composition comprising the same disclosed herein may be administered.

Given that most of the CD47 antibodies disclosed in the prior art are capable of causing hemagglutination of human red blood cells, there is still an urgent need at present to obtain a novel anti-CD47 antibody that not only can effectively promote the phagocytosis of macrophages but also does not cause the cell agglutination. The anti-PD-L1/CD47 bispecific antibody disclosed herein meets this need. It can effectively promote phagocytosis, does not cause significant agglutination of red blood cells, and more preferably, does not bind to human red blood cells significantly.

In some embodiments, the anti-PD-L1/CD47 bispecific antibody of the present invention can bind to PD-L1 with ultra-high affinity and bind to CD47 with high affinity or moderate affinity; the selective binding of the anti-PD-L1/CD47 bispecific antibody of the present invention to the tumor cells is promoted by the specific binding to PD-L1 on the tumor cells, the binding to CD47 expressed in many normal tissues is avoided, and the side effects are reduced; the effective dose range of the anti-PD-L1/CD47 bispecific antibody of the present invention can be remarkably expanded based on the fact that the affinity for CD47 is far lower than that for PD-L1.

In some embodiments, cancers treated and/or prevented with the antibody described herein include, but are not limited to, solid tumors, hematological cancers (e.g., leukemia, lymphoma or myeloma, such as multiple myeloma), and metastatic lesions. In one embodiment, the cancer is a solid tumor. Examples of solid tumors include malignancies, such as sarcomas and cancers of multiple organ systems, for example, those cancers that invade the lung, breast, ovary, lymphoid, gastrointestinal tract (e.g., colon), anus, genital and genitourinary tract (e.g., kidney, bladder epithelium, bladder cells, and prostate), pharynx, CNS (e.g., brain or neurological or glial cells), head and neck, skin (e.g., melanoma), nasopharynx (e.g., differentiated or undifferentiated metastatic or locally recurrent nasopharyngeal carcinoma) and pancreas. The cancer may be at an early, intermediate or advanced stage, or may be metastatic cancer. In one embodiment, the tumor is tumor immune escape. In some embodiments, the tumor is a gastrointestinal tumor (e.g., cancer), such as colon cancer.

The specific dose and regimen for any particular patient will depend on a variety of factors including the particular antibody or derivative thereof used, the age and body weight, general health condition, sex and diet of the patient, and the time of administration, frequency of excretion, drug combination and the severity of the particular disease being treated. These factors are judged by medical caregivers included within the scope of those of ordinary skills in the art. The dose will also depend on the individual patient to be treated, the route of administration, the type of the formulation, the nature of the compound used, the severity of the disease and the efficacy desired. The dose employed can be determined by pharmacological and pharmacokinetic principles well known in the art. In some embodiments, the effective dose ranges from about 0.01 mg/kg to about 100 mg/kg, and can be, for example, twice a week (BIW) or once a month.

The modes of administration for the antibody or the derivative include, but are not limited to, intradermal, intramuscular, intraperitoneal, intravenous, subcutaneous, nasal, spinal epidural and oral injection. The pharmaceutical composition may be administered by any convenient route, e.g., by infusion or bolus injection, by absorption through epithelial or cutaneous mucosa (e.g., oral mucosa or rectal and intestinal mucosa), and may be co-administered with other biologically active agents. Thus, the pharmaceutical composition comprising the antibody of the present invention can be administered orally, rectally, parenterally, intracisternally, intravaginally, intraperitoneally, topically (e.g. by powder, ointment, drop or transdermal patch) or buccally, or by oral or nasal spray.

The term "parenteral" as used herein refers to modes of administration including intravenous, intramuscular, intraperitoneal, intrasternal, subcutaneous and intraarticular injections and infusions.

The mode of administration may be systemic or local. In addition, it may be desirable to introduce the antibody of the present invention into the central nervous system by any suitable route, including intracerebroventricular and intrathecal injections; intracerebroventricular injection may be assisted by an intracerebroventricular catheter connected to, for example, a reservoir (which may be an Ommaya reservoir). Pulmonary administration is also possible, for example by use of an inhaler or nebulizer, and by use of nebulized formulations.

The antibodies of the present invention may be administered locally to an area in need of treatment; this may be achieved by (but not limited to) the following: local infusion during surgery (e.g., topical application in combination with a post-operative wound dressing), by injection, by means of a catheter, by means of a suppository, or by means of an implant, the implant being of a porous, non-porous, or gelatinous material, including membranes (such as silicone rubber membranes) or fibers. Preferably, when administering a protein (including an antibody) of the present invention, care must be taken to use materials that do not absorb the protein.

Methods for treating diseases comprising administering the antibody or the derivative described herein are generally tested *in vitro,* followed by *in vivo* tests for desired therapeutic or prophylactic activities in an acceptable animal model, and finally administration in humans. Suitable animal models (including transgenic animals) are well known to those of ordinary skills in the art. For example, *in vitro* assays for demonstrating therapeutic use of the antibody or the antigen-binding fragment described herein include the effect of the antibody on a cell line or a patient tissue sample. The effect of the antibody on the cell line and/or the tissue sample can be detected using techniques known to those skilled in the art, such as the techniques disclosed elsewhere herein. In accordance with the teachings of the present invention, *in vitro* assays that can be used to determine whether to administer a specific antibody include *in vitro* cell culture experiments, wherein a patient tissue sample is cultured in a culture medium and is exposed to or otherwise administered a compound, and the effect of the compound on the tissue sample is observed.

Various known delivery systems can be used to administer the antibody or the derivative, or the polynucleotide encoding the same of the present invention, e.g., encapsulation in liposomes, microparticles, microcapsules, recombinant cells capable of expressing the compound, receptor-mediated endocytosis (see, e.g., Wu and Wu, 1987, J. Biol. Chem. 262:4429-4432), or construction of nucleic acids as part of a retrovirus or other vectors.

### Combination therapy

In some embodiments, the antibody of the present invention can be used in combination with other therapeutic or prophylactic regimens, including administration of one or more antibodies of the present invention together with or in combination with one or more other therapeutic agents or methods. For combination therapy, the antibody may be administered simultaneously or separately with other therapeutic agents. When administered separately, the antibody of the present invention can be administered before or after administration of another therapeutic agent.

In some embodiments, when the bispecific antibody of the present invention is administered to a patient, the antibody molecule or pharmaceutical composition or immunoconjugate disclosed herein and one or more other therapies, such as therapeutic modalities and/or other therapeutic agents (e.g., a chemotherapeutic agent, a radiotherapeutic agent, or a biomacromolecule drug), may also be administered in combination to the patient.

Such combination therapies encompass both combined administration (wherein two or more therapeutic agents are contained in the same formulation or separate formulations), and separate administrations (wherein the antibody of the present invention can be administered prior to, concurrently with, and/or subsequent to the administration of other therapies, e.g., therapeutic modalities or therapeutic agents). The antibody molecule and/or other therapies, e.g., therapeutic agents or therapeutic modalities, can be administered when a disease is active or when the disease is in remission or less active. The antibody molecule can be administered prior to, concurrently with or subsequent to other therapies, or during remission of a disease.

In some embodiments, the bispecific antibody of the present invention is administered in combination with a chemotherapeutic agent. In some embodiments, chemotherapeutic agents that can be administered with the anti-PD-L1/CD47 bispecific antibody of the present invention include, but are not limited to, antibiotic derivatives (e.g., doxorubicin, bleomycin, daunorubicin and actinomycin D), antiestrogens (e.g., tamoxifen), antimetabolites (e.g., fluorouracil, 5-FU, methotrexate, floxuridine, interferon α-2b, glutamic acid, mithramycin, mercaptopurine and 6-thioguanine), cytotoxic agents (e.g., carmustine, BCNU, lomustine, CCNU, cytarabine, cyclophosphamide, estramustine, hydroxyurea, procarbazine, mitomycin, busulfan, cisplatin and vincristine sulfate), hormones (e.g., medroxyprogesterone, estramustine sodium phosphate, ethinylestradiol, estradiol, megestrol acetate, methyltestosterone, diethylstilbestrol diphosphate, chlorotrianisene and testolactone), nitrogen mustard derivatives (e.g., melphalan, chlorambucil, dichloromethyl diethylamine (mechlorethamine) and thiotepa), steroids and combinations thereof (e.g., betamethasone sodium phosphate), and other compounds (e.g., dacarbazine, asparaginase, mitotane, vincristine sulfate, vinblastine sulfate and etoposide).

In some embodiments, the antibody of the present invention is administered in combination with cytokines. Cytokines that can be administered with the antibody of the present invention include, but are not limited to, IL-2, IL-3, IL-4, IL-5, IL-6, IL-7, IL-10, IL-12, IL-13, IL-15, and the like.

In some embodiments, the bispecific antibody of the present invention is administered in combination with a biomacromolecule drug. Examples of biomacromolecule drugs include immunotherapeutic agents, including but not limited to therapeutic antibodies suitable for treating a patient. Some examples of therapeutic antibodies include simtuzumab, abagovomab, adecatumumab, afutuzumab, alemtuzumab, altumomab, amatuximab, anatumomab, arcitumomab, bavituximab, bectumomab, bevacizumab, bivatuzumab, blinatumomab, brentuximab, cantuzumab, catumaxomab, cetuximab, citatuzumab, cixutumumab, clivatuzumab, conatumumab, daratumumab, drozitumab, duligotumab, dusigitumab, detumomab, dacetuzumab, dalotuzumab, ecromeximab, elotuzumab, ensituximab, ertumaxomab, etaracizumab, farletuzumab, ficlatuzumab, figitumumab, flanvotumab, futuximab, ganitumab, gemtuzumab, girentuximab, glembatumumab, ibritumomab, igovomab, imgatuzumab, indatuximab, inotuzumab, intetumumab, ipilimumab, iratumumab, labetuzumab, lexatumumab, lintuzumab, lorvotuzumab, lucatumumab, mapatumumab, matuzumab, milatuzumab, minretumomab, mitumomab, moxetumomab, narnatumab, naptumomab, necitumumab, nimotuzumab, nofetumomab, ocaratuzumab, ofatumumab, olaratumab, onartuzumab, oportuzumab, oregovomab, panitumumab, parsatuzumab, patritumab, pemtumomab, pertuzumab, pintumomab, pritumumab, racotumomab, radretumab, rilotumumab, rituximab, robatumumab, satumomab, sibrotuzumab, siltuximab, solitomab, tacatuzumab, taplitumomab, tenatumomab, teprotumumab, tigatuzumab, tositumomab, trastuzumab, tucotuzumab, ublituximab, veltuzumab, vorsetuzumab, votumumab, zalutumumab, and the like.

In some embodiments, the antibody of the present invention can be used with an immune checkpoint inhibitor. In some embodiments, the antibody of the present invention is administered in combination with other therapeutic or prophylactic regimens, such as radiotherapy.

### Pharmaceutical composition

The present invention further provides a pharmaceutical composition. Such a composition comprises an effective dose of an antibody or an antigen-binding fragment and a pharmaceutically acceptable carrier. In some embodiments, the pharmaceutical composition further comprises an anti-cancer agent (e.g., an immune checkpoint inhibitor).

In some embodiments, the term "pharmaceutically acceptable" refers to a substance approved by a government regulatory agency or listed in commonly-recognized pharmacopeias for use in animals, and particularly in humans. In addition, the "pharmaceutically acceptable carrier" generally refers to a non-toxic solid, semi-solid or liquid filler, diluent, encapsulating material or formulation auxiliary of any type.

The term "carrier" refers to a diluent, an adjuvant, an excipient or a carrier that can be administered to a patient together with the active ingredient. Such pharmaceutical carriers can be sterile liquids, such as water and oils, including oils originating from petroleum, animal, plant or synthesis, such as peanut oil, soybean oil, mineral oil, sesame oil, etc. Water is a preferred carrier when the pharmaceutical composition is administered intravenously. A saline aqueous solution, a glucose aqueous solution, and a glycerol solution can also be used as a liquid carrier, especially for injection. Suitable pharmaceutical excipients include starch, glucose, lactose, sucrose, gelatin, malt, rice, flour, chalk, silica gel, sodium stearate, glycerol monostearate, talc, sodium chloride, skimmed milk powder, glycerol, propylene, glycol, water, ethanol and the like. If desired, the composition may also comprise a small amount of a wetting agent, an emulsifier, or a pH buffering agent such as acetate, citrate or phosphate. Antibacterials such as benzyl alcohol or methylparaben, antioxidants such as ascorbic acid or sodium bisulfite, chelating agents such as ethylenediamine tetraacetic acid, and tonicity adjusting agents such as sodium chloride or dextrose are also contemplated. Such compositions may be in the form of solutions, suspensions, emulsions, tablets, pills, capsules, pulvises, sustained-release formulations and the like. The composition may be formulated as a suppository using conventional binders and carriers such as triglycerides. Oral formulations may comprise standard carriers such as mannitol, lactose, starch, magnesium stearate, sodium saccharin, cellulose and magnesium carbonate of pharmaceutical grade. Examples of suitable pharmaceutical carriers are described in Remington's Pharmaceutical Sciences by E. W. Martin, which is hereby incorporated herein by reference. Such compositions will contain a clinically effective dose of an antibody or an antigen-binding fragment, preferably in purified form, together with an appropriate amount of a carrier, to provide a form suitable for administration to a patient. The formulation should be suitable for the administration mode. The formulation may be enclosed in ampules, disposable syringes or multiple dose vials made of glass or plastic.

In some embodiments, the composition is formulated into a pharmaceutical composition suitable for intravenous injection into a human body according to conventional steps. A composition for intravenous administration is usually a solution in sterile isotonic aqueous buffer. The composition may also comprise a solubilizer and a local anesthetic such as lidocaine to alleviate the pain at the injection site. In general, the active ingredients are provided in a unit dosage form individually or as a mixture. For example, the active ingredients are encapsulated in sealed containers (such as ampoule bottles or sachets) that can indicate the amount of the active agent, in the form of lyophilized powder or anhydrous concentrate. Where the composition is administered by infusion, the composition can be dispensed in infusion bottles containing sterile water or saline of pharmaceutical grade. Where the composition is administrated by injection, an ampoule bottle containing sterile water or saline for injection can be used, so that the active ingredients can be mixed before administration.

The compound of the present invention may be formulated in a neutral or salt form. Pharmaceutically acceptable salts include salts formed with anions derived from acids such as hydrochloric acid, phosphoric acid, acetic acid, oxalic acid, tartaric acid, etc., and salts formed with cations derived from, e.g., sodium, potassium, ammonium, calcium, iron hydroxide, isopropylamine, triethylamine, 2-ethylaminoethanol, histidine, procaine, etc.

### Examples

The technical schemes of the present invention will be further illustrated below through specific examples, which are not intended to limit the protection scope of the present invention. Some nonessential modifications and adjustments made by other people according to the concept of the present invention still fall within the protection scope of the present invention.

### Example 1: Preparation of Antigens

Preparation of CD47-His antigen: a CD47 recombinant protein was constructed by adding 8×HIS tag (SEQ ID NO: 3) to the C-terminus of the human CD47 extracellular domain (ECD) protein (SEQ ID NO: 1). The ECD region of human CD47 was genetically synthesized and subcloned into a mammalian expression vector (e.g., pcDNA3.1(+) expression vector). After transient transfection into HEK293F cells, a CD47-His antigen purified by nickel-based immobilized metal affinity chromatography (GE Healthcare) was obtained.

Preparation of PD-L1-His antigen: a PD-L1 recombinant protein was constructed by adding 8×HIS tag (SEQ ID NO: 3) to the C-terminus of the human PD-L1 extracellular domain (ECD) protein (SEQ ID NO: 2). The ECD region of human PD-L1 was genetically synthesized and subcloned into a mammalian expression vector (e.g., pcDNA3.1(+) expression vector). After transient transfection into HEK293F cells, a PD-L1-His antigen purified by nickel-based immobilized metal affinity chromatography (GE Healthcare) was obtained.
CD47-ECD (SEQ ID NO: 1): 
MWPLVAALLLGSACCGSAQLLFNKTKSVEFTFCNDTVVIPCFVTNMEAQNTTEVYVK
WKFKGRDIYTFDGALNKSTVPTDFSSAKIEVSQLLKGDASLKMDKSDAVSHTGNYTC
EVTELTREGETIIELKYRVVSWFSP
PD-L1-ECD (SEQ ID NO: 2): 
MRIFAVFIFMTYWHLLNAFTVTVPKDLYVVEYGSNMTIECKFPVEKQLDLAALIVYW
EMEDKNIIQFVHGEEDLKVQHSSYRQRARLLKDQLSLGNAALQITDVKLQDAGVYR
CMISYGGADYKRITVKVNAPYNKINQRILVVDPVTSEHELTCQAEGYPKAEVIWTSSD
HQVLSGKTTTTNSKREEKLFNVTSTLRINTTTNEIFYCTFRRLDPEENHTAELVIPELPL
AHPPNER
8×HIS (SEQ ID NO: 3):
   HHHHHHHH

### Example 2: Preparation of Antibodies

### 2.1. Anti-PD-L1 scFv displayed in yeast

The nucleic acid sequence encoding an scFv fragment (including a heavy chain variable region, a light chain variable region, and linker of the heavy chain variable region and the light chain variable region (G₄S)₃) of an anti-PD-L1 antibody was synthesized, inserted into a vector, and transfected into host saccharomyces cerevisiae by an electroporation apparatus to obtain positive yeast clones. The positive yeast clones obtained above were picked out and subjected to monoclonal culture. The heavy chain variable region and the light chain variable region of the scFv expressed on the surface of the ATE positive yeast clone were consistent with those of atezolizumab, the scFvs expressed on the surfaces of other positive yeast clones are shown in Table 1, the corresponding sequence of each VH SEQ ID NO: is shown in Table 2, and corresponding CDRs are shown in Table 3. The amino acid sequence of the linker is GGGGSGGGGSGGGGS, and the nucleic acid sequence thereof is ggtggaggcggttcaggcggaggtggctctggcggtggcggatcg.

**Table 1: scFvs displayed by positive yeast clones**

| No. | VH SEQ ID NO: | VL SEQ ID NO: |
|---|---|---|
| CP11-27 | 49 | 75 |
| CP11-36 | 50 | 75 |
| CP11-46 | 51 | 75 |
| CP11-47 | 52 | 75 |
| CP11-55 | 53 | 75 |
| CP11-71 | 54 | 75 |
| CP14-16 | 55 | 75 |
| CP14-80 | 56 | 75 |
| CP21-8 | 57 | 75 |
| CP21-47 | 58 | 75 |
| CP21-59 | 59 | 75 |
| CP22-34 | 60 | 75 |
| CP22-40 | 61 | 75 |
| CP22-44 | 62 | 75 |
| CP22-45 | 63 | 75 |

**Table 2: Heavy chain variable region sequences of anti-PD-L 1 antibody**

| Name | Variable region sequence | SEQ ID NO: |
|---|---|---|
| CP11-27 | EVQLVESGGGLVQPGGSLRLSCAASGFTFSDSWIHWVRQAPGKGLEWVG WISPYGGSTYYADSVKGRFTISADTSKNTAYLQMNSLRAEDTAVYYCARR HWPGGFDYWGQGTLVTVSS | 49 |
| CP11-36 | EVQLVESGGGLVQPGGSLRLSCAASGFTFSDSWIHWVRQAPGKGLEWVG WISPYGGSTYYADDFRHRFTISADTSKNTAYLQMNSLRAEDTAVYYCARR HWPGGFDYWGQGTLVTVSS | 50 |
| CP11-46 | EVQLVESGGGLVQPGGSLRLSCAASGFTFSDSWIHWVRQAPGKGLEWVG WISPYGGSTYYADSLGDRFTISADTSKNTAYLQMNSLRAEDTAVYYCARRH WPGGFDYWGQGTLVTVSS | 51 |
| CP11-47 | EVQLVESGGGLVQPGGSLRLSCAASGFTFSDSWIHWVRQAPGKGLEWVG WISPYGGSTYYADHLSQRFTISADTSKNTAYLQMNSLRAEDTAVYYCARRH WPGGFDYWGQGTLVTVSS | 52 |
| CP11-55 | EVQLVESGGGLVQPGGSLRLSCAASGFTFSDSWIHWVRQAPGKGLEWVG WISPYGGSTYYADGMRSRFTISADTSKNTAYLQMNSLRAEDTAVYYCARR HWPGGFDYWGQGTLVTVSS | 53 |
| CP11-71 | EVQLVESGGGLVQPGGSLRLSCAASGFTFSDSWIHWVRQAPGKGLEWVG WISPYGGSTYYADSYRSRFTISADTSKNTAYLQMNSLRAEDTAVYYCARRH WPGGFDYWGQGTLVTVSS | 54 |
| CP14-16 | EVQLVESGGGLVQPGGSLRLSCAASGFTFSDSWIHWVRQAPGKGLEWVG WISPYGGSTYYADPYVGRFTISADTSKNTAYLQMNSLRAEDTAVYYCARR HWPGGFDYWGQGTLVTVSS | 55 |
| CP14-80 | EVQLVESGGGLVQPGGSLRLSCAASGFTFSDSWIHWVRQAPGKGLEWVG WISPYGGSTYYADSLKGRFTISADTSKNTAYLQMNSLRAEDTAVYYCARRH WPGGFDYWGQGTLVTVSS | 56 |
| CP21-8 | EVQLVESGGGLVQPGGSLRLSCAASGFTFSDSWIHWVRQAPGKGLEWVG WISPYGGSTYYADSYRARFTISADTSKNTAYLQMNSLRAEDTAVYYCARR HWPGGFDYWGQGTLVTVSS | 57 |
| CP21-47 | EVQLVESGGGLVQPGGSLRLSCAASGFTFSDSWIHWVRQAPGKGLEWVG WISPYGGSTYYADYLHGRFTISADTSKNTAYLQMNSLRAEDTAVYYCARR HWPGGFDYWGQGTLVTVSS | 58 |
| CP21-59 | EVQLVESGGGLVQPGGSLRLSCAASSFSLKDSWIHWVRQAPGKGLEWVG WISPYGGSTYYADSVKGRFTISADTSKNTAYLQMNSLRAEDTAVYYCARR HWPGGFDYWGQGTLVTVSS | 59 |
| CP22-34 | EVQLVESGGGLVQPGGSLRLSCAASGWATRDSWIHWVRQAPGKGLEWVG WISPYGGSTYYADSVKGRFTISADTSKNTAYLQMNSLRAEDTAVYYCARR HWPGGFDYWGQGTLVTVSS | 60 |
| CP22-40 | EVQLVESGGGLVQPGGSLRLSCAASGFTFSDSWIHWVRQAPGKGLEWVG WISPYGGSTYYADSVKGRFTISADTSKNTAYLQMNSLRAEDTAVYYCARR HWPGGLLPWGQGTLVTVSS | 61 |
| CP22-44 | EVQLVESGGGLVQPGGSLRLSCAASGLRPADSWIHWVRQAPGKGLEWVG WISPYGGSTYYADSVKGRFTISADTSKNTAYLQMNSLRAEDTAVYYCARR HWPGGFDYWGQGTLVTVSS | 62 |
| CP22-45 | EVQLVESGGGLVQPGGSLRLSCAASNSHLRDSWIHWVRQAPGKGLEWVG WISPYGGSTYYADSVKGRFTISADTSKNTAYLQMNSLRAEDTAVYYCARR HWPGGFDYWGQGTLVTVSS | 63 |

**Table 3: CDR sequences of heavy chain variable regions of anti-PD-L1 antibody**

| Name | VH CDR1 | SEQ ID NO: | VH CDR2 | SEQ ID NO: | VH CDR3 | SEQ ID NO: |
|---|---|---|---|---|---|---|
| CP11-27 | GFTFSDSWIH | 4 | GWISPYGGSTYYADSVKG | 9 | RHWPGGFDY | 19 |
| CP11-36 | GFTFSDSWIH | 4 | GWISPYGGSTYYADDFRH | 10 | RHWPGGFDY | 19 |
| CP11-46 | GFTFSDSWIH | 4 | GWISPYGGSTYYADSLGD | 11 | RHWPGGFDY | 19 |
| CP11-47 | GFTFSDSWIH | 4 | GWISPYGGSTYYADHLSQ | 12 | RHWPGGFDY | 19 |
| CP11-55 | GFTFSDSWIH | 4 | GWISPYGGSTYYADGMRS | 13 | RHWPGGFDY | 19 |
| CP11-71 | GFTFSDSWIH | 4 | GWISPYGGSTYYADSYRS | 14 | RHWPGGFDY | 19 |
| CP14-16 | GFTFSDSWIH | 4 | GWISPYGGSTYYADPYVG | 15 | RHWPGGFDY | 19 |
| CP14-80 | GFTFSDSWIH | 4 | GWISPYGGSTYYADSLKG | 16 | RHWPGGFDY | 19 |
| CP21-8 | GFTFSDSWIH | 4 | GWISPYGGSTYYADSYRA | 17 | RHWPGGFDY | 19 |
| CP21-47 | GFTFSDSWIH | 4 | GWISPYGGSTYYADYLHG | 18 | RHWPGGFDY | 19 |
| CP21-59 | SFSLKDSWIH | 5 | GWISPYGGSTYYADSVKG | 9 | RHWPGGFDY | 19 |
| CP22-34 | GWATRDSWIH | 6 | GWISPYGGSTYYADSVKG | 9 | RHWPGGFDY | 19 |
| CP22-45 | NSHLRDSWIH | 7 | GWISPYGGSTYYADSVKG | 9 | RHWPGGFDY | 19 |
| CP22-44 | GLRPADSWIH | 8 | GWISPYGGSTYYADSVKG | 9 | RHWPGGFDY | 19 |
| CP22-40 | GFTFSDSWIH | 4 | GWISPYGGSTYYADSVKG | 9 | RHWPGGLLP | 20 |

### 2.2. Anti-CD47 antibodies and anti-PD-L1 antibodies, and preparation thereof

### 2.2.1. Anti-CD47 antibodies

Combinations of VH and VL for 11 IgG1 complete antibodies are shown in Table 4; VH and CH constitute the heavy chains of the antibodies, and VL and CL constitute the light chains of the antibodies; The sequence of CH is set forth in SED ID NO: 81, and the sequence of CL is set forth in SED ID NO: 82. The IgG1 complete antibodies are indicated by "antibody" + antibody number, e.g., antibody 17 (the heavy chain is composed of the VH set forth in SED ID NO: 65 and the CH set forth in SED ID NO: 81, and the light chain is composed of the VL set forth in SED ID NO: 98 and the CL set forth in SED ID NO: 82).

Corresponding sequences of the VHs and VLs in Table 4 are shown in Table 5, wherein the heavy chain CDRs are shown in Table 6.

**Table 4: Variable region combinations of anti-CD47 antibodies**

| Antibody No. | VH SEQ ID NO: | VL SEQ ID NO: | | Antibody No. | VH SEQ ID NO: | VL SEQ ID NO: |
|---|---|---|---|---|---|---|
| L12-6-1 | 64 | 97 | | 10 | 70 | 98 |
| 17 | 65 | 98 | | 16 | 71 | 98 |
| 3-3 | 66 | 98 | | 18 | 72 | 98 |
| 3-6 | 67 | 98 | | 24 | 73 | 98 |
| 3 | 68 | 98 | | 25 | 74 | 98 |
| 6 | 69 | 98 | | L12-6 | 64 | 102 |

**Table 5: Variable region sequences of antibodies**

| Name | Sequence | SEQ ID NO: |
|---|---|---|
| L12-6-VH/L 12-6-1-VH | QVQLQESGPGLVKPSETLSLTCTVSGGSLDNYYWSWIRQPPGKGLEWI GYIYYSGNTNYNPSLKSRVTISVDTSKNQFSLKLSSVTAADTAVYYCA RGGRFLERYWGQGTLVTVSS | 64 |
| 17-VH | QVQLQESGPGLVKPSETLSLTCTVSGGSLDNYYWSWIRQPPGKGLEWI GYIYYSGNTNYNPSLKSRVTISVDTSKNQFSLRLRSVTAADTAVYYCA RGGRFLERYWGQGTLVTVSS | 65 |
| 3-3-VH | QVQLQESGPGLVKPSETLSLTCTVSGGSLDNYYWSWIRQPPGKGLEWI GYIYYSGNTNYNPSLKSRVTISVDTSKNQFSLRLRSVTAADTAVYYCA RGGRIFGGYWGQGTLVTVSS | 66 |
| 3-6-VH | QVQLQESGPGLVKPSETLSLTCTVSGGSLDNYYWSWIRQPPGKGLEWI GYIYYSGNTNYNPSLKSRVTISVDTSKNQFSLRLRSVTAADTAVYYCA RGGRIFAGYWGQGTLVTVSS | 67 |
| 3-VH | QVQLQESGPGLVKPSETLSLTCTVSGGSLDGYYWSWIRQPPGKGLEWI GRIYGNSTTNYNPSLKSRVTISVDTSKNQFSLRLRSVTAADTAVYYCA RGGRIFAGYWGQGTLVTVSS | 68 |
| 6-VH | QVQLQESGPGLVKPSETLSLTCTVSGGSLNDYYWSWIRQPPGKGLEWI GRIYGNGDTNYNPSLKSRVTISVDTSKNQFSLRLRSVTAADTAVYYCA RGGRILAGYWGQGTLVTVSS | 69 |
| 10-VH | QVQLQESGPGLVKPSETLSLTCTVSGGSLDNYYWSWIRQPPGKGLEWI GRIYYNGNTNYNPSLKSRVTISVDTSKNQFSLRLRSVTAADTAVYYCA RGGRILAGYWGQGTLVTVSS | 70 |
| 16-VH | QVQLQESGPGLVKPSETLSLTCTVSGGSLDNYYWSWIRQPPGKGLEWI GYIYYSGNTNYNPSLKSRVTISVDTSKNQFSLRLRSVTAADTAVYYCA RGGRIFSGYWGQGTLVTVSS | 71 |
| 18-VH | QVQLQESGPGLVKPSETLSLTCTVSGGSLDNYYWSWIRQPPGKGLEWI GYIYYSGNTNYNPSLKSRVTISVDTSKNQFSLRLRSVTAADTAVYYCA RGRGIFGYWGQGTLVTVSS | 72 |
| 24-VH | QVQLQESGPGLVKPSETLSLTCTVSGGSLDNYYWSWIRQPPGKGLEWI GRIYGDSATNYNPSLKSRVTISVDTSKNQFSLRLRSVTAADTAVYYCA RGGRIFSGYWGQGTLVTVSS | 73 |
| 25-VH | QVQLQESGPGLVKPSETLSLTCTVSGGSLDNYYWSWIRQPPGKGLEWI GYIYYSGNTNYNPSLKSRVTISVDTSKNQFSLRLRSVTAADTAVYYCA RGGRIFGHWGQGTLVTVSS | 74 |
| L12-6-1-VL | EIVLTQPPSSLSASVGDRVTIPCRASLSIGSFLNWYQQRPGEAPKLLIFA ASSLRSGVPSRFSGSGSGTDFTLTISGLQPEDFATYYCQQTYTTPYTFG QGTKVDIK | 97 |
| L12-6-VL | EIVLTQSPSSLSASVGDRVTIPCRASLSIGSFLNWYQQRPGEAPKLLIFA ASSLRSGVPSRFSGSGSGTDFTLTISGLQPEDFATYYCQQTYTTPYTFG QGTKVDIK | 102 |
| 17-VL | DIQMTQSPSSVSASVGDRVTISCRANQAIGTWLAWYQQKPGKAPKLLI YAASTLQSGVPSRFSGSGSGTEFTLTISSLQAEDVAVYYCQQYYTTPLT FGGGTKLEIK | 98 |
| X-VL | DIQMTQSPSSVSASVGDRVTISCRANQAIGTWLAWYQQKPGKAPKLLI YAASTLQSGVPSRFSGSGSGTEFTLTISSLQAEDVAVYYCQQYYTTPLT FGGGTKLEIK | 98 |

| | | |
|---|---|---|
| Note: X-VL represents 3-3-VL, 3-6-VL, 3-VL, 6-VL, 10-VL, 16-VL, 18-VL, 24-VL and 25-VI, | | |

**Table 6: VH CDRs of antibodies**

| Name | VH CDR1 | SEQ ID NO: | VH CDR2 | SEQ ID NO: | VH CDR3 | SEQ ID NO: |
|---|---|---|---|---|---|---|
| L12-6-1-VH | DNYYWS | 21 | YIYYSGNTNYNPSLKS | 24 | GGRFLERY | 29 |
| 17-VH | DNYYWS | 21 | YIYYSGNTNYNPSLKS | 24 | GGRFLERY | 29 |
| 3-3-VH | DNYYWS | 21 | YIYYSGNTNYNPSLKS | 24 | GGRIFGGY | 30 |
| 3-6-VH | DNYYWS | 21 | YIYYSGNTNYNPSLKS | 24 | GGRIFAGY | 31 |
| 3-VH | DGYYWS | 22 | RIYGNSTTNYNPSLKS | 25 | GGRIFAGY | 31 |
| 6-VH | NDYYWS | 23 | RIYGNGDTNYNPSLKS | 26 | GGRILAGY | 32 |
| 10-VH | DNYYWS | 21 | RIYYNGNTNYNPSLKS | 27 | GGRILAGY | 32 |
| 16-VH | DNYYWS | 21 | YIYYSGNTNYNPSLKS | 24 | GGRIFSGY | 33 |
| 18-VH | DNYYWS | 21 | YIYYSGNTNYNPSLKS | 24 | GRGIF GY | 34 |
| 24-VH | DNYYWS | 21 | RIYGDSATNYNPSLKS | 28 | GGRIFSGY | 33 |
| 25-VH | DNYYWS | 21 | YIYYSGNTNYNPSLKS | 24 | GGRIFGH | 35 |
| L12-6-VH | DNYYWS | 21 | YIYYSGNTNYNPSLKS | 24 | GGRFLERY | 29 |

The positive control Hu5F9-G4 (magrolimab) for the anti-CD47 antibodies is a human CD47 antibody transiently expressed in HEK293F cells, and its sequence is identical to that of the antibody "Hu5F9" in U.S. Patent No. US2015/0183874A1.

### 2.2.2. Anti-PD-L1 antibodies

CP11-36, CP 11-46, CP 11-47, CP11-55 and CP 11-71 were selected to prepare IgG1 complete antibodies, wherein the VHs and VLs are shown in Table 1, the sequence of CH is set forth in SED ID NO: 81, and the sequence of CL is set forth in SED ID NO: 82. The above 5 anti-PD-L1 antibodies were named: L1-R2-4-36, L1-R2-4-46, L1-R2-4-47, L1-R2-4-55 and L1-R2-4-71.

### 2.2.3. Anti-CD47 antibodies and anti-PD-L1 antibodies having the common light chain

The assembly scheme of the antibodies is shown in Table 7. The assembly scheme of the variable regions is "VH + VL", the sequence of CH is set forth in SED ID NO: 81, and the sequence of CL is set forth in SED ID NO: 82. The light chain variable region sequences are shown in Table 8, and the light chain variable region CDRs are shown in Table 9.

**Table 7: Antibody number and assembly scheme**

| Anti-PD-L1 monoclonal antibody | | | | | Anti-CD47 monoclonal antibody | | | | |
|---|---|---|---|---|---|---|---|---|---|
| Antibody No. | VH | SEQ ID NO: | VL | SEQ ID NO: | Antibody No. | VH | SEQ ID NO: | VL | SEQ ID NO: |
| L1-R2-4 | CP11-27 | 49 | R2-4 | 75 | 47-R2-4 | L12-6-VH | 64 | R2-4 | 75 |
| L1-R2-6 | CP11-27 | 49 | R2-6 | 76 | 47-R2-6 | L12-6-VH | 64 | R2-6 | 76 |
| L1-R2-18 | CP11-27 | 49 | R2-18 | 77 | 47-R2-18 | L12-6-VH | 64 | R2-18 | 77 |
| L1-R2-78 | CP11-27 | 49 | R2-78 | 78 | 47-R2-78 | L12-6-VH | 64 | R2-78 | 78 |
| L1-R2-85 | CP11-27 | 49 | R2-85 | 79 | 47-R2-85 | L12-6-VH | 64 | R2-85 | 79 |
| L1-R2-89 | CP11-27 | 49 | R2-89 | 80 | 47-R2-89 | L12-6-VH | 64 | R2-89 | 80 |

**Table 8. Light chain variable region sequences of antibodies**

| Name | Variable region sequence | SEQ ID NO: |
|---|---|---|
| R2-4 | EIVLTQSPSSLSASVGDRVTITCLASQTIGTWLAWYQQKPGKSPQLLIYAASTL QSGVPSRFSGSGSGTDFTLTISSLQPEDVATYYCQQYYSTPRTFGQGTKVEIK | 75 |
| R2-6 | EIVLTQSPSSVSASVGDKLTMTCQASQDIGKHLNWYQQKPGKPPKLLIYGASN LKTGVPSRFSGTGSGTDFTLTISSLQPEDFATYYCQQSYTTPYTFGQGTRLEIK | 76 |
| R2-18 | EIVLTQSPSSVSASVGDRITITCRASQGIGSWLAWYQQKPGKAPSLLIYAASSLQ SGVPSRFSGSGSGTDFTLTISSLQTEDVAVYYCQQYFSTPYTFGQGTKVDIK | 77 |
| R2-78 | DIQMTQSPSSFSASTGDRVTITCRASQGISSYLAWYQQKPGKAPKLLIYAASRL QSGVPSRFSGSGSGTDFTLTISSLQAEDVAVYYCQQYYSTPYTFGQGTKVDIK | 78 |
| R2-85 | DIQMTQSPSSLSASVGDRVTIPCRASQGIGKFLAWYQQKPGKAPSLLIYAASSL QSGVPSRFSGSGSGTDFTLTISSLQTEDVAVYYCQQYFSTPYTFGQGTKVDIK | 79 |
| R2-89 | DIQMTQSPSSLSASVGDRVTITCLASQTIGTWLAWYQQKPGKSPQLLIYAASSL QSGVPSRFSGSGSGTDFTLTISSLQPEDVATYYCQQYYSTPYTFGQGTKVDIK | 80 |

**Table 9: CDR sequences of light chain variable regions of antibodies**

| Name | LCDR1 | SEQ ID NO: | LCDR2 | SEQ ID NO: | LCDR3 | SEQ ID NO: |
|---|---|---|---|---|---|---|
| R2-4 | LASQTIGTWLA | 36 | AASTLQS | 41 | QQYYSTPRT | 45 |
| R2-6 | QASQDIGKHLN | 37 | GASNLKT | 42 | QQSYTTPYT | 46 |
| R2-18 | RASQGIGSWLA | 38 | AASSLQS | 43 | QQYFSTPYT | 47 |
| R2-78 | RASQGISSYLA | 39 | AASRLQS | 44 | QQYYSTPYT | 48 |
| R2-85 | RASQGIGKFLA | 40 | AASSLQS | 43 | QQYFSTPYT | 47 |
| R2-89 | LASQTIGTWLA | 36 | AASSLQS | 43 | QQYYSTPYT | 48 |

### 2.2.4. Preparation of antibodies

The gene encoding the heavy chain of an antibody and the gene encoding the light chain of the antibody were cloned into expression vectors, respectively, to obtain a heavy chain expression vector and a light chain expression vector. Then the HEK293F cells were transiently transfected with the heavy chain expression vector and the light chain expression vector, and after cell expression, the cell supernatant was purified by using a Protein A column through immobilized metal affinity chromatography (IMAC) to obtain the antibody, and the sequence was confirmed by sequencing.

### 2.3. Anti-PD-L1/CD47 bispecific antibodies and preparation thereof

### 2.3.1. Anti-PD-L1/CD47 bispecific antibodies

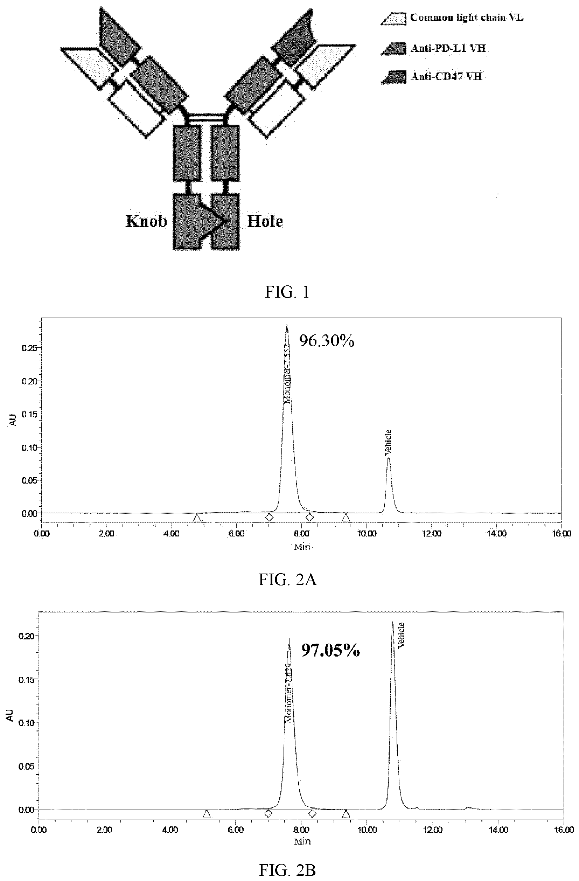

The structural schematic diagram of the anti-PD-L1/CD47 bispecific antibody is shown in FIG. 1, and the amino acid sequences of the variable regions of the 5 antibodies are shown in Table 10. As shown in the structural schematic diagram of FIG. 1, the anti-PD-L1/CD47 bispecific antibody consists of 4 polypeptide chains.

In antibodies BsAb-36, BsAb-46, BsAb-47 and BsAb-71, the C-terminus of the VH of the anti-PD-L1 was linked to the N-terminus of the constant region (SEQ ID NO: 83) derived from human IgG1, and the Fc region of the anti-PD-L1 comprised a "knob-into-hole" mutation for stable association with the anti-CD47 heavy chain; for the light chain, the R2-4 (SEQ ID NO: 75) and the human κ light chain constant region (SEQ ID NO: 82) were linked to form a common light chain; the C-terminus of the VH of the anti-CD47 was linked to the N-terminus of a constant region (SEQ ID NO: 84) derived from human IgG1, and the Fc region of the anti-CD47 comprised a "knob-into-hole" mutation for stable association with the anti-PD-L1 heavy chain.

The antibody BsAb-71-N297A was obtained by the N297A mutation in the Fc region of BsAb-71, for purpose to reduce the ADCC effect of the antibody. Namely, for BsAb-71-N297A, the anti-PD-L1 heavy chain is set forth in SEQ ID NO: 92, the anti-CD47 heavy chain is set forth in SEQ ID NO: 93, and the light chain is set forth in SEQ ID NO: 96.

**Table 10: Antibody number and assembly scheme**

| Antibody No. | Variable region sequence | | | | | |
|---|---|---|---|---|---|---|
| | Anti-PD-L1 heavy chain variable region | SEQ ID NO: | Anti-CD47 heavy chain variable region | SEQ ID NO: | Common light chain variable region | SEQ ID NO: |
| BsAb-36 | CP11-36 | 50 | L12-6-VH | 64 | R2-4 | 75 |
| BsAb-46 | CP11-46 | 51 | | | | |
| BsAb-47 | CP11-47 | 52 | | | | |
| BsAb-71 | CP11-71 | 54 | | | | |
| BsAb-71-N297A | CP11-71 | 54 | | | | |

### 2.3.2. Preparation of anti-PD-L1/CD47 bispecific antibodies

Nucleotide sequences encoding the anti-PD-L1/CD47 bispecific antibodies constructed in step 2.3.1 were each constructed into an expression vector, and the anti-PD-L1/CD47 antibodies were obtained after expression in the HEK293F cells and purification. The specific operation is as follows:
The gene (taking BsAb-71-N297A as an example; the gene sequence is set forth in SEQ ID NO: 99) encoding the anti-PD-L1 heavy chain and the gene (taking BsAb-71-N297A as an example; the gene sequence is set forth in SEQ ID NO: 100) encoding the anti-CD47 heavy chain were each cloned into an expression vector to obtain an anti-PD-L1 heavy chain plasmid and an anti-CD47 heavy chain plasmid; and the gene (taking BsAb-71-N297A as an example; the gene sequence is set forth in SEQ ID NO: 101) encoding the light chain of the anti-PD-L1/CD47 antibody was cloned into an expression vector to obtain a light chain plasmid. HEK293F cells were transiently transfected with the anti-PD-L1 heavy chain plasmid, the anti-CD47 heavy chain plasmid and the light chain plasmid which were correctly constructed in a molar ratio of 1:1:2. After cells expression, the cell supernatant was subjected to affinity chromatography and ion exchange chromatography to obtain the anti-PD-L1/CD47 antibody (e.g., BsAb-71-N297A). The antibody sequence was verified to be correct by sequencing. The purity of the collected samples was detected by size exclusion chromatography (SEC). SEC results for some of the exemplary antibodies are shown in FIGs. 2A and 2B. The purity of the bispecific antibody BsAb-46 is 96.30%, and that of the BsAb-71 is 97.05%.

The anti-PD-L1 heavy chain nucleotide sequence SEQ ID NO: 99 is as follows:

GAGGTGCAGCTGGTGGAATCCGGCGGAGGGCTGGTGCAGCCAGGTGGCTCTCTG
AGACTGAGTTGCGCCGCTTCAGGATTCACCTTTTCCGACTCTTGGATTCACTGGGT
GCGCCAGGCCCCCGGAAAGGGACTGGAGTGGGTCGGTTGGATTTCTCCTTACGGA
GGGAGTACATACTATGCCGACTCTTATAGGTCGCGATTCACTATCTCCGCTGATACT
AGCAAAAACACCGCATATCTGCAGATGAACTCCCTGAGGGCAGAAGACACAGCCG
TCTACTATTGTGCAAGGCGGCATTGGCCCGGTGGCTTTGATTACTGGGGTCAGGGT
ACCCTGGTGACCGTGTCCTCCGCCTCCACCAAGGGCCCCTCCGTGTTCCCTCTGGC
CCCCTCTTCCAAGAGCACCAGCGGCGGCACCGCCGCTCTGGGATGTCTGGTGAAG
GATTACTTTCCCGAGCCTGTGACCGTGAGCTGGAACTCCGGCGCCCTGACCTCCG
GCGTGCACACATTCCCTGCCGTGCTGCAGAGCAGCGGCCTGTACTCCCTGAGCAG
CGTGGTGACCGTGCCTAGCTCCAGCCTGGGCACCCAGACCTACATCTGTAACGTG
AACCACAAGCCTTCCAACACCAAGGTGGACAAGAAGGTGGAGCCTAAGAGCTGT
GACAAGACCCACACCTGCCCTCCTTGTCCTGCCCCTGAGCTGCTGGGCGGCCCAT

CTGTGTTCCTGTTCCCTCCCAAGCCCAAGGACACCCTGATGATCAGCAGAACCCCC
GAGGTGACCTGTGTGGTGGTGGATGTGTCCCACGAGGACCCTGAGGTGAAGTTTA
ACTGGTACGTGGATGGCGTGGAGGTGCACAATGCCAAGACCAAGCCCAGAGAGG
AGCAGTACgccTCCACCTACAGaGTGGTGTCCGTGCTGACCGTGCTGCACCAGGACT
GGCTGAATGGCAAGGAGTACAAGTGTAAGGTGAGCAACAAGGCCCTGCCCGCCC
CTATCGAGAAGACCATCAGCAAGGCCAAGGGCCAGCCTAGGGAGCCTCAGGTGTA
CACCCTGCCCCCCTGCAGAGACGAGCTGACCAAGAATCAGGTGAGCCTGTGGTGC
CTGGTGAAGGGCTTCTACCCCAGCGACATCGCCGTGGAGTGGGAGTCCAATGGCC
AGCCTGAGAACAATTACAAGACCACCCCCCCTGTGCTGGATAGCGATGGCAGCTT
CTTTCTGTACAGCAAGCTGACCGTGGATAAGTCCAGGTGGCAGCAGGGCAATGTG
TTTAGCTGTAGCGTGATGCACGAGGCCCTGCACAATCACTACACCCAGAAGAGCC
TGAGCCTGTCCCCCGGCAAG

The nucleic acid sequence of its Fc is (SEQ ID NO: 87):

GCCTCCACCAAGGGCCCCTCCGTGTTCCCTCTGGCCCCCTCTTCCAAGAGCACCA
GCGGCGGCACCGCCGCTCTGGGATGTCTGGTGAAGGATTACTTTCCCGAGCCTGT
GACCGTGAGCTGGAACTCCGGCGCCCTGACCTCCGGCGTGCACACATTCCCTGCC
GTGCTGCAGAGCAGCGGCCTGTACTCCCTGAGCAGCGTGGTGACCGTGCCTAGCT
CCAGCCTGGGCACCCAGACCTACATCTGTAACGTGAACCACAAGCCTTCCAACAC
CAAGGTGGACAAGAAGGTGGAGCCTAAGAGCTGTGACAAGACCCACACCTGCCC
TCCTTGTCCTGCCCCTGAGCTGCTGGGCGGCCCATCTGTGTTCCTGTTCCCTCCCA
AGCCCAAGGACACCCTGATGATCAGCAGAACCCCCGAGGTGACCTGTGTGGTGGT
GGATGTGTCCCACGAGGACCCTGAGGTGAAGTTTAACTGGTACGTGGATGGCGTG
GAGGTGCACAATGCCAAGACCAAGCCCAGAGAGGAGCAGTACgccTCCACCTACA
GaGTGGTGTCCGTGCTGACCGTGCTGCACCAGGACTGGCTGAATGGCAAGGAGTA
CAAGTGTAAGGTGAGCAACAAGGCCCTGCCCGCCCCTATCGAGAAGACCATCAGC
AAGGCCAAGGGCCAGCCTAGGGAGCCTCAGGTGTACACCCTGCCCCCCTGCAGAG
ACGAGCTGACCAAGAATCAGGTGAGCCTGTGGTGCCTGGTGAAGGGCTTCTACCC
CAGCGACATCGCCGTGGAGTGGGAGTCCAATGGCCAGCCTGAGAACAATTACAAG
ACCACCCCCCCTGTGCTGGATAGCGATGGCAGCTTCTTTCTGTACAGCAAGCTGAC
CGTGGATAAGTCCAGGTGGCAGCAGGGCAATGTGTTTAGCTGTAGCGTGATGCAC
GAGGCCCTGCACAATCACTACACCCAGAAGAGCCTGAGCCTGTCCCCCGGCAAG

The anti-CD47 heavy chain nucleotide sequence SEQ ID NO: 100 is as follows:

CAGGTGCAGCTGCAGGAGTCCGGCCCTGGCCTGGTGAAGCCTTCCGAGACCCTGT
CCCTGACCTGTACCGTGAGCGGCGGCAGCCTGGATAACTATTACTGGAGCTGGATC
CGGCAGCCTCCTGGCAAGGGCCTGGAGTGGATCGGCTACATCTACTATTCCGGCAA

CACCAATTACAACCCTTCCCTGAAGAGCCGGGTGACCATCTCCGTGGACACCAGC
AAGAACCAGTTTAGCCTGAAGCTGTCCTCCGTGACCGCCGCTGATACCGCCGTGTA
CTACTGTGCCAGGGGCGGCCGGTTCCTGGAGAGATATTGGGGCCAGGGTACCCTG
GTGACCGTGAGCAGCGCCAGCACCAAGGGCCCCTCCGTGTTCCCTCTGGCCCCTA
GCAGCAAGTCCACCAGCGGCACCGCCGCTCTGGGATGTCTGGTGAAGGACTA
CTTTCCTGAGCCCGTGACCGTGTCCTGGAACTCCGGCGCCCTGACCTCCGGCGTG
CACACATTCCCCGCCGTGCTGCAGTCCTCCGGCCTGTACTCCCTGTCCAGCGTGGT
GACCGTGCCTAGCTCCAGCCTGGGCACCCAGACCTACATCTGTAACGTGAACCAC
AAGCCCTCCAATACCAAGGTGGATAAGAAGGTGGAGCCCAAGTCCTGCGACAAGA
CCCACACCTGTCCTCCTTGCCCCGCCCCCGAGCTGCTGGGAGGACCTTCTGTGTTC
CTGTTCCCTCCCAAGCCCAAGGACACCCTGATGATCAGCAGAACCCCTGAGGTGA
CCTGTGTGGTGGTGGACGTGAGCCACGAGGACCCTGAGGTGAAGTTCAACTGGTA
CGTGGACGGCGTGGAGGTGCACAATGCCAAGACCAAGCCCAGAGAGGAGCAGTA
CgcCtcCACCTACAGAGTGGTGTCCGTGCTGACCGTGCTGCACCAGGATTGGCTGAA
CGGCAAGGAGTACAAGTGCAAGGTGTCCAATAAGGCCCTGCCCGCCCCCATCGAG
AAGACCATCTCCAAGGCCAAGGGCCAGCCCAGAGAGCCTCAGGTGTGTACCCTGC
CTCCCTCCAGGGATGAGCTGACCAAGAACCAGGTGTCCCTGAGCTGCGCCGTGAA
GGGCTTCTACCCCAGCGATATCGCCGTGGAGTGGGAGTCCAACGGCCAGCCCGAG
AATAATTACAAGACCACCCCCCCCGTGCTGGACAGCGACGGATCTTTCTTTCTGGT
GTCCAAGCTGACCGTGGATAAGTCCAGGTGGCAGCAGGGCAACGTGTTTAGCTGT
AGCGTGATGCACGAGGCCCTGCACAACCACTACACCCAGAAGTCCCTGTCCCTGA
GCCCCGGCAAG

The nucleic acid sequence of its Fc is (SEQ ID NO: 88):

GCCAGCACCAAGGGCCCCTCCGTGTTCCCTCTGGCCCCTAGCAGCAAGTCCACCA
GCGGCGGCACCGCCGCTCTGGGATGTCTGGTGAAGGACTACTTTCCTGAGCCCGT
GACCGTGTCCTGGAACTCCGGCGCCCTGACCTCCGGCGTGCACACATTCCCCGCC
GTGCTGCAGTCCTCCGGCCTGTACTCCCTGTCCAGCGTGGTGACCGTGCCTAGCTC
CAGCCTGGGCACCCAGACCTACATCTGTAACGTGAACCACAAGCCCTCCAATACC
AAGGTGGATAAGAAGGTGGAGCCCAAGTCCTGCGACAAGACCCACACCTGTCCTC
CTTGCCCCGCCCCCGAGCTGCTGGGAGGACCTTCTGTGTTCCTGTTCCCTCCCAAG
CCCAAGGACACCCTGATGATCAGCAGAACCCCTGAGGTGACCTGTGTGGTGGTGG
ACGTGAGCCACGAGGACCCTGAGGTGAAGTTCAACTGGTACGTGGACGGCGTGG
AGGTGCACAATGCCAAGACCAAGCCCAGAGAGGAGCAGTACgcCtcCACCTACAGA
GTGGTGTCCGTGCTGACCGTGCTGCACCAGGATTGGCTGAACGGCAAGGAGTACA
AGTGCAAGGTGTCCAATAAGGCCCTGCCCGCCCCCATCGAGAAGACCATCTCCAA

GGCCAAGGGCCAGCCCAGAGAGCCTCAGGTGTGTACCCTGCCTCCCTCCAGGGAT
GAGCTGACCAAGAACCAGGTGTCCCTGAGCTGCGCCGTGAAGGGCTTCTACCCCA
GCGATATCGCCGTGGAGTGGGAGTCCAACGGCCAGCCCGAGAATAATTACAAGAC
CACCCCCCCCGTGCTGGACAGCGACGGATCTTTCTTTCTGGTGTCCAAGCTGACCG
TGGATAAGTCCAGGTGGCAGCAGGGCAACGTGTTTAGCTGTAGCGTGATGCACGA
GGCCCTGCACAACCACTACACCCAGAAGTCCCTGTCCCTGAGCCCCGGCAAG

The light chain nucleotide sequence SEQ ID NO 101 is as follows:

GAAATTGTGCTGACTCAGTCTCCATCCTCCCTGTCTGCATCTGTAGGAGACAGAGT
CACCATCACTTGCCTGGCAAGTCAGACCATTGGTACATGGTTAGCATGGTATCAGC
AGAAACCAGGGAAATCTCCTCAGCTCCTGATCTATGCTGCATCCACTTTGCAATCA
GGGGTCCCATCTCGGTTCAGTGGCAGTGGATCTGGGACAGATTTCACTCTCACCAT
CAGCAGCCTGCAGCCTGAAGATGTTGCAACTTATTACTGTCAGCAATATTATAGTAC
TCCTCGGACGTTCGGCCAAGGGACCAAGGTGGAGATTAAACGTACGGTGGCTGCA
CCATCTGTCTTCATCTTCCCGCCATCTGATGAGCAGTTGAAATCTGGAACTGCCTCT
GTTGTGTGCCTGCTGAATAACTTCTATCCCAGAGAGGCCAAAGTACAGTGGAAGG
TGGATAACGCCCTCCAATCGGGTAACTCCCAGGAGAGTGTCACAGAGCAGGACAG
CAAGGACAGCACCTACAGCCTCAGCAGCACCCTGACGCTGAGCAAAGCAGACTA
CGAGAAACACAAAGTCTACGCCTGCGAAGTCACCCATCAGGGCCTGAGCTCGCCC
GTCACAAAGAGCTTCAACAGGGGAGAGTGT

The nucleic acid sequence of its CL is (SEQ ID NO: 89):

CGTACGGTGGCTGCACCATCTGTCTTCATCTTCCCGCCATCTGATGAGCAGTTGAA
ATCTGGAACTGCCTCTGTTGTGTGCCTGCTGAATAACTTCTATCCCAGAGAGGCCA
AAGTACAGTGGAAGGTGGATAACGCCCTCCAATCGGGTAACTCCCAGGAGAGTGT
CACAGAGCAGGACAGCAAGGACAGCACCTACAGCCTCAGCAGCACCCTGACGCT
GAGCAAAGCAGACTACGAGAAACACAAAGTCTACGCCTGCGAAGTCACCCATCA
GGGCCTGAGCTCGCCCGTCACAAAGAGCTTCAACAGGGGAGAGTGT

### Example 3: Binding of Anti-PD-L1 scFv to Antigen Protein PD-L1-His-Biotin

The binding of the anti-PD-L1 scFv displayed by the yeast to the antigen protein PD-L1-His-Biotin was detected by flow cytometry. The positive yeast clones in the step 2.1 of Example 2 were each incubated with 1 nM of PD-L1-His-Biotin antigen protein at room temperature for 1 h, washed 3 times with PBS at pH 7.4, incubated with Streptavidin-PE fluorescent secondary antibody at room temperature for 30 min, and then washed 3 times with PBS at pH 7.4. The fresh PBS was then added, and the samples was loaded on the machine for assay.

Preparation of PD-L1-His-Biotin: the PD-L1-His antigen protein obtained by purification and 10 nM biotin (Sigma-Aldrich, B4501-1G) in DMSO were incubated at room temperature for 2 h which 1 mg protein with 26.6 µL 10 nM biotin, and then dialysis was performed using PBS at pH 7.4.

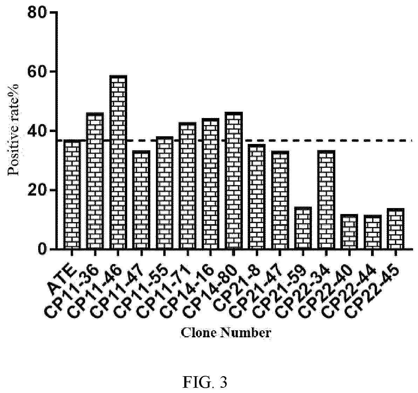

As shown in FIG. 3, the anti-PD-L1 scFvs of the present invention (CP 11-3 6, CP 11-46, CP11-71, CP14-16 and CP14-80) have significantly better binding activity to PD-L1-His-Biotin antigen protein than that of the positive control ATE scFv; compared with the positive control ATE scFv, the anti-PD-L1 scFvs of the present invention (CP-11-47, CP-11-55, CP21-8, CP21-47 and CP22-34) have a similar binding activity to PD-L1-His-Biotin antigen protein.

### Example 4: Activity of Anti-CD47 Antibodies in Blocking SIRPα

A competitive ELISA was carried out. 11 antibodies (antibody L12-6, antibody 17, antibody 3, antibody 3-3, antibody 3-6, antibody 6, antibody 10, antibody 16, antibody 18, antibody 24 and antibody 25) and the positive control (antibody Hu5F9-G4) were each diluted to different concentrations (12, 6, 3, 2, 1.5, 1.2, 0.75, 0.375, 0.188 and 0.094 µg/mL) and incubated with the CD47-His antigen (2 µg/mL) coated on ELISA plates at room temperature for 1 h. The plates were washed four times with PBST, then the ligand SIRPα-Fc-Bio (0.1 µg/mL) (prepared according to Thermo Scientific EZ-Link^{®} NHS-Biotin Reagents kit) was added and incubated with the CD47-His antigen at room temperature for 1 h, and then the plates were washed four times with PBST. The bound SIRPα-Fc-Bio and HRP-conjugated streptavidin resulted in chemiluminescence reactions, and the OD450 values were measured using a plate reader. The IC₅₀ values of the antibodies were calculated from the OD450 values, thereby determining the activity of the antibodies in blocking SIRPα. The results are shown in Table 11. The results show that the anti-CD47 antibodies described above can inhibit SIRPα binding to CD47.

**Table 11: IC₅₀ values of anti-CD47 antibodies**

| Antibody | IC₅₀ (µg/mL) | | Antibody | IC₅₀ (µg/mL) |
|---|---|---|---|---|
| Antibody L12-6 | 0.913 | | Antibody 10 | 1.056 |
| Antibody 17 | 0.795 | | Antibody 16 | 0.975 |
| Antibody 3 | 0.844 | | Antibody 18 | 1.232 |
| Antibody 3-3 | 0.939 | | Antibody 24 | 1.412 |
| Antibody 3-6 | 1.122 | | Antibody 25 | 1.31 |
| Antibody 6 | 1.102 | | Positive control | 1.095 |

### Example 5: Affinity of Anti-PD-L1 Antibody and PD-1/PD-L1 Blockade Activity

### The dissociation constant (KD) measured by Biacore

The equilibrium dissociation constant (KD) of the binding of the antibodies in the step 2.2.2 of Example 2 in the present invention to the corresponding antigens was determined by a kinetic binding assay using a Biacore system (GE). According to the method in the manual, 5µg of the antigen protein PD-L1-His was immobilized on the chip, the affinity assay was performed with the anti-PD-L1 antibody at the highest concentration of 100 nM (diluted in a ratio of 1:2, 5 gradients) as the mobile phase, and the results were analyzed by using Biacore T200 Evaluation Software. The ATE antibody was used as a positive control (the amino acid sequence of the ATE antibody is identical to that of atezolizumab, which is expressed by HEK293F cells). The KD data of exemplary antibodies are shown in Table 12, and indicate that the antibodies L1-R2-4-36, L1-R2-4-46, L1-R2-4-47, L1-R2-4-55 and L1-R2-4-71 of the present invention exhibit a higher affinity to PD-L1 antigen than the ATE antibody.

**Table 12: Dissociation constants (KD*) of exemplary antibodies determined by Biacore kinetic binding assay**

| Antibody No. | KD (M) |
|---|---|
| L1-R2-4-36 | 3.41×10⁻¹⁰ |
| L1-R2-4-46 | 2.82×10⁻¹⁰ |
| L1-R2-4-47 | 2.90×10⁻¹⁰ |
| L1-R2-4-55 | 3.71×10⁻¹⁰ |
| L1-R2-4-71 | 2.82×10⁻¹⁰ |
| ATE | 1.84×10⁻⁹ |

| | |
|---|---|
| Note: *the fitting method is 1: 1 Binding. | |

The method for detecting the biological activity of the anti-PD-L1 antibody was used to detect the inhibitory effect of the anti-PD-L1 antibody on the binding of PD-1/PD-L1.

### Detecting blocking activity of anti-PD-L1 antibodies on PD-1/PD-L1 by MOA-based method

Anti-PD-1/PD-L1 antibodies can relieve the inhibitory effect on a downstream NFAT signaling pathway by blocking the binding of PD-1 to PD-L1. The mechanisms of action (MOA) detection system (PD-1/PD-L1 Blockade Bioassay, Propagation Model, Catalog J1252) from Promega, Inc. was used. According to the method provided in the product manual, the activation of NFAT signal was reflected by detecting the expression of luciferase reporter genes, thereby detecting the inhibitory effects of the antibodies in the step 2.2.2 of Example 2 on the binding of PD-1/PD-L1. The negative control IgG-Isotype was purchased from Sino Biological Inc. (Cat. No.: HG1K).

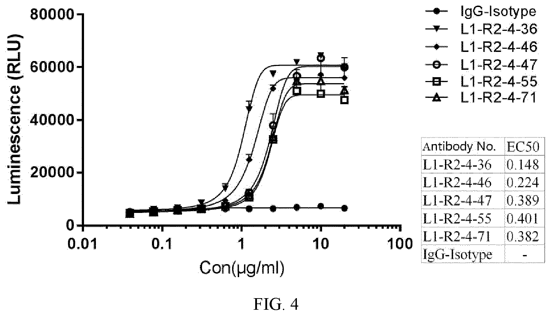

CHO-PD-L1 cells (from the MOA detection system described above) were plated the day before the activity assay: cell passage was performed 1-2 days before plating CHO-PD-L1 cells, the culture supernatant was discarded, and the cells were washed once with sterile PBS. An appropriate amount of Trypsin (Gibco) was added for digesting in an incubator at 37 °C with 5% CO₂ for 3-5 min. Fresh medium was added to terminate the digestion, and the cells were transferred to a 50 mL centrifuge tube and counted. A desired volume of cells was taken and centrifuged at 900 rpm for 5 min. The cells were resuspended to 4×10⁵ cells/mL with DMEM-F12 medium (Gibco). The cells were added to a 96-well white cell culture plate (Corning) at 100 µL/well. The cells were incubated in an incubator at 37 °C with 5% CO₂ overnight. Jurkat-PD1 cells (from the MOA detection system described above) were prepared on the day of detection: after counting, a desired volume of cells was taken and centrifuged at 900 rpm for 5 min. The cells were resuspended to 1.25×10⁶ cells/mL with assay buffer (1640 medium (Gibco) + 1% FBS) for next step. 95 µL/well of the CHO-PD-L1 cell culture supernatant was discarded, 40 µL/well of the test samples (the anti-PD-L1 antibodies, the ATE antibody and the negative control HG1K antibody described above with the highest concentration of 20 µg/mL, 2-fold diluted in assay buffer for 9 gradients) and followed 40 µL/well of Jurkat-PD1 cells were added. The mixture was well mixed by gentle shaking and incubated in an incubator at 37 °C with 5% CO₂ for 6 h. Detection: the ONE-Glo^{™} Luciferase Assay System (purchased from Promega, E6120) was thawed and prepared before use. After 6 h, the ONE-Glo^{™} reagent was added at 50 µL/well. The mixture was left to stand at room temperature for 5-10 min, and then the plate was read. The experimental results are shown in FIG. 4. The antibodies of the present invention, L1-R2-4-36, L1-R2-4-46, L1-R2-4-47, L1-R2-4-55 and L1-R2-4-71, all can effectively block the PD-1/PD-L1 interaction; compared with the control antibody ATE, the antibodies (L1-R2-4-36 and L1-R2-4-46) showed a stronger blocking activity.

### Example 6: Affinity and Activity of Antibodies with Common Light Chain

### The dissociation constant (KD) measured by Biacore

The dissociation constants(KD) of the anti-PD-L1 antibodies and anti-CD47 antibodies in the step 2.2.3 of Example 2 were determined by the Biacore method which was used in Example 5. The results are shown in Table 13.

**Table 13: Dissociation constants (KD*) of exemplary antibodies determined by Biacore kinetic binding assay**

| Anti-PD-L1 monoclonal antibodies | | Anti-CD47 monoclonal antibodies | |
|---|---|---|---|
| Antibody No. | KD (M) | Antibody No. | KD (M) |
| L1-R2-4 | 1.67×10⁻⁹ | 47-R2-4 | 3.29×10⁻⁹ |
| L1-R2-6 | 1.03×10⁻⁸ | 47-R2-6 | 1.11×10⁻⁹ |
| L1-R2-18 | 3.92×10⁻⁹ | 47-R2-18 | 3.12×10⁻¹⁰ |
| L1-R2-78 | 8.99×10⁻⁹ | 47-R2-78 | 6.55×10⁻⁷ |
| L1-R2-85 | 1.15×10⁻⁸ | 47-R2-85 | 2.75×10⁻⁷ |
| L1-R2-89 | 4.08×10⁻⁹ | 47-R2-89 | 3.61×10⁻¹⁰ |

| | | | |
|---|---|---|---|
| Note: *the fitting method is 1:1 Binding. | | | |

From the above data, the different light chains of the present invention can each form monoclonal antibodies together withanti-PD-L1 and anti-CD47 heavy chains, which can bind to human PD-L1 or human CD47 proteins in solution, respectively.

### Detecting blocking activity of anti-PD-L1 antibodies on PD-1/PD-L1 by MOA-based method

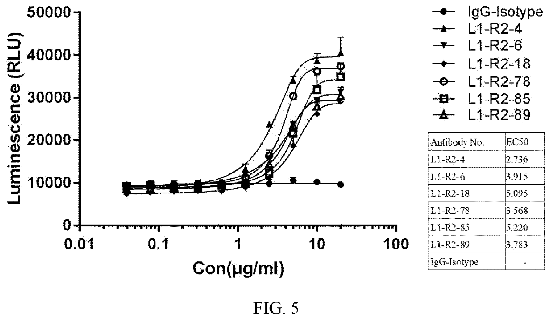

The PD-1/PD-L1 blocking activity of the anti-PD-L1 antibodies in the step 2.2.3 of Example 2 was detected using the MOA-based method in Example 5. The experimental results are shown in FIG. 5, the anti-PD-L1 antibodies with different light chains of the present invention can effectively block the PD1/PD-L1 interaction.

### Red blood cell agglutination experiment for anti-CD47 antibodies

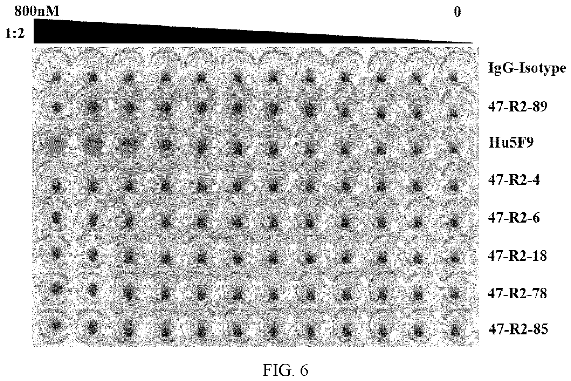

It has been known that most of the anti-CD47 antibodies have the side effect of promoting agglutination of red blood cells, so the therapeutic use of these anti-CD47 antibodies is limited. Therefore, the effect on agglutination of red blood cells of the anti-CD47 antibodies of the present invention in the step 2.2.3 of Example 2 was further detected. The specific detection method is as follows: fresh human blood was collected and washed with normal saline three times to prepare 2% human red blood cell suspension; the 2% human red blood cell suspension was mixed with a test antibody (the highest final concentration being 800 nM, two-fold serial dilution to obtain 11 diluted concentrations in total) in equal volume, and the mixture was incubated at 37 °C for 4 h; and then the condition of the agglutination of red blood cells caused by the antibody was evaluated by tilting the 96-well U-bottom plate at 45° and observing the flow direction of the red blood cell cluster, and if the red blood cell cluster flowed in a straight line, it indicated that the red blood cells did not agglutinate. In the experiment performed as described in the above assay, the results of the red blood cell agglutination reaction are shown in FIG. 6. At high concentrations, the 47-R2-89 and the control antibody Hu5F9-G4 caused agglutination of red blood cells, but the other exemplary antibodies did not. It can be seen that most of the anti-CD47 antibodies of the present invention significantly reduce agglutination of red blood cells, and thus have significantly reduced side effects in clinical treatment and can be widely used in the treatment of various cancers.

### Phagocytosis promotion experiment of anti-CD47 antibodies

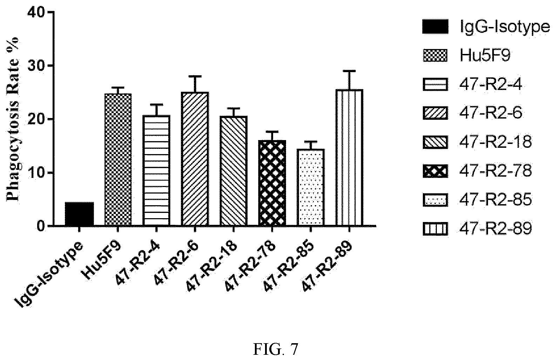

An *in vitro* phagocytosis assay was carried out to assess whether the anti-CD47 antibodies in the step 2.2.3 of Example 2 can promote phagocytosis of CD47-expressing target cells by macrophages. Briefly, in the presence of anti-CD47 antibodies (0.7 nM), RAW264.7 macrophages (2×10⁵ cells/mL) and CFSE (Invitrogen, Cat. No.: C34554)-labeled Raji cells (4×10⁵ cells/mL) were plated to a 24-well plate in a ratio of 1:2 and incubated in the dark at 37 °C for 2 h. After incubation, the cells were washed twice with PBS, and then 100 µL of APC-F4/80 fluorescent antibody (eBiosciences) was added. The mixture was incubated on ice (in the dark) for 30 min, washed twice with PBS buffer, and analyzed by flow cytometry. The phagocytosis index was analyzed. The phagocytosis index was calculated as: phagocytosis index = number of CFSE-F4/80 double positive macrophages (i.e., number of macrophages that phagocytosed tumor cells)/5000 macrophages. As can be seen from FIG. 7, the anti-CD47 antibodies of the present invention and Hu5F9-G4 all can effectively induce phagocytosis of target cells by macrophages.

### Example 7: Determination of the Melting Temperature (Tm) of Anti-PD-L1/CD47 Bispecific Antibodies

The Tm values of the exemplary bispecific antibodies and the reference antibody (L1-R2-4-71 was used herein as a reference) were determined by DSC (differential scanning calorimetry, Malvern Panalytical, MicroCal VP-Capillary), and the thermal stability of the exemplary bispecific antibodies was preliminarily judged based thereon.

The sample protein was dissolved in 1× PBS buffer (pH 7.4) to prepare a solution at a concentration of 2 mg/mL. The specific heat capacity (Cp) of the sample or blank buffer was scanned starting at 40 °C at a rate of 180 °C/h. The result of the corresponding buffer was subtracted from the results of sample scanning, and the obtained Cp values were plotted relative to the temperature, wherein the temperature corresponding to the peak value featuring significantly increased Cp value was the Tm value of the sample.

As can be seen from Table 14, similar to the monoclonal antibodies, the exemplary bispecific antibodies and the L1-R2-4-71 reference sample all show significant Tm values, including a CH2 dissolution temperature of around 70 °C and a CH3 dissolution temperature of around 85 °C. Meanwhile, it can be seen that compared with the L1-R2-4-71, the Tm values of the bispecific antibodies are slightly lower, but the difference is not significant. Therefore, it can be preliminarily determined that the bispecific antibodies are close to the monoclonal antibody and have good thermal stability.

**Table 14: Tm values of anti-PD-L1/CD47 bispecific antibodies**

| Heating rate was 180 °C/h | Tm1 (°C) | Tm2 (°C) | Tm3 (°C) |
|---|---|---|---|
| | CH2 | Fab | CH3 |
| L1-R2-4-71 | 72.41 | 82.51 | 87.23 |
| BsAb-36 | 70.64 | 80.56 | 84.35 |
| BsAb-46 | 71.68 | 81.27 | 85.92 |
| BsAb-47 | 71.66 | 81.61 | 86.13 |
| BsAb-71 | 72.41 | 80.92 | 85.85 |

### Example 8: Binding Activity of Anti-PD-L1/CD47 Antibodies to Two Antigens

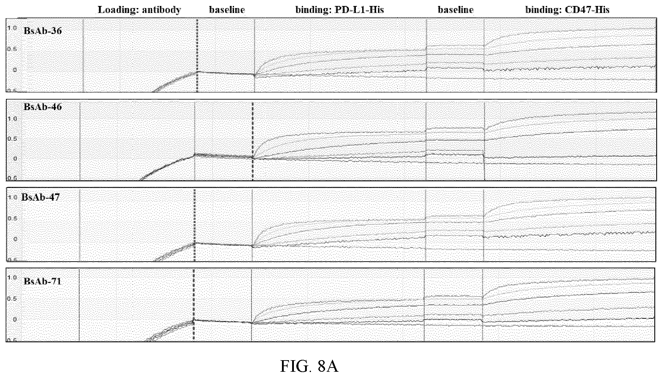

The binding activity of the exemplary bispecific antibodies for PD-L1 and CD47 was determined by a kinetic binding assay using the Octet system (ForteBio). At the beginning of the experiment, the ProA biosensor ARC sensor (Pall, 1506091) was immersed in PBS buffer and equilibrated at room temperature for 10 min. 100 µL/well of the bispecific antibody solution (BsAb-36, BsAb-46, BsAb-47 or BsAb-71 (20 µg/mL)), 100 µL/well of the PD-L1-His antigen solution (200 nM, 40 nM, 8 nM, 1.6 nM, 0.32 nM and 0) and 100 µL/well of the CD47-His antigen solution (200 nM, 40 nM, 8 nM, 1.6 nM, 0.32 nM and 0) were added to a 96-well black polystyrene half-area microplate (Greiner), respectively. The ProA biosensor ARC was immersed in the well containing the antibody solution for 120 s at room temperature prior to loading. The sensor was then washed in PBS buffer until it returned to the baseline and then immersed in the well containing 100 µL of PD-L1-His antigen solution. The sensor was then washed in PBS buffer until it returned to the baseline and then immersed in the well containing 100 µL of CD47-His antigen solution. The association of antibody with antigen was monitored. The rotation speed was 1000 rpm and the temperature was 30 °C. The results are shown in FIG. 8A.

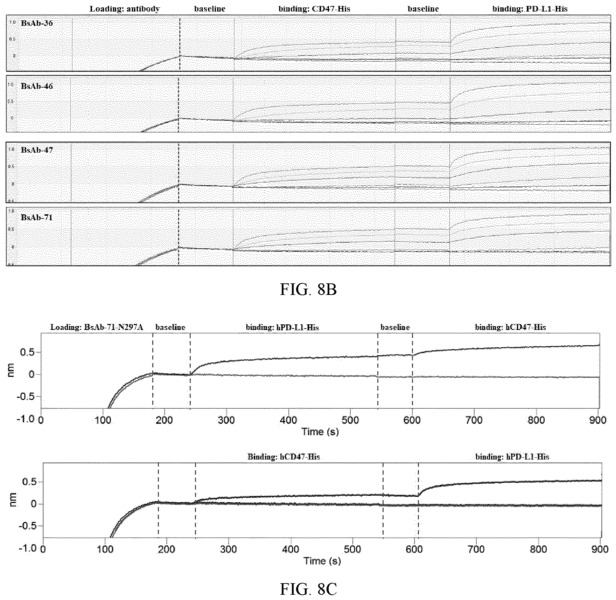

In another experiment, the procedure was as described above, but the order of binding of the antibody to the two antigens was reversed from the above experiment. The ProA biosensor ARC was immersed in the well containing the antibody solution for 120 s at room temperature prior to loading. The sensor was then washed in PBS buffer until it returned to the baseline and then immersed in the well containing 100 µL of CD47-His antigen solution. The sensor was then washed in PBS buffer until it returned to the baseline and then immersed in the well containing 100 µL of PD-L1-His antigen solution. The association of antibody with antigen was monitored. The results are shown in FIG. 8B.

In another experiment, the procedure was as described above, but the bispecific antibody was BsAb-71-N297A. The binding activity of the bispecific antibody BsAb-71-N297A for the two antigens was detected. The results are shown in FIG. 8C and indicate that BsAb-71-N297A was capable of simultaneously binding to PD-L1 and CD47 proteins in solution.

As can be seen from the above results, the exemplary bispecific antibodies of the present invention are capable of simultaneously binding to PD-L1 and CD47 proteins in solution and do not interfere with each other when binding to different epitopes.

### Example 9: Determination of Dissociation Constants of Anti-PD-L1/CD47 Bispecific Antibodies

The equilibrium dissociation constants (KD) of the anti-PD-L1/CD47 bispecific antibodies constructed as described above to human PD-L1-His or human CD47-His antigen was determined using the Biacore method in Example 5, and the results were analyzed using different fitting methods. Table 15 shows the KD data of the exemplary antibodies.

**Table 15: Dissociation constants (KD) of exemplary antibodies to human PD-L1-His or human CD47-His antigen as determined by Biacore kinetic binding assay**

| Antibody No. | Human PD-L1-His | Human CD47-His | |
|---|---|---|---|
| | KD (M)* | KD (M)^ | KD (M)* |
| BsAb-36 | 3.52×10⁻¹⁰ | 4.49×10⁻⁹ | 2.80×10⁻⁸ |
| BsAb-46 | 3.71×10⁻¹⁰ | 3.95×10⁻⁹ | 2.91×10⁻⁸ |
| BsAb-47 | 2.23×10⁻¹⁰ | 4.77×10⁻⁹ | 2.84×10⁻⁸ |
| BsAb-71 | 1.74×10⁻¹⁰ | 3.78×10⁻⁹ | 2.78×10⁻⁸ |

| | | | |
|---|---|---|---|
| Note: * the fitting method is 1:1 Binding; ^ the fitting method is Two State Reaction. | | | |

As can be seen from the above data, by using the 1: 1 Binding fitting method, the exemplary bispecific antibodies of the present invention are capable of binding to human PD-L1-His in solution and maintain the affinity constant of the parent antibody; the exemplary bispecific antibodies of the present invention are capable of binding to human CD47-His protein in solution, have weaker affinity constant than the parental antibody, and have moderate affinity to CD47.

### Example 10: Analysis of Binding of Anti-PD-L1/CD47 Bispecific Antibodies to CHO Cells Overexpressing PD-L1 or CD47

The binding of the exemplary anti-PD-L1/CD47 bispecific antibodies of the present invention to CHO cells overexpressing human PD-L1 or human CD47 was measured by flow cytometry. Briefly, human PD-L1 or human CD47 cDNA was inserted into an expression vector and transfected into Chinese hamster ovary cancer cells (CHO, Invitrogen), resulting in CHO cells overexpressing human PD-L1 or human CD47 (PD-L1-CHO cells or CD47-CHO cells). The PD-L1-CHO cells or CD47-CHO cells were mixed with the exemplary bispecific antibodies subjected to different serial dilutions, and the mixture was incubated on ice for 1 h; the cells were washed twice with PBS, and then a PE-labeled anti-human Fc antibody (Invitrogen, 12-4998-82) fluorescent secondary antibody was added, followed by incubation on ice for 30 min in the dark; the cells were washed twice with PBS and then resuspended with PBS, and the binding of the antibody to the cells was detected by FACS. The results are shown in FIGs. 9A and 9B. As can be seen from FIG. 9A, the exemplary bispecific antibodies of the present invention were all capable of binding to PD-L1 expressed on the cell surface and maintain the binding EC₅₀ of the parental antibody. Meanwhile, as can be seen from FIG. 9B, all of the exemplary bispecific antibodies of the present invention are capable of binding to CD47 expressed on the cell surface.

In another example, the binding of the anti-PD-L1/CD47 bispecific antibody BsAb-71-N297A to CHO cells overexpressing PD-L1 or CD47 was analyzed according to the method described above. The results are shown in FIGs. 9C and 9D. As can be seen from FIG. 9C, BsAb-71-N297A is capable of binding to PD-L1 expressed on the cell surface and maintains the binding EC₅₀ of the parent antibody. Meanwhile, as can be seen from FIG. 9D, BsAb-71-N297A is capable of binding to CD47 expressed on the cell surface.

The prior reports demonstrated that CD47 protein is expressed in various normal tissues of human beings in different level, thus bringing certain hindrance to the therapeutic application of anti-CD47 antibodies. In the present invention, the affinity and binding activity of the bispecific antibodies to human CD47 are strategically reduced, and it is expected that the side effects of the antibodies can be reduced in clinical treatment, and the antibodies can be widely used in the treatment of various cancers.

### Example 11: MOA-Based Detection of Anti-PD-L1 Activity of Exemplary Bispecific Antibodies of the Present Invention

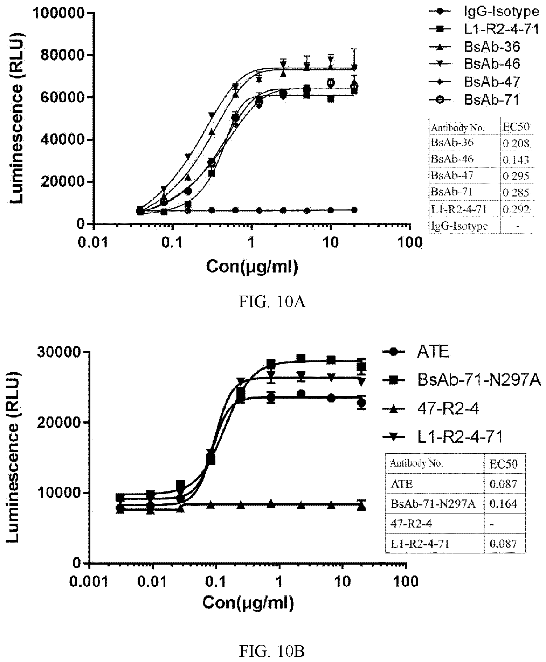

The anti-PD-L1 activity of the exemplary bispecific antibodies of the present invention (BsAb-36, BsAb-46, BsAb-47, BsAb-71 and BsAb-71-N297A) was detected using the MOA-based detection method in Example 5. The results are shown in FIGs. 10A and 10B. The bispecific antibodies of the present invention can remove the inhibitory effect of the PD-1/PD-L1 interaction on NFAT signaling pathway, and the activity is better than that of L1-R2-4-71 (an anti-PD-L1 antibody) used alone.

### Example 12: Detection of Activity of Anti-PD-L1/CD47 Bispecific Antibodies in Promoting Agglutination of Red Blood Cells

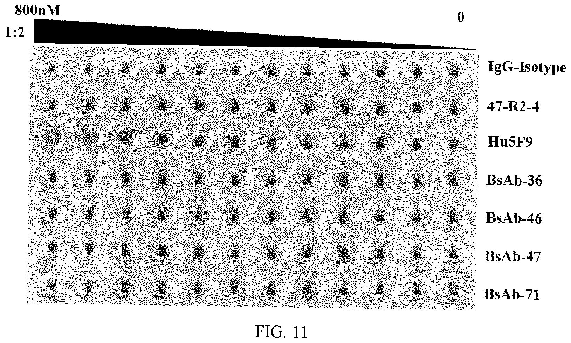

The present invention further investigated the effect on agglutination of red blood cells of the anti-PD-L1/CD47 bispecific antibodies using the detection method in red blood cell agglutination experiment described in Example 6. The results are shown in FIG. 11. The exemplary bispecific antibodies of the present invention do not cause agglutination of red blood cells, and their activity in promoting agglutination of red blood cells is significantly lower than that of the control group Hu5F9-G4, and is consistent with the parental antibody 47-R2-4.

### Example 13: Detection of Ability of Bispecific Antibodies to Promote Phagocytosis of Tumor Cells by Macrophages (phagocytosis graph)

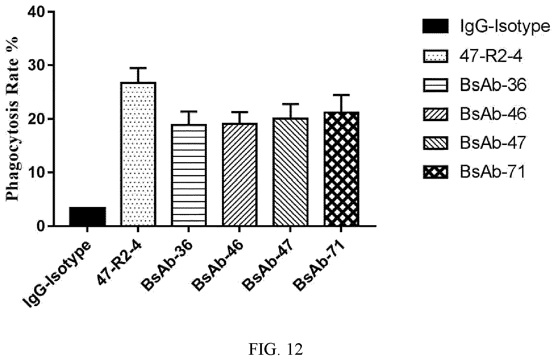

The ability of the exemplary bispecific antibodies of the present invention to promote phagocytosis of tumor cells by macrophages was measured by using a flow cytometry-based assay. The ability of the exemplary bispecific antibodies of the present invention to promote phagocytosis of target cells by macrophages was evaluated *in vitro* using the method for phagocytosis promotion experiment of anti-CD47 antibodies described in Example 6. As can be seen from FIG. 12, the anti-PD-L1/CD47 bispecific antibodies can effectively induce phagocytosis of target cells by macrophages, and the inducing activity is similar to that of the anti-CD47 monoclonal antibodies.

### Example 14: Analysis of Binding of Bispecific Antibodies to Human Red Blood Cells (RBC Binding)

It is known in the prior art that blocking the CD47-SIRPα interaction triggers phagocytosis of tumor cells and red blood cells. This mechanism of action of the anti-CD47 antibodies imply that these antibodies will induce treatment-relative erythropenia and cause severe anemia, which limiting the therapeutic application of the anti-CD47 antibodies. Differential binding activity to the red blood cells of the anti-CD47 antibody which avoid binding to normal red blood cells can reduce the side effect in clinical treatment. In this example, flow cytometry was used to detect the selective binding property of exemplary bispecific antibodies for tumor cells and human red blood cells. The specific method is as follows: fresh human blood was collected and washed with normal saline three times to prepare 2% human red blood cell suspension; the 2% human red blood cell suspension was mixed with a test antibody (the highest final concentration being 200 nM, two-fold serial dilution to obtain 11 diluted concentrations in total) in equal volume, and the mixture was incubated on ice for 1 h; the cells were washed twice with PBS, and then a PE-labeled anti-human Fc antibody (Jackson Invitrogen, 12-4998-82) fluorescent secondary antibody was added, followed by incubation on ice for 30 min in the dark; the cells were washed twice with PBS and then resuspended with PBS, and the binding of the antibody to the cells was detected by FACS. By using the same method as described above, test samples of the exemplary bispecific antibodies and human T-lymphocyte leukemia Jurkat cells were prepared. The results are shown in FIGs. 13A and 13B. At an antibody concentration of 200 nM, the bispecific antibodies of the present invention only weakly bind to human red blood cells, and the binding is significantly weaker than that of the parental antibody 47-R2-4 and the positive control antibody Hu5F9-G4 bound to human red blood cells; the differential red blood cell binding characteristic does not affect the binding of the bispecific antibodies and the parental antibody to the tumor cells, and thus the bispecific antibodies of the present invention have a higher clinical application safety and wider therapeutic adaptability.

### Example 15: Effect of Anti-PD-L1/CD47 Bispecific Antibodies on IL2 Cytokine Release from Human Peripheral Blood Mononuclear Cells (PBMCs) under Exogenous Stimulation

In this study, the exemplary antibodies incubated with PBMC cells cultured *in vitro* and derived from different donors. The expression levels of IL2 in the system were detected, so that the activating effect of different antibodies on T cells was reflected.

Isolation of PBMCs: fresh anticoagulated whole blood from donors (Donor1 and Donor2) was taken and diluted with an equal volume of PBS at a ratio of 1: 1; 7 mL of human peripheral blood lymphocyte isolation solution (Dayou, 7912011) was added into a 15 mL centrifuge tube, 7 mL of diluted blood sample was gently added above the liquid level of the human peripheral blood lymphocyte isolation solution (Dayou, 7912011), and the interface between the two liquid levels was kept clear, and the blood sample should float above the isolation solution and could not break the interface; centrifugation was performed with a swing-out rotor at 800 g for 30 min at room temperature, the accelerated speed for acceleration was set as 1 and the accelerated speed for deceleration was set as 0; after centrifugation, the red blood cells were at the bottom of the tube, the isolation solution was in the middle, the plasma/tissue homogenate layer is in the top, and a thin and compact white membrane, namely a PBMC cell layer, which between the plasma layer and the isolation solution layer. The white membrane layer was carefully pipetted into a new centrifuge tube; the pipetted PBMCs were diluted to a certain volume with PBS and then mixed well by inverting the tube. The mixture was centrifuged at room temperature with a swing-out rotor at 250 g for 10 min, the supernatant was discarded, and the cells were washed twice; the cells were resuspended in PBS and counted for later use. Antibody preparation: antibodies BsAb-71-N297A (5 µg/mL), 47-R2-4 (5 µg/mL), L1-R2-4-71 (5 µg/mL), 47-R2-4 (5 µg/mL) + L1-R2-4-71 (5 µg/mL) were prepared with PBMC culture solution.

Stimulation of PBMCs: a proper amount of PBMCs were taken and resuspended to a cell density of 1×10⁶ cells/mL with the PBMC culture solution; SEE (Toxin Technology, ET404) was added to the cell suspension above to a concentration 100 ng/mL; then SEE-added PBMC cells were added to a 96-well cell culture plate at 100 µL/well. 100 µL/well of the prepared antibody solution was added to the plate and mixed well. Finally, the concentration of SEE was 50 ng/mL, the concentration of antibody was 2.5 µg/mL and each well contained 1×10⁵ PBMC cells. The antibody was co-incubated with the cells in a carbon dioxide incubator at 37 °C for 4 days.

Detection of IL-2: a 96-well plate (Costar^{®} Assay Plate) was coated with the prepared anti-IL-2 antibody by adding the anti-IL-2 antibody to the plate at 100 µL/well, and incubated overnight at 4 °C; the plate was washed twice at 300 µL/well washing buffer by a microplate washer. The plate was gently patted dry on clean paper, the blocking solution was added at 200 µL/well, and the mixture was incubated in an electric thermostatic incubator at 37 °C for 2 h; 75 µL of experimental diluent and 25 µL of PBMC supernatant incubated with antibody for 4 days were added into each well, then the diluted IL-2 standard (Human IL-2 ELISA development kit (HRP), Mabtech) was added, and the plate was incubated at 37 °C for 2 h; the plate was washed 5 times at 300 µL/well washing buffer by a microplate washer. The plate was gently patted dry on clean paper, the anti-IL-2 biotinylated antibody was added at 100 µL/well, and the mixture was incubated in an electric thermostatic incubator at 37 °C for 1 h; the secondary antibody solution was added at 100 µL/well, and the mixture was incubated in an electric thermostatic incubator at 37 °C for 1 h; the plate was washed 8 times at 300 µL/well washing buffer by a microplate washer. The plate was gently patted dry on clean paper; the TMB single-component substrate solution was added at 100 µL/well, and the mixture was incubated at 37 °C for 10-15 min in the dark; the stop solution was added at 50 µL/well to stop the chromogenic reaction, the data were read at 450nm and analyzed by a multifunctional microplate reader.

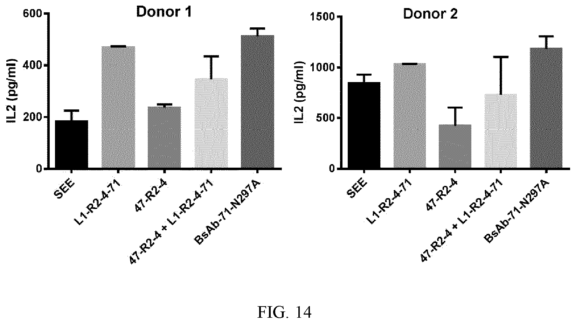

The experimental results are shown in FIG. 14. The exemplary anti-PD-L1/CD47 bispecific antibody BsAb-71-N297A of the present invention can effectively activate T cells *in vitro.*

### Example 16: In Vivo Anti-Tumor Effect of Anti-PD-L1/CD47 Bispecific Antibodies of the Present Invention

The anti-tumor effect of the anti-PD-L1/CD47 bispecific antibodies of the present invention was determined in hSIRPα transgenic C57BL/6-hSIRPα mice using MC38 cells expressing human CD47 (MC38-hCD47 (Tg), GemPharmatech Co., Ltd.).

### Human SIRPα transgenic mice: female C57BL/6-hSIRPα mice (5-8 weeks old) (GemPharmatech Co., Ltd.).

Cells for experiment: mouse colon cancer cells MC38-hCD47 (Tg) in logarithmic growth phase were collected, the culture medium was removed, and then the cells were washed twice with PBS and inoculated into the right abdominal region in an amount of 1×10⁶/100 µL/mouse.

Grouping and administration: when the mean tumor volume was 50-100 mm³, the tumor-bearing mice were randomized into groups; inclusion criteria: the tumor volume CV value was less than 30%; the day of inoculation was defined as D0, and on the day of grouping, administration was started according to the experimental protocol design. The administration dose and administration mode are shown in Table 16. After cell inoculation, the effect of tumors on the normal behavior of the animals was routinely monitored weekly. Specifically, routine monitoring included activity, food and water intake, weight gain or loss, eyes, hair and other abnormal conditions of the experimental animals. Clinical symptoms observed during the experiment were all recorded in the raw data. After the administration was started, the body weight was measured twice a week. Tumor volume was measured twice a week, and the tumor volume was calculated as follows: tumor volume (mm³) = 0.5 × a × b², where a is the long diameter of tumor, and b is the short diameter of tumor. The monitoring ended 33 days after inoculation. On day 33 after inoculation, the relative tumor growth inhibition (TGI%) was calculated by the following formula: TGI% = (1 - mean relative tumor volume in treatment group/mean relative tumor volume in vehicle group) × 100%.

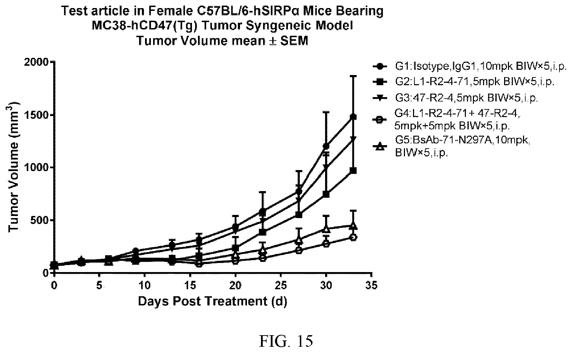

The results of tumor growth inhibition rate are shown in FIG. 15 and Table 17: on day 33 after inoculation, compared with the Isotype IgG1 group, the tumor growth inhibition rate of the single-drug 5 mg/kg L1-R2-4-71 was 32.25%, the tumor growth inhibition rate of the single-drug 5 mg/kg 47-R2-4 was 18.90%, the tumor growth inhibition rate of the drug combination of 5 mg/kg L1-R2-4-71 and 5 mg/kg 47-R2-4 was 76.73%, and the tumor growth inhibition rate of the 10 mg/kg anti-PD-L1/CD47 bispecific antibody BsAb-71-N297A was 68.70%. For the tumor growth inhibition effect, the bispecific antibody group was better than the single drug group, and compared with the drug combination group, there was no statistical difference (p *>* 0.05). The results of the body weight of the mice showed no significant difference between the body weights of the mice in each group.

**Table 16: Experiment design table**

| Group | Administration group | Dose (mg/kg) | Route | Frequency of administration/week |
|---|---|---|---|---|
| G1 | Isotype IgG1 (Sino Biological, HG1K) | 10 | Intraperitoneal injection | BIW×5 |
| G2 | L1-R2-4-71 | 5 | Intraperitoneal injection | BIW×5 |
| G3 | 47-R2-4 | 5 | Intraperitoneal injection | BIW×5 |
| G4 | L1-R2-4-71 + 47-R2-4 | 5+5 | Intraperitoneal injection | BIW×5 |
| G5 | BsAb-71-N297A | 10 | Intraperitoneal injection | BIW×5 |

**Table 17: Tumor growth inhibition rate on day 33**

| Group | Administration group | Tumor volume (mm³) | Tumor growth inhibition (TGI%) |
|---|---|---|---|
| G1 | Isotype IgG1 | 1480.47 | N/A |
| G2 | L1-R2-4-71 | 969.32 | 32.25% |
| G3 | 47-R2-4 | 1264.65 | 18.90% |
| G4 | L1-R2-4-71 + 47-R2-4 | 337.41 | 76.37% |
| G5 | BsAb-71-N297A | 450.76 | 68.70% |

Although certain representative embodiments and details have been shown for the purpose of illustrating the present invention, it will be apparent to those skilled in the art that various variations and modifications can be made to these embodiments and details without departing from the scope of the subject matter of the present invention.

## Claims

1. A bispecific antibody or an antigen-binding fragment, wherein the antibody or the antigen-binding fragment comprises a variable region a specifically binding to PD-L1, wherein the variable region a comprises one or more amino acid sequences of (a)-(f):
(a) a VHa CDR1 comprising an amino acid sequence set forth in any one of SEQ ID NOs: 4-8;
(b) a VHa CDR2 comprising an amino acid sequence set forth in any one of SEQ ID NOs: 9-18;
(c) a VHa CDR3 comprising an amino acid sequence set forth in SEQ ID NO: 19 or 20;
(d) a VLa CDR1 comprising an amino acid sequence set forth in any one of SEQ ID NOs: 36-40;
(e) a VLa CDR2 comprising an amino acid sequence set forth in any one of SEQ ID NOs: 41-44; and
(f) a VLa CDR3 comprising an amino acid sequence set forth in any one of SEQ ID NOs: 45-48.

2. The antibody or the antigen-binding fragment according to claim 1, wherein the variable region a comprises the VHa CDR1, the VHa CDR2, the VHa CDR3, the VLa CDR1, the VLa CDR2 and the VLa CDR3.

3. The antibody or the antigen-binding fragment according to claim 1, wherein the VHa CDR1 comprises an amino acid sequence set forth in SEQ ID NO: 4; the VHa CDR2 comprises an amino acid sequence set forth in any one of SEQ ID NOs: 9-14; and the VHa CDR3 comprises an amino acid sequence set forth in SEQ ID NO: 19; or
the VHa CDR1 comprises an amino acid sequence set forth in SEQ ID NO: 4; the VHa CDR2 comprises an amino acid sequence set forth in SEQ ID NO: 14; the VHa CDR3 comprises an amino acid sequence set forth in SEQ ID NO: 19; the VLa CDR1 comprises an amino acid sequence set forth in SEQ ID NO: 36; the VLa CDR2 comprises an amino acid sequence set forth in SEQ ID NO: 41; and the VLa CDR3 comprises an amino acid sequence set forth in SEQ ID NO: 45.

4. The antibody or the antigen-binding fragment according to any one of claims 1-3, wherein the antibody or the antigen-binding fragment further comprises a variable region b specifically binding to CD47, wherein the variable region b comprises one or more amino acid sequences of (g)-(l):
(g) a VHb CDR1 comprising an amino acid sequence set forth in any one of SEQ ID NOs: 21-23;
(h) a VHb CDR2 comprising an amino acid sequence set forth in any one of SEQ ID NOs: 24-28;
(i) a VHb CDR3 comprising an amino acid sequence set forth in any one of SEQ ID NOs: 29-35;
(j) a VLb CDR1 comprising an amino acid sequence set forth in any one of SEQ ID NOs: 36-40;
(k) a VLb CDR2 comprising an amino acid sequence set forth in any one of SEQ ID NOs: 41-44; and
(l) a VLb CDR3 comprising an amino acid sequence set forth in any one of SEQ ID NOs: 45-48.

5. The antibody or the antigen-binding fragment according to claim 4, wherein the variable region b comprises the VHb CDR1, the VHb CDR2, the VHb CDR3, the VLb CDR1, the VLb CDR2 and the VLb CDR3.

6. The antibody or the antigen-binding fragment according to claim 4, wherein the VHb CDR1 comprises an amino acid sequence set forth in SEQ ID NO: 21; the VHb CDR2 comprises an amino acid sequence set forth in SEQ ID NO: 24; the VHb CDR3 comprises an amino acid sequence set forth in SEQ ID NO: 29; the VLb CDR1 comprises an amino acid sequence set forth in SEQ ID NO: 36; the VLb CDR2 comprises an amino acid sequence set forth in SEQ ID NO: 41; and the VLb CDR3 comprises an amino acid sequence set forth in SEQ ID NO: 45.

7. The antibody or the antigen-binding fragment according to any one of claims 1-6, wherein the VLa CDR1 and the VLb CDR1 comprise a same amino acid sequence, the VLa CDR2 and the VLb CDR2 comprise a same amino acid sequence, and/or the VLa CDR3 and the VLb CDR3 comprise a same amino acid sequence.

8. The antibody or the antigen-binding fragment according to any one of claims 1-7, wherein the variable region a comprises a heavy chain variable region VHa and a light chain variable region VLa, wherein,
the VHa comprises an amino acid sequence set forth in any one of SEQ ID NOs: 49-63 or an amino acid sequence having at least 90% homology to the amino acid sequence set forth in any one of SEQ ID NOs: 49-63; and/or
the VLa comprises an amino acid sequence set forth in any one of SEQ ID NOs: 75-80 or an amino acid sequence having at least 90% homology to the amino acid sequence set forth in any one of SEQ ID NOs: 75-80.

9. The antibody or the antigen-binding fragment according to any one of claims 1-8, wherein,
the VHa comprises an amino acid sequence set forth in SEQ ID NO: 54 or an amino acid sequence having at least 90% homology to the amino acid sequence set forth in SEQ ID NO: 54; and/or
the VLa comprises an amino acid sequence set forth in SEQ ID NO: 75 or an amino acid sequence having at least 90% homology to the amino acid sequence set forth in SEQ ID NO: 75.

10. The antibody or the antigen-binding fragment according to any one of claims 1-9, wherein the variable region b comprises a heavy chain variable region VHb and a light chain variable region VLb, wherein,
the VHb comprises an amino acid sequence set forth in any one of SEQ ID NOs: 64-74 or an amino acid sequence having at least 90% homology to the amino acid sequence set forth in any one of SEQ ID NOs: 64-74; and/or
the VLb comprises an amino acid sequence set forth in any one of SEQ ID NOs: 75-80 or an amino acid sequence having at least 90% homology to the amino acid sequence set forth in any one of SEQ ID NOs: 75-80.

11. The antibody or the antigen-binding fragment according to any one of claims 1-9, wherein the VHb comprises an amino acid sequence set forth in SEQ ID NO: 64 or an amino acid sequence having at least 90% homology to the amino acid sequence set forth in SEQ ID NO: 64; and/or
the VLb comprises an amino acid sequence set forth in SEQ ID NO: 75 or an amino acid sequence having at least 90% homology to the amino acid sequence set forth in SEQ ID NO: 75.

12. The antibody or the antigen-binding fragment according to any one of claims 1-11, wherein the VLa and the VLb comprise a same amino acid sequence.

13. The antibody or the antigen-binding fragment according to any one of claims 1-12, further comprising a heavy chain constant region a and a heavy chain constant region b, wherein the heavy chain constant region a and the heavy chain constant region b are linked to the heavy chain variable region a and the heavy chain variable region b, respectively, and the heavy chain constant region a and/or the heavy chain constant region b comprise one or more of the following amino acid mutations in which amino acid positions are Eu numbered:
Y349C, S354C, T366W, T366S, L368A and Y407V; and
optionally the heavy chain constant region a and/or the heavy chain constant region b further comprise the following amino acid mutation: N297A.

14. The antibody or the antigen-binding fragment according to claim 13, wherein one of the heavy chain constant region a and the heavy chain constant region b comprises one or more of the following amino acid mutations:
N297A, S354C and T366W; and/or
the other heavy chain constant region comprises one or more of the following amino acid mutations:
N297A, Y349C, T366S, L368A and Y407V

15. The antibody or the antigen-binding fragment according to claim 13, wherein one of the heavy chain constant regions comprises an amino acid sequence set forth in SEQ ID NO: 81, 83 or 85 or an amino acid sequence having at least 90% homology to the amino acid sequence set forth in SEQ ID NO: 81, 83 or 85; and/or the other of the heavy chain constant regions comprises an amino acid sequence set forth in SEQ ID NO: 81, 84 or 86 or an amino acid sequence having at least 90% homology to the amino acid sequence set forth in SEQ ID NO: 81, 84 or 86.

16. A bispecific antibody or an antigen-binding fragment, wherein the antibody or the antigen-binding fragment comprises a heavy chain a, a heavy chain b and two identical light chains, wherein the heavy chain a is paired with one of the light chains to form a PD-L1 antigen-binding site, and the heavy chain b is paired with the other of the light chains to form a CD47 antigen-binding site.

17. The antibody or the antigen-binding fragment according to claim 16, wherein the heavy chain a comprises an amino acid sequence set forth in SEQ ID NO: 92 or 94 or an amino acid sequence having at least 90% homology to the amino acid sequence set forth in SEQ ID NO: 92 or 94; the heavy chain b comprises an amino acid sequence set forth in SEQ ID NO: 93 or 95 or an amino acid sequence having at least 90% homology to the amino acid sequence set forth in SEQ ID NO: 93 or 95; and the light chains comprise an amino acid sequence set forth in SEQ ID NO: 96 or an amino acid sequence having at least 90% homology to the amino acid sequence set forth in SEQ ID NO: 96.

18. A polynucleotide, encoding the antibody or the antigen-binding fragment according to any one of claims 1-15; or
an expression vector, comprising a polynucleotide encoding the antibody or the antigen-binding fragment according to any one of claims 1-17; or
a cell, comprising the polynucleotide encoding the antibody or the antigen-binding fragment according to any one of claims 1-17.

19. A composition, comprising the antibody or the antigen-binding fragment according to any one of claims 1-17, the polynucleotide, the expression vector or the cell according to claim 18 and a pharmaceutically acceptable carrier.

20. Use of the antibody or the antigen-binding fragment according to any one of claims 1-17, the polynucleotide, the expression vector or the cell according to claim 18 or the composition according to claim 19 in the manufacture of a medicament for treating a disease.
